# EUROPEAN PATENT APPLICATION

(11) **EP 4 729 025 A1**
(43) Date of publication of application: **22.04.2026**
(21) Application number: 23941206.7
(22) Date of filing: 18.08.2023
(51) Int. Cl.: A61F 2/24

(54) **PARAVALVULAR LEAK PREVENTION MATERIAL, PARAVALVULAR LEAK PREVENTION DEVICE, PARAVALVULAR LEAK PREVENTION SKIRT, MOLD, PROSTHETIC HEART VALVE, PROSTHETIC IMPLANT, AND RELATED METHOD**

(30) Priority: 16.06.2023 CN 202310718517; 16.06.2023 CN 202310720481; 16.06.2023 CN 202310722472; 15.08.2023 CN 202311029301
(71) Applicant: Venus MedTech (HangZhou), Inc., Hangzhou, Zhejiang 310052 (CN)
(72) Inventor: KUANG, Dajun, Hangzhou, Zhejiang 310052 (CN); XU, Shasha, Hangzhou, Zhejiang 310052 (CN); QI, Jesse Jun, Hangzhou, Zhejiang 310052 (CN); ZHEN, Ni, Hangzhou, Zhejiang 310052 (CN); ZHOU, Yan, Hangzhou, Zhejiang 310052 (CN); YANG, Yake, Hangzhou, Zhejiang 310052 (CN); XIAO, Min, Hangzhou, Zhejiang 310052 (CN)
(74) Representative: KIPA AB
(86) International application number: PCT/CN2023/113805
(87) International publication number: WO 2024/254980

(57) **Abstract**

This disclosure discloses a perivalvular leakage preventing component, a perivalvular leakage preventing device, a perivalvular leakage preventing skirt, a mold, a prosthetic heart valve, a prosthetic implant, and related methods. The prosthetic implant includes: a stent, being a radially deformable tubular structure having a through blood flow channel; a plurality of leaflets, each leaflet having a fixed edge and an opposite free edge, wherein the fixed edge is fixed to the stent, and the free edge is configured to control the blood flow channel; and a perivalvular leakage preventing structure arranged around the circumference of the stent. The perivalvular leakage preventing structure is: a sealing assembly, at least a portion of which extends radially outward from the stent and the sealing assembly includes a deformable structure; and/or an outer skirt wrapped around an outer periphery of the stent. The perivalvular leakage preventing structures of this disclosure enables the inflow end of the stent to form an adaptive sealing effect, thereby reducing perivalvular leakage.

## Description

### TECHNICAL FIELD

The present disclosure relates to the field of medical devices, and in particular to a perivalvular leakage preventing component, a perivalvular leakage preventing device, a perivalvular leakage preventing skirt, a mold used therefor, a prosthetic heart valve manufactured therefrom, a prosthetic implant, and related methods.

### DESCRIPTION OF THE PRIOR ART

Heart valve replacement surgery is widely used clinically as an effective means for treating heart valve diseases. Perivalvular leakage (PVL) is a type of non-structural valve dysfunction, often occurring early and late after prosthetic valve replacement surgery. Currently, two main methods for treating PVL are surgical treatment and interventional treatment. Although surgical treatment is becoming increasingly mature, the complications and mortality rates of redo surgery are significantly increased, and the recurrence rate of PVL is high; interventional treatment can effectively reduce the severity of PVL and improve its symptoms, but redo surgery still carries certain risks.

Currently, some technicians, from the perspective of design of valve of prosthetic implants, mainly reduce the incidence of PVL by adding a skirt at an inflow end of the valve to seal the PVL position. The materials used for the skirt can be broadly classified into biological materials and polymer materials. However, conventional skirt designs lack self-adaptive adjustment capability. Technicians often increase a thickness of the skirt to prevent PVL, but this increases the entry size of a delivery system. After the valve is retrieved multiple times during the interventional delivery process, the skirt is likely to be damaged.

### SUMMARY OF THE DISCLOSURE

In order to solve the problem of perivalvular leakage after a prosthetic implant is implanted in a body, the present disclosure discloses a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a perivalvular leakage preventing structure, arranged circumferentially around the stent, and the perivalvular leakage preventing structure being:
   a sealing assembly, at least a portion of the sealing assembly protruding radially outward from the stent, and the sealing assembly including a deformable structure; and/or
   an outer skirt wrapped around an outer periphery of the stent.

Optionally, the prosthetic implant further includes an inner skirt located radially inside the stent; wherein the sealing assembly and at least one of the inner skirt and the outer skirt are formed as a one-piece structure, or the sealing assembly is wrapped within a radial gap between the inner skirt and the outer skirt.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a sealing assembly, made of a deformable structure, and arranged circumferentially and continuously around the stent, at least a portion of the sealing assembly radially protruding outward from the stent;
a skirt, including an inner skirt located radially inside the stent and an outer skirt located radially outside the stent, the sealing assembly and at least one of the inner skirt and the outer skirt being a one-piece structure, or the sealing assembly wrapped within a radial gap between the inner skirt and the outer skirt, the sealing assembly fixed to the inner skirt through hollowed regions of the stent grids and wrapping around at least a portion of struts constituting the stent grid.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and an outer skirt located radially outside the stent, the inner skirt and the outer skirt forming a one-piece structure and made of PU.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a sealing assembly, including deformable structure, arranged circumferentially around the stent, at least a portion of the sealing assembly radially protruding outward from the stent;
a skirt, relative to a radial position of the stent, the skirt including an inner skirt and an outer skirt, the inner skirt and the outer skirt connected at an inflow side of the stent, forming a pouch structure, at least a portion of the stent and at least a portion of the sealing assembly located within the pouch structure.

The inner skirt and the outer skirt are connected as a one-piece structure or as separate components at the inflow side of the stent. For example, the inner skirt and the outer skirt are both made of PU, and are connected as a one-piece structure at the inflow side of the stent.

Or, the inner skirt is made of PU, and the outer skirt is made of PET fabric. Or, the inner skirt and the outer skirt are both made of PET fabric.

Optionally, the pouch structure extends continuously along the stent circumference. Or the pouch structure is arranged at intervals along the stent circumference. For example, the pouch structure is arranged at 3-6 placement points etc.

Optionally, all the sealing assembly are disposed in the pouch structure. The sealing assembly located within the pouch structure allows that the outer skirt provides additional protection for the sealing assembly and works in synergy with the radially protruding perivalvular leakage preventing effect. The relatively smooth surface of the outer skirt helps reduce frictional resistance during loading or delivery.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent, the sealing assembly and the outer skirt forming a one-piece structure, and the sealing assembly located at outer side of the outer skirt.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent; the sealing assembly and at least one of the inner skirt and the outer skirt forming a one-piece structure, or the sealing assembly encapsulated within the radial gap between the inner skirt and the outer skirt;
the sealing assembly including a plurality of sealing blocks, the plurality of sealing blocks arranged in multiple groups spaced apart circumferentially around the stent, each group having at least one sealing block, spacing regions exist between adjacent groups, with all the sealing blocks located away from the spacing regions along the stent circumference. That is, all the distributing regions of the plurality of sealing blocks are spaced apart circumferentially around the stent.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent; the sealing assembly and at least one of the inner skirt and the outer skirt forming a one-piece structure, or the sealing assembly encapsulated within the radial gap between the inner skirt and the outer skirt;
the sealing assembly includes a plurality of sealing blocks embedded in stent grids; sealing blocks in two circumferentially adjacent stent grids are arranged offset from each other along an axial direction of the stent; and along the axial direction of the stent, opposite sides of a sealing block are a wider side and a narrower side respectively, and the sealing blocks in two adjacent stent grids are positioned with the wider sides facing each other.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent; the sealing assembly and at least one of the inner skirt and the outer skirt forming a one-piece structure, or the sealing assembly encapsulated within the radial gap between the inner skirt and the outer skirt; the sealing assembly forms at least two circles of peaks around the outer periphery of the stent, the two circles of peaks are arranged along an axial direction of the stent and define a trough region therebetween for accommodating a native valve annulus, and the peaks in the same circle are a plurality of sealing blocks spaced circumferentially or a sealing ring continuously distributed circumferentially.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent; the sealing assembly and at least one of the inner skirt and the outer skirt forming a one-piece structure, or the sealing assembly encapsulated within the radial gap between the inner skirt and the outer skirt;
a reinforcement member, embedded in the skirt and connected to the stent via sutures passing through the reinforcement member, the reinforcement member voids the sealing assembly.

This disclosure further provides a prosthetic implant, including:
a stent, a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, including an inner skirt located radially inside the stent and/or an outer skirt located radially outside the stent;
a sealing assembly, including deformable structure, arranged circumferentially around the stent, at least a portion of the sealing assembly radially protruding outward from the stent; the sealing assembly and at least one of the inner skirt and the outer skirt forming a one-piece structure, or the sealing assembly encapsulated within the radial gap between the inner skirt and the outer skirt;
a radiopaque marker, pre-embedded in the skirt and/or the sealing assembly, joining regions between adjacent leaflets at the stent are joint portions, the circumferential position of the radiopaque markers correspond to respective joint portions, at least one group of markers is provided, positioned adjacent to the inflow side of the stent in the axial direction, and an axial distance between the group of the radiopaque makers and the leaflets is sufficient to at least accommodate a native annulus.

Optionally, the stent has an inflow side and an opposite outflow side, the outer skirt is arranged on the inflow side; the stent has stent grids, and the axial span of the outer skirt is 0.5 to 2 grids.

Optionally, the outer skirt has an inflow edge and an outflow edge; along an axial direction of the stent, the outflow edge of the outer skirt is adjacent to a midpoint position of the fixed edge of the leaflet and does not extend beyond the midpoint position towards the outflow side.

Optionally, the stent has a waist portion, and an outflow edge of the outer skirt is located at the waist portion.

Optionally, the stent has a waist portion, and a distance between an outflow edge of the outer skirt and the waist portion is 0.5 to 1 grid.

Optionally, a pouch structure is defined between the outer skirt and the inner skirt, and the sealing assembly is located within the pouch structure.

Optionally, along a radial direction of the stent, the pouch structure protrudes outward beyond an outer peripheral wall of the stent by 0.5 to 20 mm.

Optionally, the pouch structure is a fully enclosed structure.

Optionally, the pouch structure is a semi-open structure with openings facing an inflow side and/or an outflow side.

Optionally, a plurality of the openings are provided at intervals around the stent circumference.

Optionally, the inner skirt and the outer skirt are:
both made of polyurethane; or
one made of polyurethane and the other made of a biological material; or
both biological materials.

Optionally, the inner skirt and the outer skirt each have an inflow edge and an outflow edge; the outflow edge of the inner skirt is connected to the fixed edges of the plurality of leaflets; the inner skirt is formed from a first sheet, the outer skirt is formed from a second sheet; and configurations of the inner skirt and the outer skirt is:
the first sheet is fixed to an inner side of the stent, the second sheet is fixed to an outer side of the stent, and the inflow end of the stent is exposed; or
the first sheet is fixed to an inner side of the stent, the first sheet is everted outward at the inflow side of the stent and wraps around the inflow end of the stent, the second sheet material is fixed to an outer side of the stent and is connected to the everted portion of the first sheet; or
the second sheet is fixed to an outer side of the stent, the second sheet is inverted inward at the inflow side of the stent and wraps around the inflow end of the stent, the first sheet is fixed to an inner side of the stent and is connected to the inverted portion of the second sheet; or
the first sheet is fixed to an inner side of the stent, the second sheet is fixed to an outer side of the stent, and the first sheet and the second sheet are a one-piece structure and wrap the inflow end of the stent;
wherein the sealing assembly and one of the first sheet and the second sheet are formed as a one-piece structure; or the sealing assembly is bonded to at least one of the first sheet and the second sheet; or the sealing assembly is sandwiched between the first sheet and the second sheet, and a radial position of the sealing assembly is disposed at the inner side or the outer side of the second sheet.

Optionally, the inflow edge of the inner skirt and the inflow edge of the outer skirt are substantially aligned, and are connected as a one-piece structure or as separate components to seal the blood flow channel; in a radial direction, the sealing assembly is disposed at an inner side or an outer side of the outer skirt.

Optionally, shapes of inflow edges of the inner skirt and the outer skirt are independent of each other, each of the inflow skirt and the outer skirt has a smooth inflow edge or an inflow edge conforming to a shape of the stent grids.

Optionally, an outflow edge of the outer skirt extends smoothly along the stent circumference; or an outflow edge of the outer skirt has axial undulations along the stent circumference.

Optionally, a degree of axial undulation matches a shape of struts of the stent or matches a shape of an axial edge of the sealing assembly.

Optionally, the first sheet and/or the second sheet are made of polyurethane with a thickness of 0.02-0.2 mm.

Optionally, the material of the sealing assembly is polyurethane and the same material as at least one of the first sheet and the second sheet, and the sealing assembly and at least one of the first sheet and the second sheet are formed as one-piece structure.

Optionally, the first sheet is made of polyurethane, and the second sheet is made of PET fabric.

Optionally, the inner skirt and the outer skirt are fixed to each other by at least one of bonding or suturing.

Optionally, the inner skirt and the outer skirt are fixed to each other continuously or at intervals along the stent circumference. For example, an outflow side of the outer skirt and a corresponding part of the inner skirt are fixed in the above-mentioned continuous or intermittent manner.

Optionally, the material of the sealing assembly is polyurethane or other polymer materials.

Optionally, the sealing assembly may have internal voids. For example, the sealing assembly may have a foam structure with a pore size of 5-500 micrometers and a porosity of 40%-95%.

Optionally, the sealing assembly may be made of swellable material that has absorbed water.

Optionally, an arrangement of the sealing assembly is one of the following:
a plurality of sealing blocks arranged circumferentially along the stent; or
the sealing assembly being an annular member located around an outer periphery of the stent, and the sealing assembly directly sleeved over the outer periphery of the stent; or
the sealing assembly directly sleeved over an outer periphery of the outer skirt.

Optionally, the plurality of sealing blocks are arranged in multiple groups spaced apart circumferentially around the stent, each group has at least one sealing block, spacing regions exist between adjacent groups, with all sealing blocks located away from the spacing regions along the stent circumference.

Optionally, the sealing blocks are arranged in two to nine groups, for example, three to six groups. The groups of the sealing blocks are evenly arranged along the stent circumference.

Optionally, the plurality of sealing blocks are arranged in multiple groups spaced apart circumferentially around the stent, each group includes 1 to 10 sealing blocks, and distribution regions of the sealing blocks within the same group are continuously arranged along the stent circumference.

Optionally, distribution regions of all the plurality of sealing blocks are continuously arranged along the stent circumference.

Optionally, in the radial direction, the sealing blocks are disposed at an inner side of the outer skirt; and each sealing block is embedded in a corresponding stent grid or each sealing block is randomly filled between the two skirts.

Optionally, one grid contains only one continuously distributed sealing block; or one grid contains multiple spaced sealing blocks.

Optionally, the stent has multiple rows of grids, and all sealing blocks are distributed only within a same row.

Optionally, the sealing blocks in two adjacent stent grids are arranged offset from each other along an axial direction of the stent.

Optionally, along the axial direction of the stent, opposite sides of a sealing block are a wider side and a narrower side respectively, and the sealing blocks in two adjacent stent grids are positioned with the wider sides facing each other.

Optionally, the wider sides of all the sealing blocks are connected to each other and extend continuously the stent circumference.

Optionally, viewed from a radial perspective of the stent, a shape of the sealing blocks is circular, elliptical, rectangular, rhombic, triangular, or hexagonal.

Optionally, each sealing block is embedded in a corresponding stent grid, and viewed from a radial perspective of the stent; the sealing block fills the entire stent grid in which it is located, or occupy only a partial region of the stent grid in which it is located.

Optionally, the sealing block is located in a central region of the stent grid in which it is located, or adjacent to one axial end of the stent grid in which it is located.

Optionally, a single stent grid contains two sealing blocks spaced apart axially, with each sealing block located adjacent to one axial end of the stent grid.

Optionally, in a longitudinal cross-section of the sealing block, the sealing block has an inner side facing the stent and an opposite outer side, and (the inner side fits against the skirt) a shape of an edge of the outer side is a broken line, a smooth curved line, or a combination of both.

Optionally, an edge of an outer side of the sealing block is a broken line, and the longitudinal cross-section of the sealing block is triangular, rectangular, or trapezoidal.

Optionally, an edge of an outer side of the sealing block is a smooth curve and has a radially highest point (roughly the point of maximum thickness of the sealing block), and along the axial direction, the highest point within the stent grid is closer to the inflow side.

Optionally, along a radial direction of the stent, a thickness of the sealing block is uniform or non-uniform distributed, and a maximum thickness of the sealing block is 0.5-5 mm.

Optionally, the sealing assembly overall spans 0.5-3 grids (such as 0.5-2 grids) along an axial direction.

Optionally, a shape of a longitudinal cross-section (external profile) of the annular member is circular, truncated circular (for example, a semicircle), trapezoidal, rectangular, elliptical, B-shaped, triangular, etc.

Optionally, an installation location of the sealing assembly is one of the following:
the sealing assembly is fixed to the inner skirt and protrudes from a radial inside of the stent towards a radial outside through corresponding grids, and the outer skirt surrounds an outside of the sealing assembly; or
the sealing assembly is fixed to an inner side of the outer skirt and is located within an interlayer between the outer skirt and the inner skirt; or
the sealing assembly is fixed to an outer side of the outer skirt.

Optionally, the outer skirt surrounds an outside of the sealing assembly, and the outer skirt wraps all or part of the sealing assembly.

Optionally, in a longitudinal section of the sealing assembly, a shape of an edge of an outer side of the sealing assembly has two peaks and a trough between the two peaks (such as the B-shape described above), and an outflow side of the outer skirt is located at the position of the trough.

Optionally, the outer skirt and the inner skirt are connected to the stent by sutures, at least one of the outer skirt and the inner skirt includes the reinforcement member, and the sutures pass through the reinforcement member.

Optionally, the reinforcement member is a suture reinforcement wire or a reinforcement fabric pre-embedded within the skirt; or the reinforcement member is a reinforcement piece directly attached to the outer skirt and/or the inner skirt.

Optionally, the material of the reinforcement member is PU or PET.

Optionally, the reinforcement piece is located on an outer side of the outer skirt or on an inner side of the inner skirt, or radially between the outer skirt and the inner skirt.

Optionally, the reinforcement piece and the skirt to which the reinforcement piece is attached form a one-piece structure or are fixed as separate parts.

Optionally, at a junction of the inner skirt and the leaflet, the reinforcement piece, the leaflet, and the inner skirt are stacked and fixed in any order.

Optionally, the reinforcement member is distributed over the entire inner skirt or only located at an outflow side of the inner skirt and connected to the fixed edge of the leaflet. That is, an edge of the reinforcement member is aligned with the outflow side of the inner skirt.

Optionally, the reinforcement member avoids or pass through the sealing assembly; the sutures avoid or pass through the sealing assembly.

Optionally, the suture reinforcement wire extends along the stent circumference to form a wire loop, the wire loop is a single circle or multiple rings, wherein multiple rings of the wire loops are arranged spaced apart or extend continuously in a spiral manner.

Optionally, at least one of the outer skirt, the inner skirt, the sealing assembly, and the reinforcement member is pre-embedded with a radiopaque marker.

Optionally, the radiopaque marker is formed by at least one of the following methods:
the radiopaque marker is pre-formed and acts as a pre-embedded component in a combining process;
the radiopaque marker is mixed into at least one raw material used in a combining process.

Optionally, the radiopaque marker is strip-shaped, powdered, sheet-shaped, or block-shaped.

Optionally, the radiopaque marker is strip-shaped and extends along an axial direction or a circumferential direction of the stent (can indicate position and orientation).

Optionally, the sealing assembly is an annular member, and the radiopaque marker is arranged circumferentially along the annular member.

Optionally, the sealing assembly includes a plurality of sealing blocks, with radiopaque markers provided in at least two of them (such as three to six, or even more).

Optionally, the radiopaque marker is located in a central region (e.g., a center point or centerline) of its sealing block.

Optionally, the radiopaque marker includes a first marker and a second marker, the two markers are located at the corresponding peaks, respectively.

Optionally, the radiopaque marker is located at the trough.

Optionally, joining regions between two circumferentially adjacent leaflets at the stent are joint portions, the radiopaque marker includes multiple groups, and along a circumference of the stent, each group corresponds to a position of a respective joint portion.

Optionally, along an axial direction of the stent, the radiopaque marker is adjacent to an inflow side of the stent.

The present disclosure integrates the skirt with the self-expanding sealing assembly. By leveraging the excellent shape adaptability of the sealing assembly, it enhances the perivalvular leakage preventing effect.

In order to solve the problem of perivalvular leakage after a prosthetic implant is implanted in a body, the present disclosure discloses a perivalvular leakage preventing device of a prosthetic implant. The perivalvular leakage preventing device may also be understood as a further improvement of the aforementioned sealing assembly, which utilizes the pulling wire (one form of the reinforcement member mentioned earlier) to connect the plurality of sealing blocks.

This disclosure further provides a perivalvular leakage preventing device for a prosthetic implant, wherein the prosthetic implant includes a stent with a grid structure and being tubular, the perivalvular leakage preventing device includes:
a plurality of sealing blocks arranged around a circumference of the stent, each sealing block made of a deformable structure and embedded in a corresponding grid structure;
a pulling wire, the sealing blocks are connected to each other via the pulling wire.

Optionally, according to the correspondence with the grid structure, the sealing blocks are arranged in multiple rows along an axial direction of the stent, and adjacent rows are staggered relative to each other.

Optionally, the sealing blocks in the same row are connected only by the pulling wire.

Optionally, a connecting strip integrally formed with the sealing blocks is provided between adjacent sealing blocks in the same row.

Optionally, the sealing block fully fills the grid structure in which the sealing block is located.

Optionally, the sealing block partially fills the grid structure in which the sealing block is located.

Optionally, the sealing blocks are arranged in two rows, wherein the sealing blocks in the first row fully fill the grid structures where they are located, and the sealing blocks in the second row partially fill the grid structures where they are located.

Optionally, in the first row, a spaced portion is present between every two adjacent sealing blocks, and the sealing blocks in the second row are located in the corresponding spaced portions.

Optionally, along an axial direction of the stent, the grid structure has a central region and edge regions disposed on two sides of the central region; along the radial direction of the stent, a thickness of the sealing block decreases from the central region towards the edge regions of the grid where it is located; along a stent circumferential direction, a width of the sealing block decreases from the central region towards the edge regions of the grid where it is located.

Optionally, the sealing block has a porous structure.

Optionally, the sealing block is made of foamed polyurethane.

Optionally, the porosity of the sealing block is 50-85.

Optionally, along a radial direction of the stent, the perivalvular leakage preventing device has an inner side and an outer side, and a position of the pulling wire is adjacent to the inner side.

Optionally, the pulling wire is located on the inner side of the stent, and the sealing blocks protrude outward from an outer peripheral surface of the stent.

Optionally, the perivalvular leakage preventing device has a coiled state wound around the stent and a relatively unfolded state; in the unfolded state, a plurality of pulling wires are arranged along their extension direction.

Optionally, at least one pulling wire passes through all the sealing blocks.

Optionally, the sealing blocks are arranged in multiple rows along an axial direction of the stent, and at least one pulling wire passes through all the sealing blocks in one of the rows.

Optionally, two of the pulling wires are parallel to each other.

Optionally, two of the pulling wires intersect.

Optionally, at least one pulling wire extends along a length direction of the perivalvular leakage preventing device.

Optionally, in the unfolded state, at least one pulling wire extends along a length direction of the perivalvular leakage preventing device.

Optionally, in the unfolded state, at least two pulling wires extend along a length direction of the perivalvular leakage preventing device.

Optionally, the pulling wires are arranged in at least two groups, each group consists of multiple parallel wires, one of the groups extends along a length direction of the perivalvular leakage preventing component, and another group extends along a width direction of the perivalvular leakage preventing device.

Optionally, the pulling wires of different extension directions belong to the same pulling wire and are meanderingly arranged.

Optionally, according to the correspondence with the grid structure, the sealing blocks are arranged in one or more rows; at least one pulling wire passing through the sealing blocks in the same row extends along the length direction of the perivalvular leakage preventing device in the unfolded state. Preferably, at least two pulling wires passing through the sealing blocks of the same row are provided.

Optionally, in the unfolded state, along a width direction of the perivalvular leakage preventing device, at least one of the pulling wires, which pass through the sealing blocks in the same row, is adjacent to a central region of the sealing blocks.

Optionally, a limiting component is fixed to the pulling wire and is embedded within the sealing block.

Optionally, the limiting component is a knot.

Optionally, the limiting component further includes a meandering structure connected to the knot.

Optionally, the pulling wires are all connected via the limiting component.

Optionally, the pulling wire is made of PTFE material.

Optionally, the perivalvular leakage preventing device further includes an inner membrane; in the coiled state, the inner membrane is located on the inner side, and the sealing blocks are fixed on an outer side of the inner membrane.

Optionally, in the same row, a spaced portion is defined between two adjacent sealing blocks; along its extending path, a portion of the pulling wire is located in the spaced portion and is fixed to the inner membrane.

Optionally, the inner membrane and the sealing blocks are made of the same material.

Optionally, a thickness of the inner membrane is 0.1-0.5 mm.

This disclosure provides a perivalvular leakage preventing device for a prosthetic implant, wherein the prosthetic implant has a stent with a grid structure and is tubular, the perivalvular leakage preventing device including:
a plurality of sealing blocks arranged around the circumference of the stent, each sealing block being made of a deformable structure and embedded in a corresponding cell structure;
an inner membrane, the plurality of sealing blocks fixed to one side of the inner membrane, and the inner membrane and the plurality of sealing blocks are made of the same material.

This disclosure provides a prosthetic implant including a stent and a perivalvular leakage preventing device wrapped around the stent, the stent being tubular and having a grid structure, the perivalvular leakage preventing device including:
a plurality of sealing blocks, the plurality of sealing blocks arranged around the circumference of the prosthetic implant, each sealing block made of a deformable structure and embedded in a corresponding grid structure;
a pulling wire, the plurality of sealing blocks connected to each other via the pulling wire to constitute the perivalvular leakage preventing device;
or the perivalvular leakage preventing device includes:
   a plurality of sealing blocks arranged around the circumference of the stent, each sealing block made of a deformable structure and embedded in a corresponding grid structure;
   an inner membrane, the plurality of sealing blocks fixed to one side of the inner membrane.

Optionally, the inner membrane and the plurality of sealing blocks are made of the same material.

Optionally, the prosthetic implant is a prosthetic heart valve.

This disclosure provides a method for producing a prosthetic implant, including:
providing a stent and a perivalvular leakage preventing device separately, wherein the stent is tubular and has a cell structure, and the perivalvular leakage preventing device includes:
a plurality of sealing blocks, multiple blocks arranged around the circumference of the prosthetic implant, each sealing block made of a deformable structure;
a pulling wire, the sealing blocks connected to each other via the pulling wire to constitute the perivalvular leakage preventing device;
the processing method including:
   curling the perivalvular leakage preventing device and connecting ends of the perivalvular leakage preventing device to form a coiled state;
   fixing the perivalvular leakage preventing device in the coiled state to the stent, during fixation, each sealing block embedded in a corresponding grid structure.

This disclosure provides a method for producing a prosthetic implant, including:
providing a stent and a perivalvular leakage preventing device separately, wherein the stent is tubular and has a grid structure, and the perivalvular leakage preventing device includes:
a plurality of sealing blocks, the plurality of sealing blocks arranged around the circumference of the prosthetic implant, each sealing block made of a deformable structure;
a pulling wire, the sealing blocks connected to each other via the pulling wire to constitute the perivalvular leakage preventing device;
the processing method including:
   placing one part of the perivalvular leakage preventing device along a length direction of the perivalvular leakage preventing device into an inside or an outside of the stent and fixing the perivalvular leakage preventing device to the stent;
   gradually fixing the remaining part of the perivalvular leakage preventing device to the stent until ends of the perivalvular leakage preventing device are connected to form a coiled state, during fixation, each sealing block embedded in a corresponding grid structure.

This disclosure provides a mold for forming a perivalvular leakage preventing device, including:
a main body having a working surface on one side, the working surface defining:
a plurality of mold cavities configured to form sealing blocks;
a wire groove intersecting with the mold cavities for receiving a pulling wire, the formed sealing blocks are connected by the pulling wire to constitute the perivalvular leakage preventing device.

Optionally, the wire groove is open on the working surface.

Optionally, the wire groove extends to an edge of the main body.

Optionally, the wire groove extends along a straight line.

This disclosure provides a mold for forming a perivalvular leakage preventing device, including:
a main body having a working surface on one side, the working surface defining a plurality of mold cavities configured to form sealing blocks;
a forming frame stacked on the working surface and surrounding a periphery of openings of the mold cavities.

Optionally, the forming frame and the main body are separate structures or an integral structure. The forming frame and the wire groove may be combined.

Optionally, the working surface has a recessed region, and the plurality of mold cavities are located within the recessed region.

Optionally, a depth of the recessed region and a thickness of the forming frame both correspond to a thickness of the inner membrane.

This disclosure provides a method for producing a perivalvular leakage preventing device using a mold, including:
adding raw material of a sealing block into a plurality of mold cavities of the mold;
laying a pulling wire, an extension path of the pulling wire intersects with the mold cavities;
curing the raw material of the sealing block under predetermined conditions;
demolding the formed sealing blocks connected via the pulling wire to obtain the perivalvular leakage preventing device.

Optionally, adding raw material of a sealing block and laying a pulling wire are performed in any order, or alternately.

Optionally, the raw material of the sealing block is fluid or paste-like, and is leveled or scraped flat to be flush with the working surface.

Optionally, before curing the raw material of the sealing block, raw material of an inner membrane is further used to form a coating on a top side of the raw material of the sealing block, and then the raw material of the inner membrane and the raw material of the sealing block are cured simultaneously.

Optionally, at least one of the raw material of the sealing block and raw material of an inner membrane is paste-like.

Optionally, the raw material of the sealing block contains solid particles, and the method further includes removing the solid particles after demolding.

Optionally, a curing temperature is 10-60°C, and a curing time is 1-24 hours.

The perivalvular leakage preventing device of the present disclosure may serve as a foam-based sealing component, a sealing cuff, or a sealing skirt of an endovascular prosthesis, such as a transcatheter aortic valve implantation device. Following structural improvements to the perivalvular leakage preventing device, the present disclosure demonstrates enhanced compatibility with interventional delivery methods while maintaining effective sealing performance.

This disclosure further provides a perivalvular leakage preventing skirt, which offers effective sealing and excellent deliverability. A perivalvular leakage preventing skirt, at least a portion of the perivalvular leakage preventing skirt has a flexible substrate, and at least a portion is provided with a reinforcement component in the form of a fabric, and the flexible substrate and the reinforcement component have an overlapping region.

Optionally, the perivalvular leakage preventing skirt includes:
the flexible substrate made of polyurethane membrane;
the reinforcement component in the form of a fabric, wherein at least a portion of the reinforcement component and at least a portion of the flexible substrate overlap and are fixed to each other.

Optionally, at least a portion of the reinforcement component extends beyond the flexible substrate.

Optionally, the perivalvular leakage preventing skirt further includes a sealing assembly fixed to the flexible substrate, the sealing assembly is made of a deformable structure.

Optionally, an elongation at break of the flexible substrate is not less than 200%, and a tensile strength of the flexible substrate is 30-100 MPa.

Optionally, the sealing assembly is made of polyurethane with an open-pore structure.

Optionally, the pores in the sealing assembly are interconnected and have uniform pore size.

Optionally, the porosity of the sealing assembly is 50%-95%.

Optionally, the fabric is a flexible fabric with a thickness range of 0.04 mm to 0.2 mm.

Optionally, the raw material of the fabric is one of polyester (Polyethylene terephthalate, PET), nylon (Polyamide fiber, PA), or polyetheretherketone (PEEK).

Optionally, the fabric is a weft-knitted fabric.

Optionally, the perivalvular leakage preventing skirt has an inflow end and an opposite outflow end according to an intended blood flow direction in a physiological environment, and the portion of the reinforcement component extending beyond the flexible substrate is located at the outflow end.

Optionally, the sealing assembly is located at the inflow end of the perivalvular leakage preventing skirt.

Optionally, in the axial direction of the perivalvular leakage preventing skirt, the sealing assembly is spaced apart from the reinforcement component.

Optionally, a distance between the sealing assembly and the reinforcement component is 3-20 mm.

Optionally, the sealing assembly and the reinforcement component are located on the same side of the flexible substrate; or the sealing assembly and the reinforcement component are located on different sides of the flexible substrate.

Optionally, the sealing assembly protrudes relative to the flexible substrate.

Optionally, the flexible substrate has a first edge and an opposite second edge, the first edge corresponds to a position of the inflow end of the perivalvular leakage preventing skirt, the second edge is configured to be fixed to the stent after being sutured to a leaflet, and the second edge is closer to the outflow end of the stent than the first edge;
the reinforcement component has a third edge and an opposite fourth edge, and the fourth edge of the reinforcement component has the same shape as the second edge of the flexible substrate.

Optionally, the third edge and the fourth edge of the reinforcement component have the same shape, the reinforcement component is substantially wavy, and has the same width throughout.

Optionally, the third edge of the reinforcement component is a smooth arc.

Optionally, the fourth edge of the reinforcement component is aligned with the second edge of the flexible substrate.

Optionally, the flexible substrate has a first edge and an opposite second edge, the second edge is closer to the outflow end of the stent than the first edge;
the reinforcement component has a third edge and an opposite fourth edge, and the fourth edge of the reinforcement component extends beyond the second edge of the flexible substrate to form an extension part for suturing.

Optionally, the sealing assembly consists of at least one protrusion fixed onto the flexible substrate.

Optionally, the reinforcement component and the flexible substrate are connected by at least one of the following methods: solvent bonding, solution bonding, thermal fusion bonding, or suture splicing.

Optionally, the solvent used for solvent bonding is at least one of dimethyl sulfoxide (DMSO), N,N-dimethylformamide (DMF), or N,N-dimethylacetamide (DMAC).

Optionally, the solution used for solution bonding is a polyurethane solution with a polyurethane content of 5 - 20 wt.%, polyurethane solution is at least one of dimethyl sulfoxide (DMSO), N,N-dimethylformamide (DMF), or N,N-dimethylacetamide (DMAC).

Optionally, when using a solution or solvent for bonding, after the bonding area is fully wetted by the solvent or solution, it is dried at a temperature of 50-70°C.

Optionally, a temperature for thermal fusion bonding is 50-70°C, and a heating time is 0.5-2 hours.

Optionally, the sealing assembly and the flexible substrate are connected via a bonding region, and the bonding region is formed by contacting, penetrating, and curing the portion of the flexible substrate adjacent to the protrusion in a solution form.

Optionally, a thickness T1 of the flexible substrate is 0.03 mm-0.05 mm, a thickness T2 of the reinforcement component is 0.04 mm-0.2 mm, a thickness at a location where the flexible substrate and the reinforcement component are overlapped and fixed is T3, which is 0.05 mm-0.24 mm, and T1, T2, T3 satisfy the constraint: 0.05 mm≥T1+T2-T3≥0.01 mm.

This disclosure provides a method for producing a perivalvular leakage preventing skirt, including:
attaching a reinforcement component and a sealing assembly respectively to a flexible substrate;
cutting the reinforcement component and the flexible substrate into a predetermined shape.

Optionally, connecting the reinforcement component and the sealing assembly to the flexible substrate adopts any one of the following sequences:
a) attaching the reinforcement component and then the sealing assembly sequentially to the flexible substrate;
b) attaching the sealing assembly and then the reinforcement component sequentially to the flexible substrate.

Optionally, cutting the reinforcement component and the flexible substrate into the predetermined shape is performed in any one of the following sequences:
1) cutting the flexible substrate and the reinforcement component separately, before connecting the reinforcement component to the flexible substrate;
2) cutting the flexible substrate and the reinforcement component simultaneously, after connecting the reinforcement component to the flexible substrate.

Optionally, the preparation of the sealing assembly includes:
mixing a polyurethane solution with solid particles to obtain a pre-mix;
curing and washing the pre-mix, thereby obtaining the sealing assembly having a pore structure.

Optionally, a particle size of the solid particles is 80-120 mesh.

This disclosure further provides a prosthetic implant, including:
a stent, being a deformable tubular structure with a side wall having hollowed regions, an interior of the stent forming a blood flow channel;
one or more leaflets, connected to the stent for controlling the blood flow channel;
a perivalvular leakage preventing skirt, the perivalvular leakage preventing skirt located substantially inside the stent, an outflow end of the perivalvular leakage preventing skirt connected to the leaflets, and the sealing assembly protruding outward from the stent via the hollowed region of the stent.

Optionally, the sealing assembly is located at an inflow end of the stent, and a height of the sealing assembly along an axial direction corresponds to 1 to 2 rows of grids at the inflow end of the stent.

This disclosure further provides a method for producing the prosthetic implant, including:
connecting the leaflets and the perivalvular leakage preventing skirt to each other to form a tubular pre-assembly;
assembling the pre-assembly to the stent.

The perivalvular leakage preventing skirt provided in this disclosure offers at least one or more of the following beneficial effects:
a) The perivalvular leakage preventing skirt employs a polyurethane membrane as the flexible substrate, which is simultaneously reinforced by a reinforcing member in the form of a fabric. Compared to porcine pericardial material, the polyurethane membrane is thinner, more flexible, and its properties are more easily controlled. The reinforcing member enhances the sewability of the flexible substrate. Specifically, in regions to be sutured, the reinforcing member bears the tensile force from the suture, compensating for the inherent strength deficiency of the flexible substrate. This not only improves suture strength but also extends the service life of the skirt.
b) The reinforcing member is fixed overlapping the flexible substrate, with at least a portion extending beyond an edge of the flexible substrate to serve as an extension part for sutures to pass through. This extension part consists solely of the reinforcing member, without the flexible substrate. This design ensures suture strength while simultaneously reducing the delivery profile.
c) The sealing assembly employs polyurethane with an open-pore structure, endowing it with excellent compression resilience. A volume of the sealing assembly in the compressed state can be as low as 10% of a volume in the natural state. During delivery, the sealing assembly can be compressed to a small radial size. After deployment at the implant site, upon pressure release, it self-expands via capillary action through the pore structure in the physiological environment. Based on its open-pore structure, it can adaptively adjust its height and shape according to complex physiological conditions, achieving active, seamless occlusion. Furthermore, this material possesses good biocompatibility and is non-thrombogenic, enabling ideal anti-perivalvular leak outcomes. Additionally, the axial length of the occlusion assembly is less than that of the stent, preventing any increase in the delivery system's dimensions.
d) A connection between the flexible substrate and the reinforcing member utilizes methods such as solvent bonding, solution bonding, or thermal fusion bonding. On one hand, this reduces a thickness at the overlapping section. On the other hand, as the solvent, solution, or molten flexible substrate can permeate the interstices of the fabric reinforcing member, it increases the bond strength, making the two less prone to delamination.
e) The sealing assembly and the flexible substrate are made of the same material, namely polyurethane. Due to the same material, a bonded region is formed through melting, penetration, and solidification at their contact interface, resulting in superior interfacial bond strength and resistance to separation.

This disclosure further provides a self-adaptive perivalvular leakage preventing component which achieves excellent occlusion performance without increasing the delivery access size.

This disclosure provides an adaptive perivalvular leakage preventing component, the perivalvular leakage preventing component including: a sheet-like and stretchable flexible substrate, and one or more protrusions attached to the flexible substrate, wherein each protrusion has a pore structure, the flexible substrate and the protrusions are both made of polyurethane material, and the connection between the flexible substrate and the protrusions is achieved by curing a polyurethane solution.

Optionally, an elongation at break of the flexible substrate is not less than 200%.

Optionally, an elongation at break of the flexible substrate is not less than 300%.

Optionally, an elongation at break of the flexible substrate is 200%-500%.

Optionally, a pore size of the protrusions is 5-500 micrometers. Preferably, a pore size of the protrusions is 5-450 micrometers. Preferably, a pore size of the protrusions is 5-400 micrometers. Preferably, a pore size of the protrusions is 5-350 micrometers. Preferably, a pore size of the protrusions is 5-300 micrometers. Preferably, a pore size of the protrusions is 5-250 micrometers.

Optionally, a porosity of the protrusions is 50%-95%. Preferably, a porosity of the protrusions is 60%-95%. Preferably, a porosity of the protrusions is 70%-90%.

Optionally, the protrusions have an open-pore structure and/or a closed pore structure.

Optionally, an average thickness of the flexible substrate is 0.02-0.20 mm. Preferably, an average thickness of the flexible substrate is 0.02-0.15 mm. Preferably, an average thickness of the flexible substrate is 0.02-0.12 mm.

Optionally, a tensile strength of the flexible substrate is 30-100 MPa. Preferably, a tensile strength of the flexible substrate is 40-100 MPa. Preferably, a tensile strength of the flexible substrate is 50-100 MPa. Preferably, a tensile strength of the flexible substrate is 50-90 MPa. Preferably, a tensile strength of the flexible substrate is 50-80 MPa.

This disclosure further provides an adaptive perivalvular leakage preventing component, the perivalvular leakage preventing component including: a sheet-like, stretchable flexible substrate, and one or more protrusions attached to the flexible substrate, wherein each protrusion has a pore structure, the flexible substrate and the protrusions are both made of polyurethane material, and the flexible substrate and the protrusions are connected via a bonding region, the bonding region is formed by contacting, penetrating, and curing a portion of the flexible substrate adjacent to the protrusion in a solution form.

This disclosure further provides an adaptive perivalvular leakage preventing component, the perivalvular leakage preventing component made of polyurethane material and including: a sheet-like , stretchable flexible substrate, and one or more protrusions connected to the flexible substrate, wherein the flexible substrate and the protrusions are both formed by a casting molding process, casting liquid for forming the protrusions is a polyurethane solution containing solid particles, and casting liquid for the flexible substrate is a polyurethane solution or a polyurethane mixture containing solid particles.

This disclosure further provides an adaptive perivalvular leakage preventing component, the perivalvular leakage preventing component includes a flexible substrate and one or more protrusions. A porosity of the protrusions is not lower than that of the flexible substrate. An interface between the flexible substrate and the protrusion is formed by contacting, penetrating, and curing a portion of the flexible substrate adjacent to the protrusion in a solution form.

Optionally, the protrusions and the flexible substrate are made of the same material.

Optionally, the protrusions and the flexible substrate are made of polyurethane.

Optionally, both the protrusions and the flexible substrate have a pore structure. A porosity of the protrusions is not lower than that of the flexible substrate.

Optionally, each protrusion has a pore structure, and the flexible substrate is a dense structure without pores.

This disclosure further provides a method for producing an adaptive perivalvular leakage preventing component, including the following steps in any order:
filling a mold with a polyurethane mixture containing solid particles, curing the mixture, and then washing the mixture to remove the solid particles to obtain protrusions;
obtaining a flexible substrate by curing a polyurethane solution, or by curing and then washing a polyurethane mixture containing solid particles;
connecting the flexible substrate and the protrusions by curing a polyurethane solution.

Optionally, a polyurethane molecular chain includes hard segments and soft segments, the soft segment content is 40-70%, and the remainder is the hard segments; the soft segment is at least one of polyether diol, polycarbonate diol, polyester diol, or polysiloxane diol; the hard segment is isocyanate, and a R value of the isocyanate is 1.0-1.1.

Optionally, in the polyurethane molecular chain, the soft segment content is 50%-65%.

Optionally, the isocyanate is at least one of TDI, HDI, MDI, NDI, PPDI, IPDI, or XDI.

Optionally, the polyurethane molecular chain further includes a chain extender (the chain extender belongs to the hard segment), and the chain extender is at least one of ethylene glycol, butanediol, hexanediol, octanediol, or ethylenediamine.

Optionally, the solvent for the polyurethane solution is at least one of dimethyl sulfoxide (DMSO), tetrahydrofuran (THF), dimethylformamide (DMF), dimethylacetamide (DMAc), dichloromethane, chloroform, or hexafluoroisopropanol (HFIP).

Optionally, the solvent for the polyurethane solution is two selected from dimethyl sulfoxide (DMSO), tetrahydrofuran (THF), dimethylformamide (DMF), dimethylacetamide (DMAc), dichloromethane, chloroform, and hexafluoroisopropanol (HFIP), and a volume ratio of the two solvents is 1-99:1. The volume ratio of the two solvents is 5-95:1. The volume ratio of the two solvents is 10-95:1. The volume ratio of the two solvents is 15-95:1. The volume ratio of the two solvents is 20-95:1. The volume ratio of the two solvents is 25-95:1. The volume ratio of the two solvents is 10-90:1. The volume ratio of the two solvents is 10-80:1. The volume ratio of the two solvents is 15-75:1. The volume ratio of the two solvents is 20-75:1. The volume ratio of the two solvents is 25-75:1.

Optionally, a concentration of polyurethane in the polyurethane solution is 0.25%-25% (w/v). Preferably, a concentration of polyurethane is 0.5%-20% (w/v). Preferably, a concentration of polyurethane is 0.5%-15% (w/v). Preferably, a concentration of polyurethane is 0.5%-10% (w/v). Preferably, a concentration of polyurethane is 5%-25% (w/v). Preferably, a concentration of polyurethane is 1%-25% (w/v). Preferably, a concentration of polyurethane is 2%-25% (w/v). Preferably, a concentration of polyurethane is 3%-25% (w/v). Preferably, a concentration of polyurethane is 4%-25% (w/v). Preferably, a concentration of polyurethane is 5%-20% (w/v). Preferably, a concentration of polyurethane is 5%-15% (w/v). Preferably, a concentration of polyurethane is 8% - 20% (w/v). Preferably, a concentration of polyurethane is 10%-20% (w/v).

Optionally, the solid particles are at least one of sodium chloride, potassium chloride, or sucrose.

Optionally, a number-average particle size of the solid particles is 5-500 micrometers. Preferably, a number-average particle size of the solid particles is 5-450 micrometers. Preferably, a number-average particle size of the solid particles is 5-400 micrometers. Preferably, a number-average particle size of the solid particles is 5-350 micrometers. Preferably, a number-average particle size of the solid particles is 5-300 micrometers. Preferably, a number-average particle size of the solid particles is 5-250 micrometers.

Optionally, in the polyurethane mixture containing solid particles, a mass fraction of the solid particles is 50%-80%. Preferably, a mass fraction of the solid particles is 55%-80%. Preferably, a mass fraction of the solid particles is 60%-80%. Preferably, a mass fraction of the solid particles is 60%-75%.

Optionally, the mold includes a plurality of mold cavities, each mold cavity corresponds to one protrusion, and the distribution of the mold cavities corresponds to the distribution of the protrusions.

Optionally, a curing temperature for the protrusions and the flexible substrate is 20-90°C. Preferably, curing temperature for the protrusions and the flexible substrate is 20-70°C. Preferably, a curing temperature for the protrusions and the flexible substrate is 20-50°C.

Optionally, the preparation of the protrusions and the flexible substrate further includes a post-treatment after curing, the post-treatment including: placing the protrusions and the flexible substrate in water at 20-40°C for at least 1 hour.

Optionally, the post-treatment includes: placing the protrusions and the flexible substrate in water at 20-40°C for 2-4 hours.

Optionally, the preparation of the protrusions and the flexible substrate further includes a pre-curing step before the curing step, the pre-curing conditions is: a pre-curing temperature of 20-70°C, a pre-curing time of 20-60 minutes.

Optionally, the pre-curing conditions are: the pre-curing temperature 30-60°C, the pre-curing time of 20-40 minutes.

Optionally, the washing of the protrusions and the flexible substrate includes: placing the protrusions in a washing agent and letting the protrusions stand for at least 4 hours.

Optionally, the washing of the protrusions and the flexible substrate includes: placing the protrusions in a washing agent and letting the protrusions stand for 4-12 hours.

Optionally, the washing of the protrusions and the flexible substrate includes: treating the protrusions in a washing agent under agitation for at least 4 hours.

Optionally, the washing of the protrusions and the flexible substrate includes: treating the protrusions in a washing agent under agitation for 4-12 hours.

Optionally, the polyurethane solution in contact with the protrusions is at room temperature or has been preheated, and a preheating temperature of the polyurethane solution is 30-70°C. Preferably, a preheating temperature of the polyurethane solution is 40-70°C. Preferably, a preheating temperature of the polyurethane solution is 50-70°C.

Optionally, the preparation of the protrusions and the flexible substrate further includes an initial curing step before the pre-curing step, and the initial curing is performed by one of the following methods:
a) blowing hot air over a surface of the polyurethane mixture containing solid particles;
b) spraying hot steam over the surface of the polyurethane mixture containing solid particles;
c) adding water, ethanol, or isopropanol dropwise into the polyurethane mixture containing solid particles;

in step a), a hot air temperature is 40-100°C, and a blowing time is 0.5-2 minutes;
in step b), a steam temperature is 40-100°C, and a spraying time is 0.5-2 minutes;
in step c), 1-2.5 mL is added.

This disclosure further provides prosthetic heart valve, including:
a stent, having a blood flow channel inside, the stent having an inflow side and an opposite outflow side, and the inflow side having a grid structure;
one or more leaflets;
a perivalvular leakage preventing component connected to the inflow side,
wherein the flexible substrate of the perivalvular leakage preventing component is located inside the stent, and protrusions are embedded in the grid structure; or the flexible substrate of the perivalvular leakage preventing component is located outside the stent, and protrusions are located on the side of the flexible substrate facing away from the stent.

In the aforementioned configuration, the protrusions act as a sealing block or a sealing assembly to reduce perivalvular leakage. The flexible substrate is disposed inside the stent to act as an inner skirt. The flexible substrate is disposed outside the stent to act as an outer skirt. The outer skirt cooperates with the sealing block to form a perivalvular leakage preventing structure.

The adaptive perivalvular leakage preventing component provided by this disclosure achieves an effective sealing competence without increasing an entry size of a delivery system, while exhibiting excellent self-adaptability. During valve crimping and loading, the valve skirt is protected against folding, uneven stress, and high friction with the delivery system.

### BRIEF DESCRIPTION OF DRAWINGS

FIG. 1 is a schematic view of a prosthetic implant provided by the present disclosure;
FIG. 2 is a schematic structural view of a prosthetic implant according to an embodiment of the present disclosure;
FIG. 3 is a schematic structural view of the prosthetic implant of FIG. 2 with the stent omitted;
FIG. 4 is a structural schematic view of a prosthetic implant according to an embodiment of the present disclosure;
FIGS. 5-7 are schematic views showing a sealing assembly protruding outward from a stent according to some embodiments of the present disclosure;
FIGS. 8-8a are schematic structural views showing the arrangement of sealing assemblies according to some embodiments of the present disclosure;
FIGS. 9-11 are partial schematic structural views showing an outer skirt covering a grid according to some embodiments of the present disclosure;
FIGS. 11a-11c are schematic views of a pouch structure between an outer skirt and an inner skirt according to some embodiments of the present disclosure;
FIG. 12 is a schematic view showing the inner skirt, the outer skirt, and a plurality of leaflets of FIG. 9 flattened out;
FIG. 13 is a schematic view showing a first sheet and a second sheet of FIG. 12 as a one-piece structure;
FIGS. 14-17 are schematic structural views illustrating configurations of an inner skirt and an outer skirt according to some embodiments of the present disclosure;
FIG. 17a is a schematic view showing a sealing assembly located on an outer side of an outer skirt according to some embodiments of the present disclosure;
FIG. 18 is a schematic structural view of a skirt with a serrated inflow edge according to an embodiment of the present disclosure;
FIGS. 19-21 are schematic views of a sealing assembly being an annular member according to some embodiments of the present disclosure;
FIG. 22 is a cross-sectional view taken along a radial direction of the annular member in FIG. 19;
FIG. 22a is a schematic view showing sealing blocks arranged spaced apart from each other according to some embodiments of the present disclosure;
FIG. 22b is a schematic view showing sealing blocks arranged continuously according to some embodiments of the present disclosure;
FIGS. 23-25 are schematic views of sealing blocks viewed from a radial direction of a stent according to some embodiments of the present disclosure;
FIG. 25a is a schematic view showing sealing blocks arranged with their wider sides facing each other according to some embodiments of the present disclosure;
FIGS. 26-27a are cross-sectional views of sealing blocks according to some embodiments of the present disclosure;
FIGS. 28-30 are schematic views showing installation positions of a sealing assembly according to the present disclosure;
FIG. 31 is a schematic view showing wound sutures and a wound reinforcement member according to an embodiment of the present disclosure;
FIG. 31a is a schematic view of a localized elongated inner skirt according to an embodiment of the present disclosure;
FIG. 32 is a schematic view showing a reinforcement wire in FIG. 31 extending circumferentially along the stent;
FIG. 33a is a structural schematic view showing a fully reinforced inner skirt according to an embodiment of the present disclosure;
FIG. 33b is a structural schematic view showing of a partial reinforced inner skirt according to an embodiment of the present disclosure;
FIG. 33c is a structural schematic view showing a reinforced outer skirt and a reinforced sealing assembly according to an embodiment of the present disclosure;
FIG. 33d is a schematic structural view showing integrally reinforced inner and outer skirts;
FIG. 34a is a schematic structural view showing inner and outer skirts integrally mounted on a stent (reinforcement component located on the outer side of the outer skirt);
FIG. 34b is a schematic structural view showing inner and outer skirts integrally mounted on a stent (reinforcement component located on both inner and outer sides of the outer skirt);

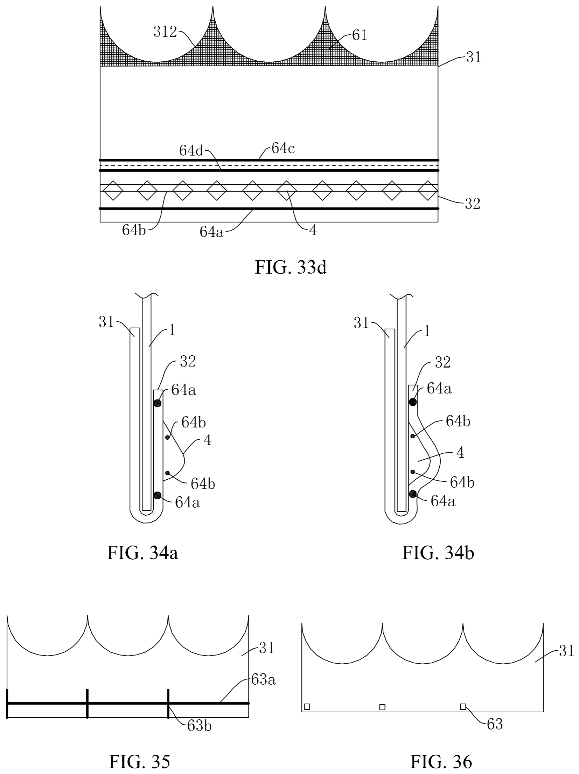

FIG. 34 is a schematic structural view showing the reinforcement of a skirt with a one-piece structure according to an embodiment of the present disclosure;
FIGS. 35-37 are schematic views showing a partial reinforced skirt according to some embodiments of the present disclosure;

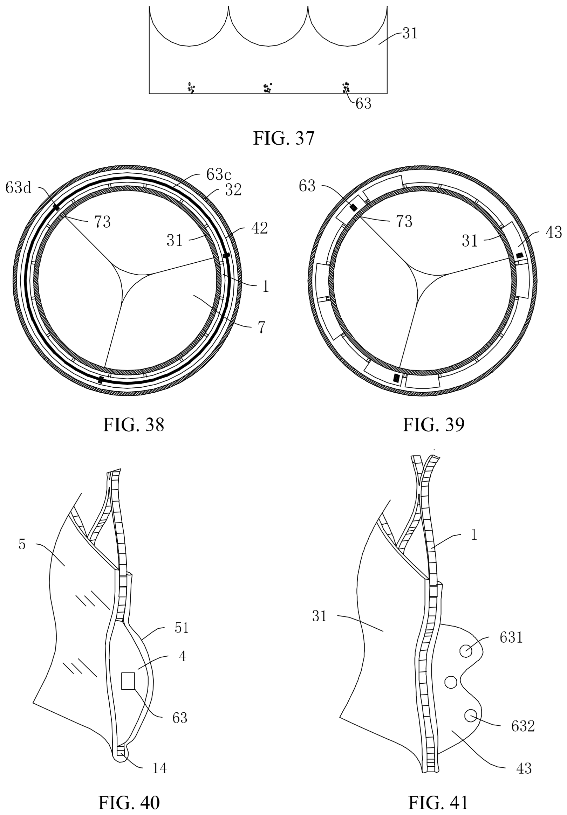

FIGS. 38-41 are schematic views showing the distribution of radiopaque markers according to the present disclosure;

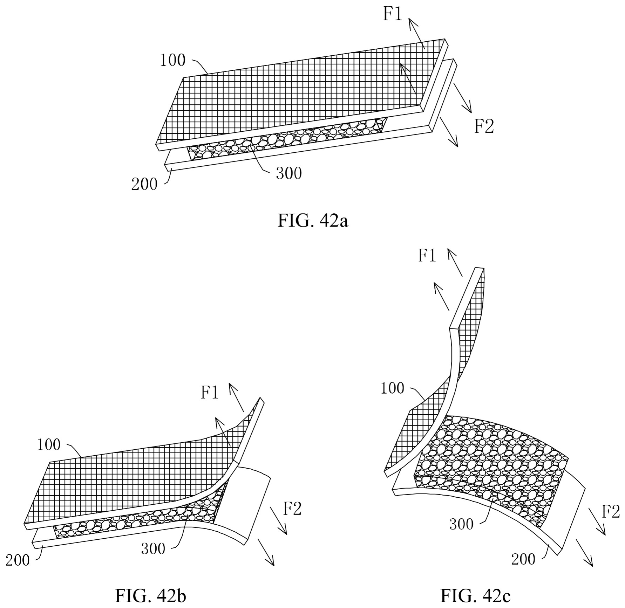

FIGS. 42a-42c are schematic views of a peel test process;

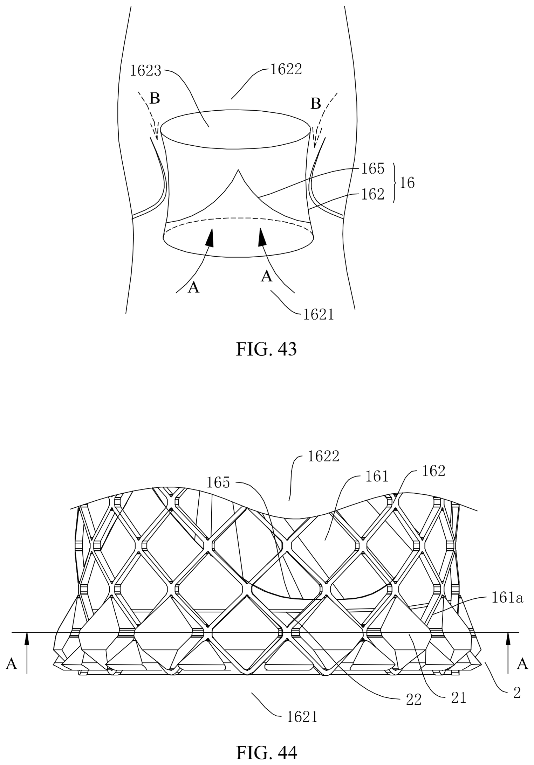

FIG. 43 is a schematic view of a prosthetic implant after in vivo deployment;
FIG. 44 is a schematic structural view of a perivalvular leakage preventing device embedded in a prosthetic implant according to the present disclosure;

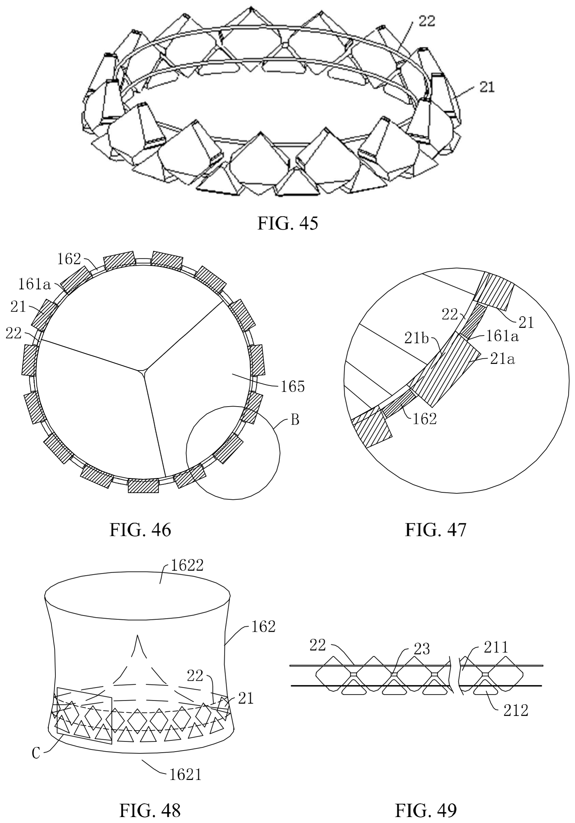

FIG. 45 is a structural schematic view of the perivalvular leakage preventing device in FIG. 44;
FIG. 46 is a cross-sectional view taken along direction A-A in FIG. 44;
FIG. 47 is a partial enlarged view of part B in FIG. 46;
FIG. 48 is a schematic view showing sealing blocks of adjacent rows interleaved with each other according to the present disclosure;
FIG. 49 is a schematic structural view of the perivalvular leakage preventing device of FIG. 48 in an unfolded state;

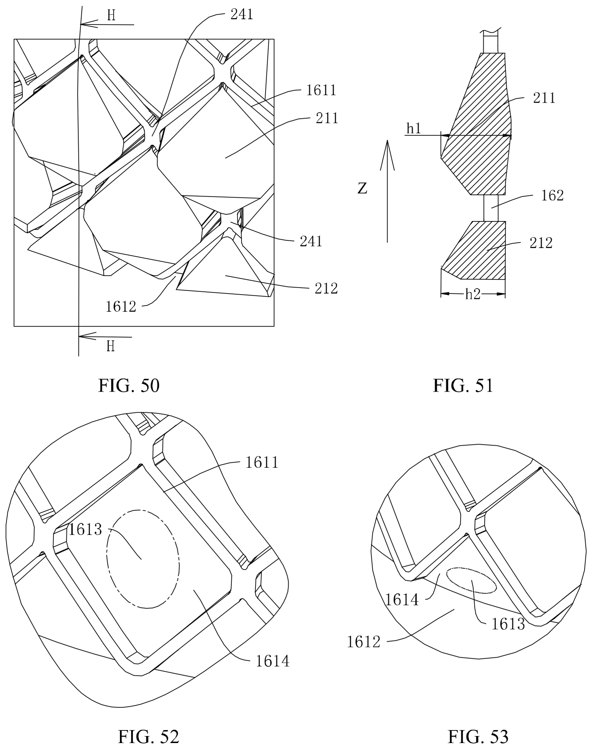

FIG. 50 is a partial enlarged view of part C in FIG. 48;
FIG. 51 is a partial cross-sectional view taken along direction H-H in FIG. 50;
FIG. 52 is a schematic structural view of a closed-grid in FIG. 50;
FIG. 53 is a schematic structural view of an open-grid in FIG. 50;

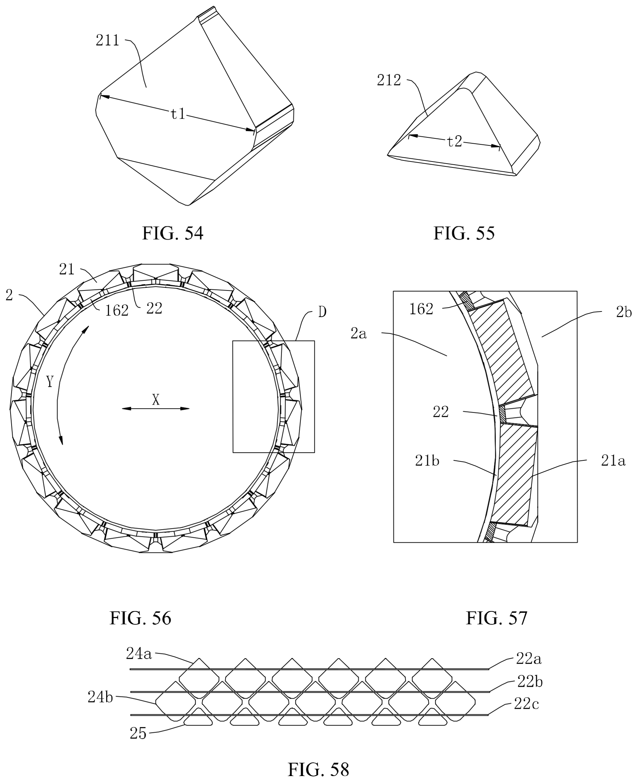

FIG. 54 is a schematic structural view of a sealing block to filling a closed-grid in FIG. 50;
FIG. 55 is a schematic structural view of a sealing block filling an open-grid in FIG. 50;
FIG. 56 is a schematic view of the perivalvular leakage preventing device of FIG. 48 from another perspective;
FIG. 57 is a partial enlarged view of part D in FIG. 56;
FIG. 58 is a schematic view of the perivalvular leakage preventing device of FIG. 56 in an unfolded state;

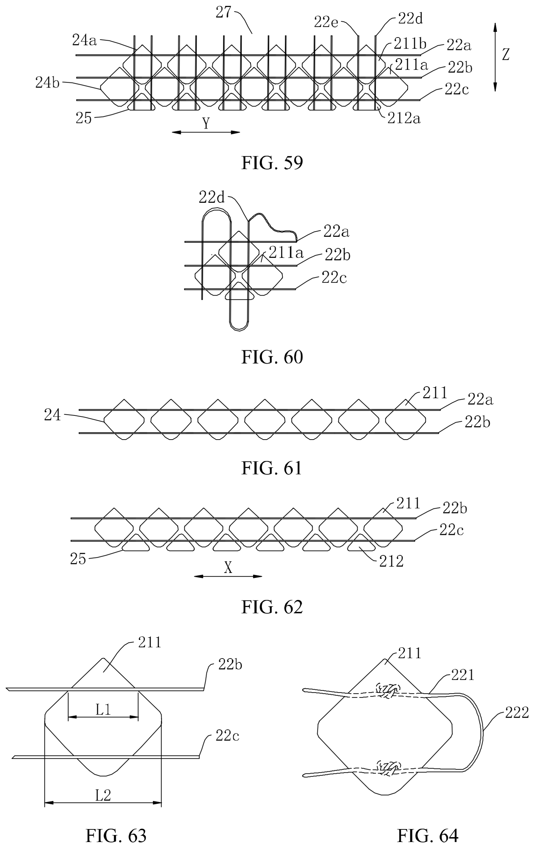

FIG. 59 is a schematic view showing pulling wires of a perivalvular leakage preventing device arranged in an interlaced pattern;
FIG. 60 is a schematic view showing pulling wires of a perivalvular leakage preventing device arranged in a meandering pattern;
FIG. 61 is a schematic view showing sealing blocks arranged in a single row in the perivalvular leakage preventing device of the present disclosure;
FIG. 62 is a schematic view showing sealing blocks arranged in two rows in the perivalvular leakage preventing device of the present disclosure;
FIG. 63 is a schematic view showing a connection between sealing blocks and pulling wires in FIG. 62.
FIG. 64 is a schematic view showing a knot embedded within a sealing block in the perivalvular leakage preventing device of the present disclosure;

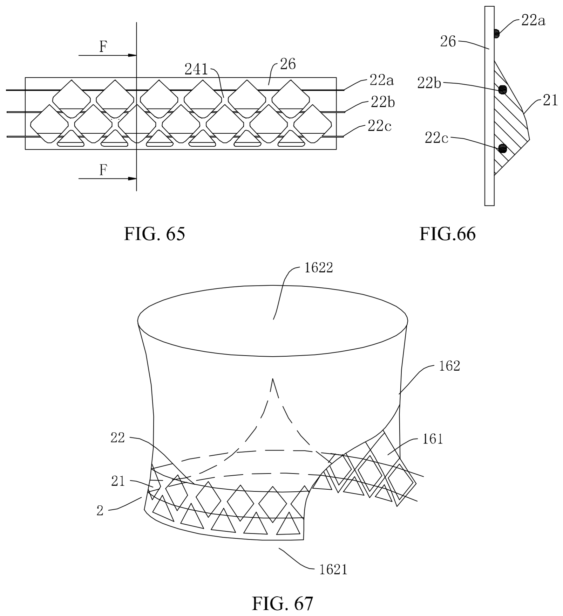

FIG. 65 is a schematic view of a perivalvular leakage preventing device with an inner membrane according to the present disclosure;
FIG. 66 is a cross-sectional view taken along direction F-F in FIG. 65;
FIG. 67 is a schematic view of a prosthetic implant provided by the present disclosure;

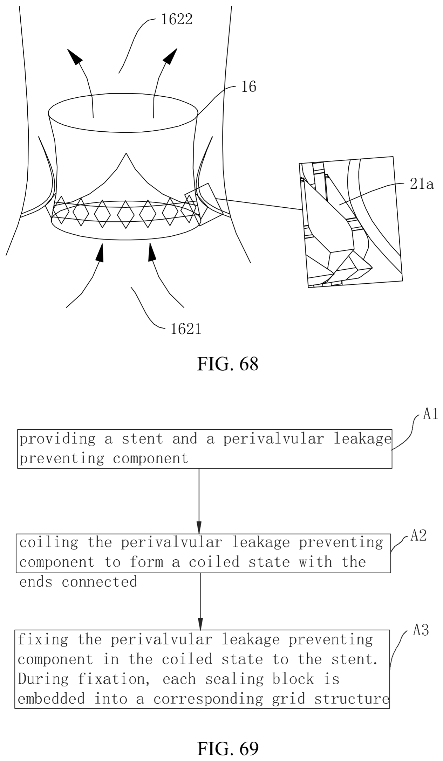

FIG. 68 is a schematic view of the prosthetic implant of FIG. 67 after in vivo deployment;
FIG. 69 is a flowchart of a method for processing a prosthetic implant provided by the present disclosure;

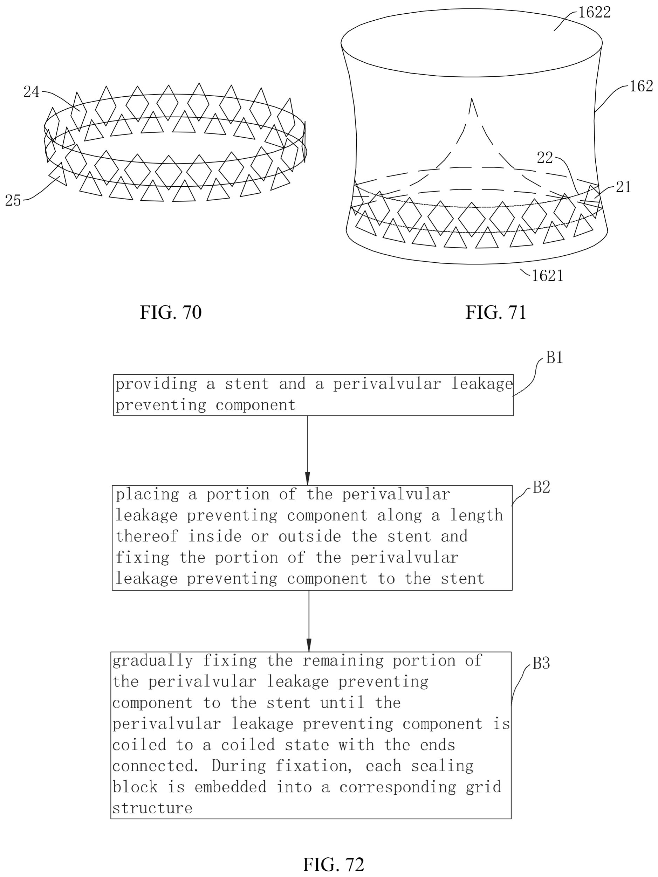

FIG. 70 is a schematic view of step A2 in the method for producing a prosthetic implant provided by the present disclosure;
FIG. 71 is a schematic view of step A3 in the method for producing a prosthetic implant provided by the present disclosure;
FIG. 72 is a flowchart of another method for producing a prosthetic implant provided by the present disclosure;

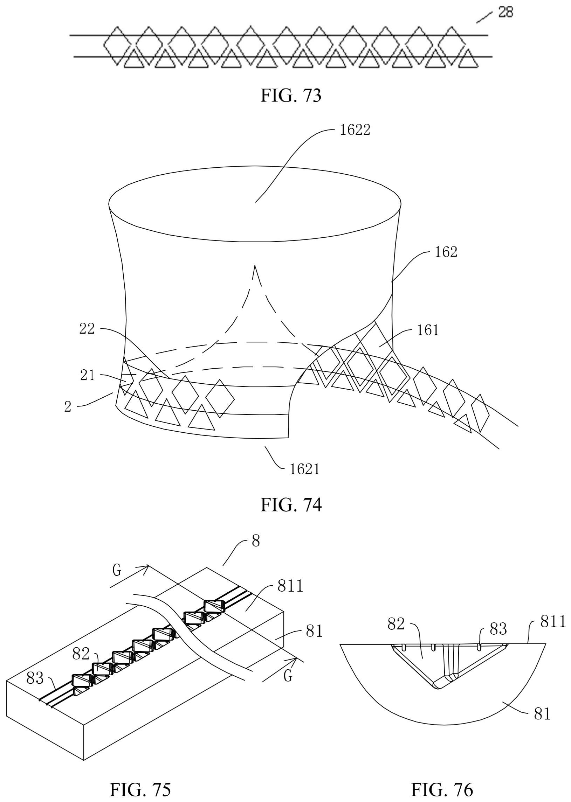

FIG. 73 is a schematic view of step B1 in the another method for processing a prosthetic implant provided by the present disclosure;
FIG. 74 is a schematic view of step B2 in the another method for processing a prosthetic implant provided by the present disclosure;
FIG. 75 is a structural schematic view of a mold for forming a perivalvular leakage preventing device provided by the present disclosure;
FIG. 76 is a partial cross-sectional view taken along direction G-G in FIG. 75;

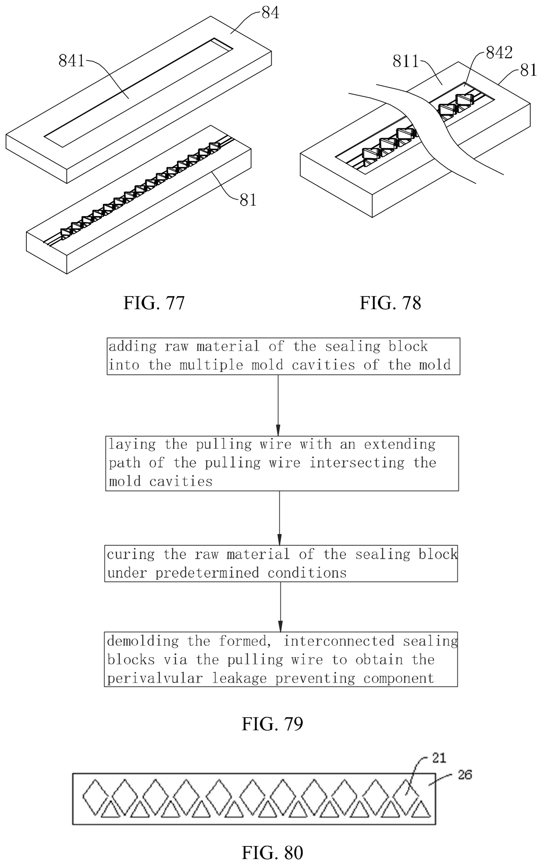

FIG. 77 is a schematic structural view of a split-type mold for forming a perivalvular leakage preventing device provided by the present disclosure;
FIG. 78 is a structural schematic view of an integrated mold for forming a perivalvular leakage preventing device provided by the present disclosure;
FIG. 79 is a flowchart of a method for producing a perivalvular leakage preventing device using a mold provided by the present disclosure;
FIG. 80 is a schematic structural view of a perivalvular leakage preventing device according to another embodiment provided by the present disclosure;

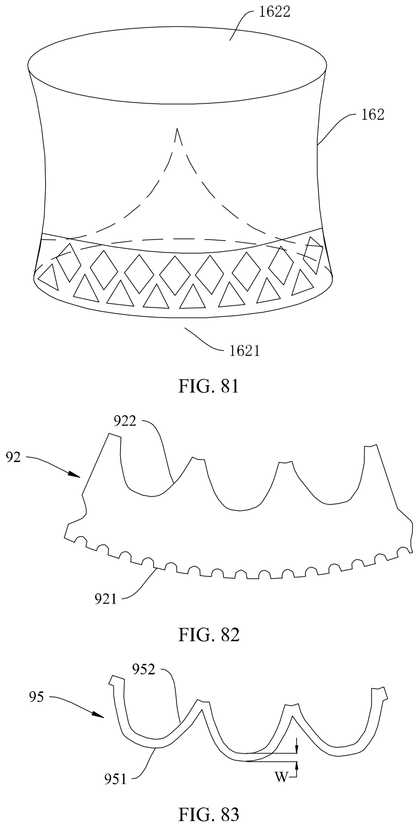

FIG. 81 is a schematic structural view of the perivalvular leakage preventing device of FIG. 80 embedded in a stent;
FIG. 82 is a schematic view of a flexible substrate according to one embodiment of a perivalvular leakage preventing skirt of the present disclosure;
FIG. 83 is a schematic view of a reinforcement component according to one embodiment of a perivalvular leakage preventing skirt of the present disclosure;

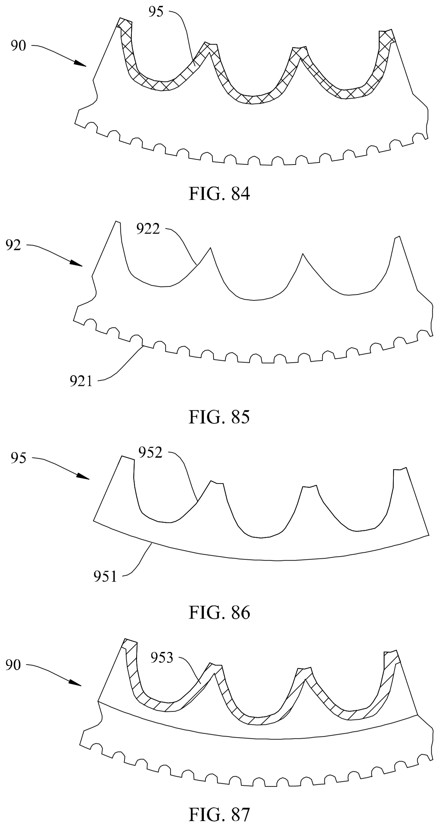

FIG. 84 is a schematic view after connecting the structures shown in FIG. 82 and FIG. 83.
FIG. 85 is a schematic view of a flexible substrate according to one embodiment of a perivalvular leakage preventing skirt of the present disclosure;
FIG. 86 is a schematic view of a reinforcement component according to one embodiment of a perivalvular leakage preventing skirt of the present disclosure;
FIG. 87 is a schematic view showing the structures shown in FIG. 85 and FIG. 86 being connected;

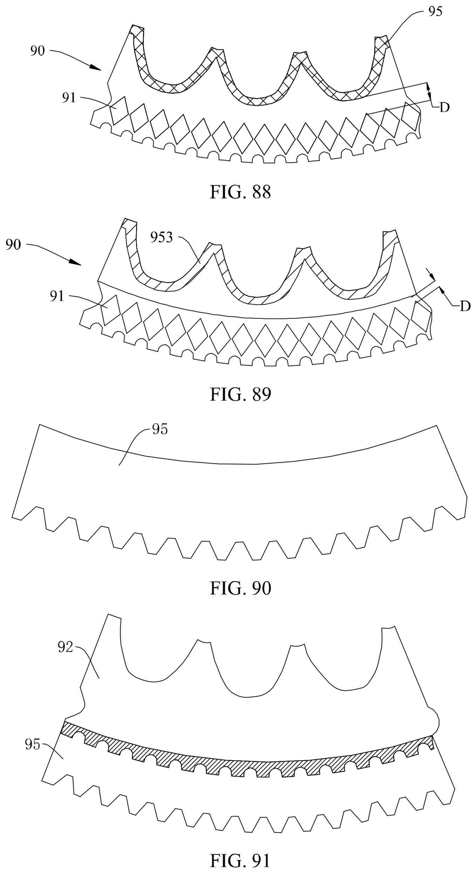

FIG. 88 is a schematic view showing the structure shown in FIG. 84 being connected with a sealing assembly;
FIG. 89 is a schematic view showing the structure shown in FIG. 87 being connected with a sealing assembly;
FIG. 90 is a schematic view of a reinforcement component according to one embodiment of a perivalvular leakage preventing skirt of the present disclosure;
FIG. 91 is a schematic view according to an embodiment of a perivalvular leakage preventing skirt (with the sealing assembly omitted) of the present disclosure;

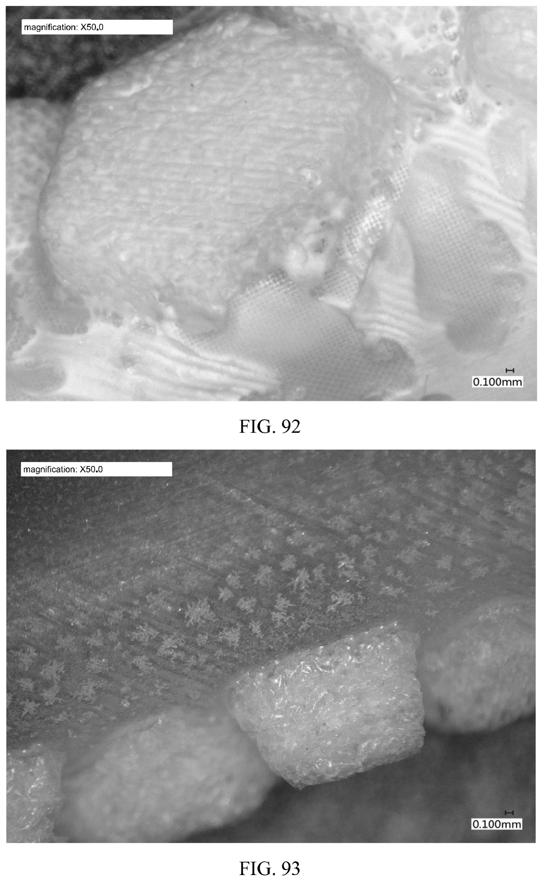

FIG. 92 is a schematic view of a perivalvular leakage preventing component produced by Comparative Example 1;
FIG. 93 is a schematic view of a perivalvular leakage preventing component produced by embodiment 1 of the self-adaptive perivalvular leakage preventing component of the present disclosure;

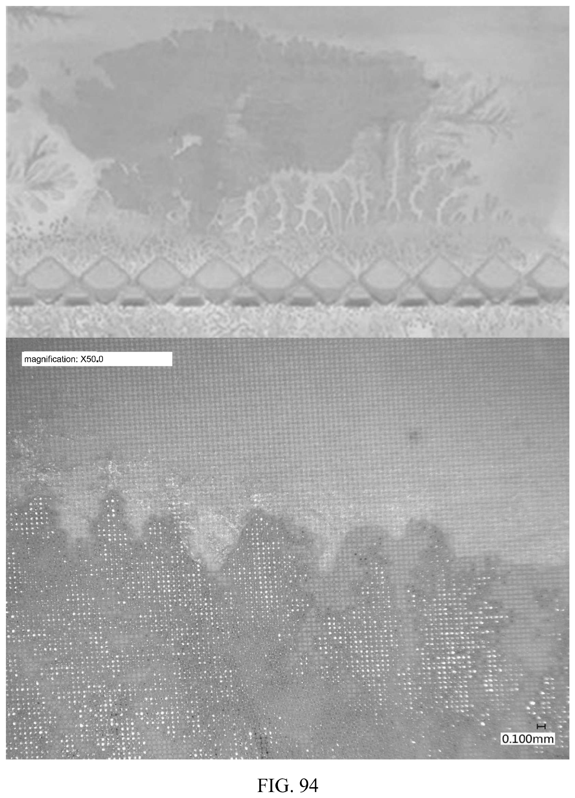

FIG. 94 is a schematic view of a perivalvular leakage preventing component produced by Comparative Example 1 (the lower part is a partial enlarged view);

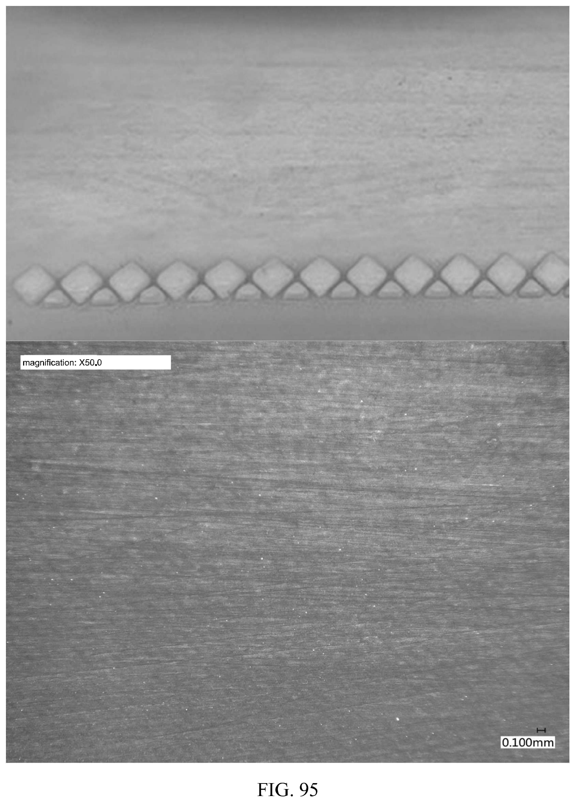

FIG. 95 is a schematic view of a perivalvular leakage preventing component produced in Example 7 (the lower part is a partial enlarged view);

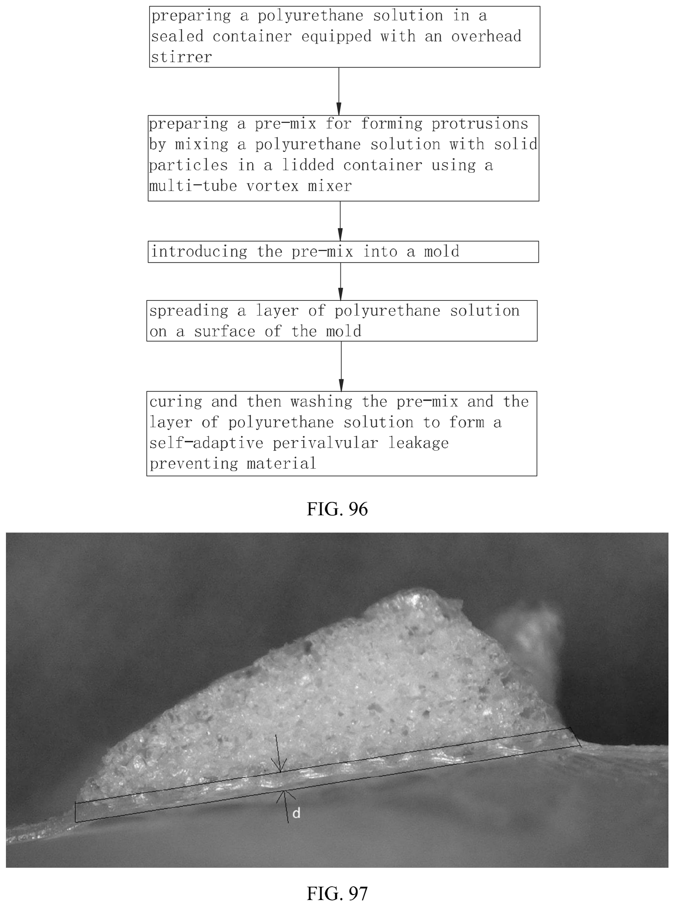

FIG. 96 is a flowchart of producing the perivalvular leakage preventing component in Example 7;
FIG. 97 is a micrograph of the perivalvular leakage preventing component produced in Example 7;

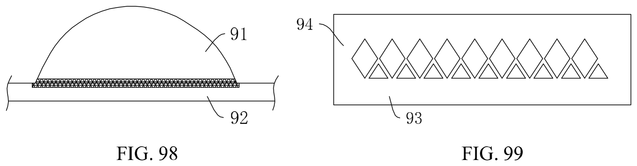

FIG. 98 is a schematic view of a bonding region of a perivalvular leakage preventing component;
FIG. 99 is a schematic view of a mold.

### List of reference symbols in the Drawings

1, stent; 11, stent grid; 111, open-grid; 112, closed-grid; 112a, closed-grid; 112b, closed-grid; 12, strut; 121, grid node; 13, blood flow channel; 131, inflow side; 132, outflow side; 14, leg; 15, waist portion;16, prosthetic implant; 161, grid structure; 161a, grid structure; 1611, closed-grid; 1612, open-grid; 1613, central region; 1614, edge region; 162, stent; 1621, inflow side; 1622, outflow side; 1623, blood flow channel; 165, leaflet;
2, perivalvular leakage preventing device; 2a, inner side; 2b, outer side; 21, sealing block; 211, sealing block; 211a, sealing block; 211b, sealing block; 212, sealing block; 212a, sealing block; 212b, sealing block; 22, pulling wire; 22a, pulling wire; 22b, pulling wire; 22c, pulling wire; 22d, pulling wire; 22e, pulling wire; 221, knot; 222, meandering structure; 23, connecting strip; 24, sealing block ring; 24a, sealing block ring; 24b, sealing block ring; 241, spaced portion; 25, sealing block ring; 26, inner membrane; 27, sealing block net; 28, sealing block strip;
3, skirt; 31, inner skirt; 311, inflow edge; 312, outflow edge; 313, tubular structure; 32, outer skirt; 321, tubular structure; 322, outflow edge; 323, inflow edge; 33, accommodating region; 33a, pouch structure; 331, opening;
4, sealing assembly; 41a, sealing assembly; 41b, sealing assembly; 42, annular member; 43, sealing block; 43a, sealing block; 431, wider side; 432, narrower side; 433, inner side; 434, outer side; 44, spacing region; 45, peak; 46, valley; 47, connection region; 48, embedding region;
5, first sheet; 51, second sheet;
6, reinforcement member; 61, reinforcement fabric; 62, suture; 63, radiopaque marker; 63a, radiopaque marker; 63b, radiopaque marker; 63c, radiopaque marker; 63d, radiopaque marker; 631, first marker; 632, second marker; 64, reinforcement wire; 64a, reinforcement wire; 64b, reinforcement wire; 64c, reinforcement wire; 65, reinforcement piece;
7, leaflet; 71, fixed edge; 72, free edge; 73, joint portion;
8, mold; 81, main body; 811, working surface; 82, mold cavity; 83, wire groove; 84, forming frame; 841, hollowed region; 842, recessed region;
90, perivalvular leakage preventing skirt; 91, sealing assembly; 92, flexible substrate; 921, first edge; 922, second edge; 93, mold; 9, mold cavity; 95, reinforcement component; 951, third edge; 952, fourth edge; 953, extension part;
100, PET film; 200, PU film; 300, interlayer.

### DESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this disclosure.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

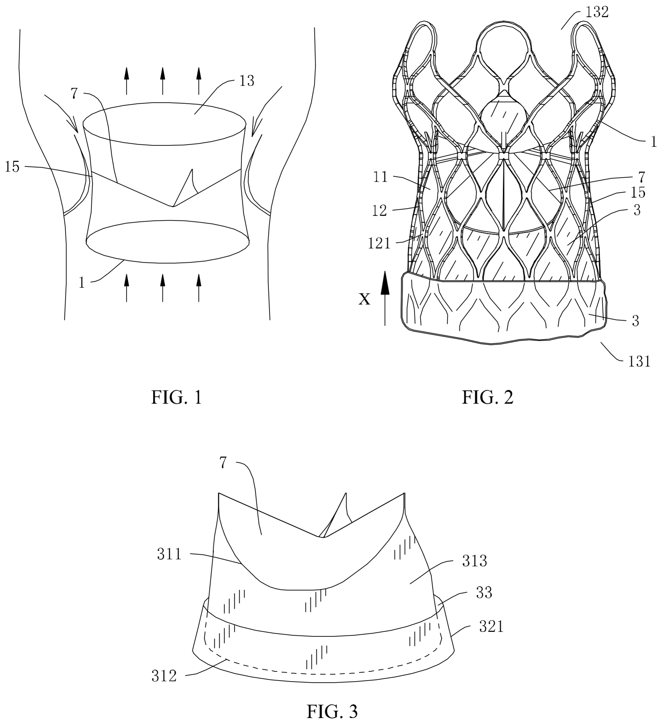

Referring to FIG. 1 to FIG. 6, to enhance the perivalvular leakage preventing effect of a skirt and improve its self-adaptive capability, one embodiment of the present disclosure provides a prosthetic implant. The prosthetic implant can be delivered to a lesion site within the body via either surgical or transcatheter interventional approaches. During transcatheter intervention, the prosthetic implant can be loaded into a distal end of a catheter assembly and delivered and deployed in the body through the operation of a control handle. The prosthetic implant described below takes a prosthetic heart valve as an example. Once deployed in the body, the prosthetic heart valve can replace the native diseased valve, restore normal valve function, and achieve the therapeutic objective.

An embodiment of the present disclosure provides a prosthetic implant, including:
a stent 1, being a radially deformable tubular structure, and the stent 1 having a through blood flow channel 13;
a plurality of leaflets 7, each leaflet having a fixed edge 71 and an opposite free edge 72, wherein the fixed edge is secured to the stent, and the free edge is configured to control the blood flow channel 13;
a perivalvular leakage preventing structure arranged circumferentially around the stent, the perivalvular leakage preventing structure being:
   a sealing assembly, at least a part of which protrudes radially outward from the stent and is made of a deformable structure; and/or
   an outer skirt wrapped around an outer periphery of the stent.

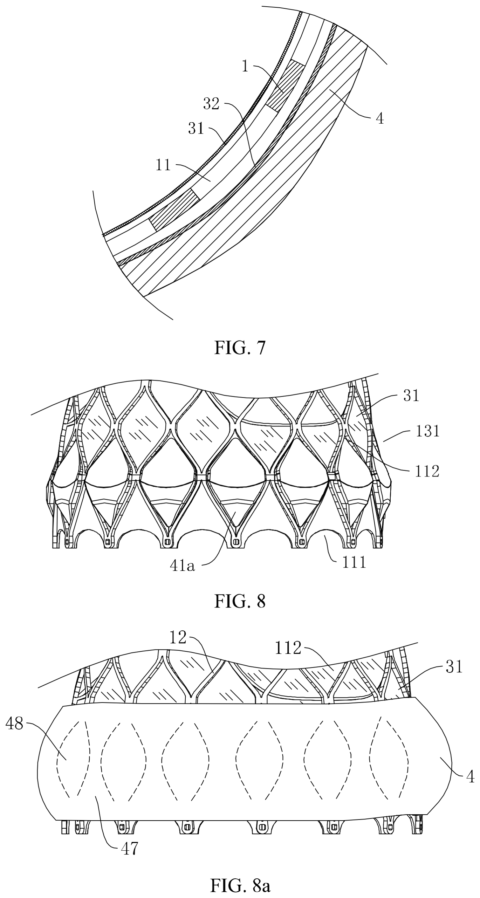

Wherein, the stent 1 has a corresponding axial direction (direction of an axis), a radial direction perpendicular to the axial direction, and a circumferential direction around the axis. The stent 1 is provided with stent grids 11, which can be understood a sidewall of the tubular structure having hollowed regions. Edges of the hollowed regions are struts 12 that form the stent grids, and intersections of adjacent struts 12 are grid nodes 121. A size and specific shape of the stent grids are not strictly limited and can be regular shapes such as rhombic, fishbone-shaped, or hexagonal. The stent can be radially compressed to be loaded in an interventional delivery system. During radially compression, the grids and the struts deform accordingly. Along the axial direction of the stent (indicated by direction X in FIG. 1), and according to the normal direction of blood flow in body, two ends of the stent are designated as an inflow side 131 and an outflow side 132, respectively. The stent 1 has multiple layers of stent grids 11, with grids in adjacent layers distributed in a staggered pattern, as shown in FIG. 8. The stent grids at the inflow side of the stent include open-grids 111 and closed-grids 112. The stent itself may be manufactured by tube cutting or weaving. Depending on its in-body deployment method, the stent is made from shape-memory materials (e.g., Nitinol) to accommodate self-expandable deployment, or stainless steel to accommodate balloon-expandable deployment.

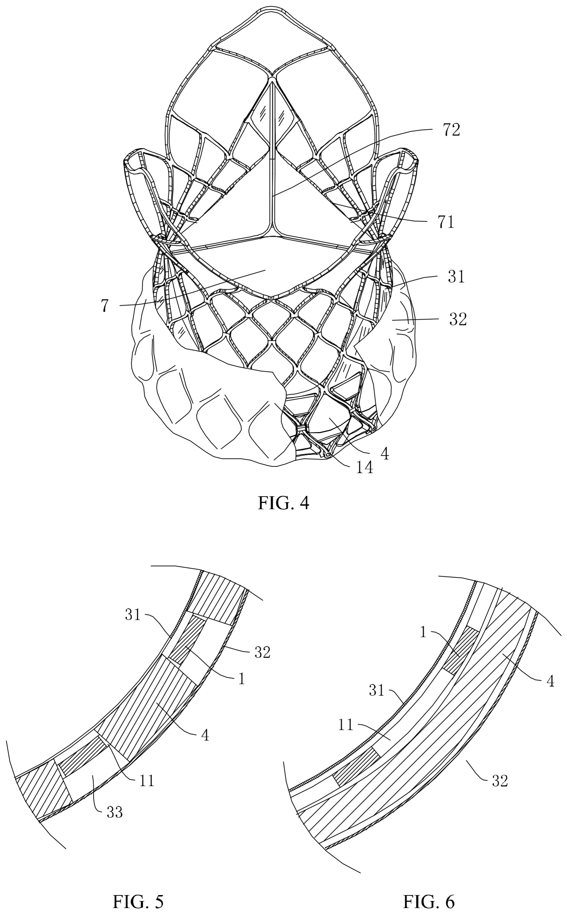

An interior of the stent 1 defines the through blood flow channel 13. The fixed edges 71 of the leaflets 7 are sutured to the stent 1. The free edges 72 of the plurality of leaflets coapt to close the blood flow channel 13. The leaflets may be made from biological materials or synthetic materials. The perivalvular leakage preventing structure has various configurations, specifically categorized as:
First Configuration: The perivalvular leakage preventing structure is an outer skirt wrapped around the outer periphery of the stent (excluding any separate sealing assembly), as shown, for example, in FIG. 2;
Second Configuration: The perivalvular leakage preventing structure is a sealing assembly that protrudes radially outward from the stent (excluding an outer skirt), as shown, for example, in FIG. 8;
Third Configuration: The perivalvular leakage preventing structure includes both an outer skirt and a sealing assembly, as shown, for example, in FIG. 4.

Some embodiments below will elaborate further on these three configurations.

In some embodiments, the prosthetic implant may further include a skirt 3. The skirt 3 includes an inner skirt 31 located radially inside the stent and/or an outer skirt 32 located radially outside the stent. The skirts are defined based on the radial position of the stent: the one located radially inside the stent is called the inner skirt, and the one located radially outside is called the outer skirt. The prosthetic implant may have only the inner skirt, or only the outer skirt, or both skirts (the inner skirt and the outer skirt). FIG. 3 and FIG. 4 illustrate configurations where the prosthetic implant has both skirts. The inner skirt 31 is sutured and joined to the fixed edges 71 of the plurality of leaflets. After manufacturing, the inner skirt 31 and the outer skirt 32 respectively form a tubular structure 313 and a tubular structure 321 adapted to conform to the stent.

An accommodating region 33, which is a radial gap between the two skirts, is defined between the tubular structure 313 of the inner skirt and the tubular structure 321 of the outer skirt. The accommodating region 33 extends circumferentially around the stent. A portion of the stent is disposed within the accommodating region 33. The various components - the skirts, the sealing assembly, and the stent - can be connected to each other by suturing, bonding, or other connecting elements, depending on the material properties. In some embodiments, the outer skirt is designed to be elastic, such that it conforms tightly to the outer periphery of the stent. When combined with a sealing assembly, the outer skirt may partially or fully envelop the sealing assembly on its outer side. In cases of partial envelopment, the outflow edge of the outer skirt snugly surrounds an outer periphery of the sealing assembly. In some embodiments, the outer periphery of the sealing assembly has undulating portions (in the radial direction). The outflow edge of the outer skirt may align with relatively protruding portions or relatively recessed portions of the sealing assembly. This is described further below in conjunction with the figures.

The sealing assembly 4 adopts a deformable structure and is arranged circumferentially along the stent. At least a part of the sealing assembly 4 protrudes radially outward beyond the stent 1. Here, "protrudes outward" can be interpreted in several ways: it can be understood that, from a cross-sectional view at the location of the sealing member, only a part of the sealing assembly protrudes beyond the stent, with the entire the sealing assembly disposed within the accommodating region between the two skirts (as shown in FIG. 5); or it can be understood that the entire sealing assembly is located outside the stent but still within the accommodating region between the two skirts (as shown in FIG. 6); or it can be understood that the entire sealing assembly is located outside the stent and outside the accommodating region between the two skirts (as shown in FIG. 7). In all three cases, the sealing assembly extends outward beyond the stent, enabling the sealing assembly to perfectly conform to the annulus after deforming upon blood absorption (i.e., self-expanding), thereby achieving the function of preventing perivalvular leakage.

The sealing assembly and the two skirts may be connected in various manners; several exemplary embodiments are described below.

First, the sealing assembly is adhesively bonded to at least one of the inner skirt and the outer skirt.

Second, the sealing assembly and at least one of the inner skirt and the outer skirt are formed as a one-piece structure. This not only omits the process of adhesively bonding the sealing assembly to the skirt but also enhances the connection strength between the two.

Second, the sealing assembly 4 is an independent structure, encapsulated within the radial gap (i.e., the accommodating region 33) between the two skirts, and is not directly connected to either of the two skirts.

All the aforementioned connection manners between the sealing assembly and the two skirts enable the inflow end of the stent to achieve a self-expanding effect, providing adaptive occlusion and reducing perivalvular leakage.

Some embodiments of the present disclosure further provide a prosthetic implant, including a stent 1, a plurality of leaflets 7, a skirt 3, and a sealing assembly 4, which may incorporate the various embodiments described above. Wherein the skirt includes an inner skirt 31 located on the inside of the stent. The sealing assembly is generally continuously circumferentially distributed, and disposed around the outer periphery of the stent. The sealing assembly is fixed to the inner skirt through the hollowed portions of the stent grids and wraps around at least some of the struts forming the stent grid.

Referring to FIG. 8a, only the inner skirt 31 located on the radial inner side of the stent is connected to the stent 1. The sealing assembly is generally ring-shaped, sleeving over the inflow side of the stent from the outside of the stent. The sealing assembly 4 includes embedding regions 48 and connecting regions 47 connecting the embedding regions. A portion of the sealing assembly, namely the embedding regions 48, may be embedded into the hollowed portions of the stent grids 11 and may be fixed to the inner skirt by adhesion or suturing. The connecting regions 47 of the sealing assembly wrap around at least some of the struts 12 forming the grid stent.

Preferably, edges of the connecting regions 47 may be configured in a serrated shape matching the stent grid, thereby preventing multi-layer stacking of the connecting regions in a compressed state of the stent and avoiding a detrimental effect on the post-compression radial profile.

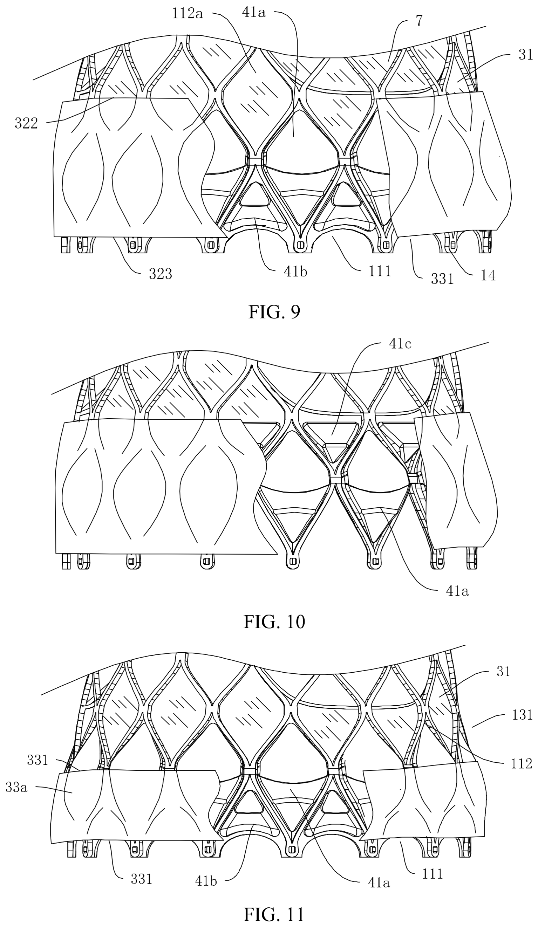

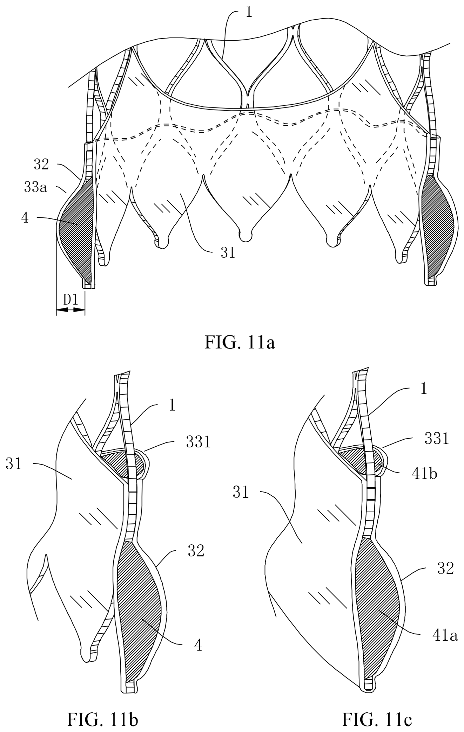

In some other embodiments, the skirt includes an inner skirt 31 located on the inner side of the stent and an outer skirt 32 located on the outer side of the stent. The outer skirt 32 is disposed on the inflow side 131, and an axial span of the outer skirt corresponds to 0.5 to 2 grids (referenced to closed-grids). Overall, the outer skirt circumferentially covers 0.5 to 2 layers of grids. For example, in FIG. 9 and FIG. 10, the outer skirt covers only one closed-grid 112 along the axis direction (i.e., circumferentially covers one layer of closed-grids), but also simultaneously covers open-grids 111. Correspondingly, the sealing assembly 4 may be disposed only in one layer of closed-grids (e.g., the sealing assembly 41a in FIG. 10), or simultaneously in two adjacent layers of closed-grids (e.g., the sealing assemblies 41a and 41c in FIG. 10), or simultaneously in both closed-grids and open-grids (e.g., the sealing assemblies 41a and 41b in FIG. 9). As another example, in FIG. 11, the outer skirt covers only 0.5 closed-grids but also covers adjacent open-grids. Correspondingly, the sealing assembly 4 may be disposed only in the open-grids 111, or in both the closed-grids 112 and the open-grids 111 (e.g., the sealing assemblies 41a and 41b in FIG. 11). Cases where the outer skirt circumferentially covers two layers of closed-grids or other scenarios can follow the distribution rules described above (not shown in the figures).

As shown in FIG. 9, the outer skirt has an inflow edge 323 and an outflow edge 322, facing the inflow side 131 and the outflow side 132 of the stent, respectively. The outflow edge 322 of the outer skirt is adjacent to a midpoint of the fixed edge of the leaflet and does not extend beyond the midpoint toward the outflow side.

In one embodiment, the stent generally has a waist portion 15 that matches the position of the native annulus. The stent is generally tubular. When a generatrix of the stent is a curved line, the portion with the smallest diameter is the waist portion. Certainly, the portion with the smallest diameter may be a straight tubular segment of a defined length, which also has an inflow side and an outflow side correspondingly.

As shown in FIG. 1, the waist portion 15 may be understood as the position near the middle of the stent with the smallest radial dimension, with both sides of the waist portion flaring outward. Preferably, the outflow edge 322 of the outer skirt 32 is located at the waist portion 15.

In some embodiments, as shown in FIG. 2, the stent 1 generally has the aforementioned waist portion 15. The outflow edge of the outer skirt may also be located on the inflow side of the waist portion, which may also be understood as the height of the outflow edge of the outer skirt along the X-direction in the figure being lower than the height of the waist portion. Preferably, a distance between the outflow edge 322 of the outer skirt and the waist portion 15 corresponds to 0.5 to 1 grid.

Referring to FIG. 9a, a pouch structure 33a is defined between the outer skirt 32 and the inner skirt 31. An interior of the pouch structure 33a is the accommodating region 33 described above, and the sealing assembly 4 is located within this pouch structure 33a.

Preferably, as shown in FIG. 11a, along the radial direction of the stent, the pouch structure protrudes outward beyond the outer peripheral wall of the stent, with a protruding distance D1 of 0.5-20 mm. More preferably, D1 is 1-15 mm. The protruding distance is understood as the state after the prosthetic implant is deployed, without considering the deformation of the pouch structure and the sealing assembly due to contact with surrounding tissues.

In some embodiments, the pouch structure 33a may be a fully enclosed structure or a semi-open structure. As shown in FIG. 11a, the pouch structure 33a is a fully enclosed structure, and the sealing assembly is completely enveloped within the pouch structure.

When the pouch structure 33a is a semi-open structure, the open portion may have openings 331 facing the outflow side and/or the inflow side. A plurality of openings 331 may be provided and arranged at intervals along the stent circumference. As shown in FIG. 11b, the pouch structure 33a has openings 331 facing the outflow side; as shown in FIG. 9, the pouch structure has openings 331 facing the inflow side; and as shown in FIG. 11, the pouch structure has a plurality of openings 331 facing both the inflow side and the outflow side, respectively.

The aforementioned inner skirt 31 and outer skirt 32 may be made of the same or different materials. For example, both the two skirts are made of PU; or both the two skirts are made of biological material; or the inner skirt is be made of PU while the outer skirt is made of biological material; or the inner skirt is made of biological material and the outer skirt is made of PU.

In some embodiments, at least one of the inner skirt 31 and the outer skirt 32 is made of PU. PU is thin, dense, and of high-strength, while also exhibiting a degree of elasticity. These properties address the pain point of weak suture retention between the two skirts or between the skirt and the leaflets.

Referring to FIG. 2 to FIG. 3, some embodiments of the present disclosure further provide a prosthetic implant, including:
a stent 1, being a radially deformable tubular structure, and the stent having a through blood flow channel;
a plurality of leaflets 7, each leaflet having a fixed edge and an opposite free edge, wherein the fixed edge is fixed to the stent, and the free edge is configured to control the blood flow channel;
a skirt 3, including an inner skirt 31 located on the radial inner side of the stent and an outer skirt 32 located on the radial outer side of the stent. The inner skirt and the outer skirt are a one-piece structure and are made of PU.

The inner skirt 31 and outer skirt 32 may incorporate the embodiments described above. During the delivery and implantation of the prosthetic implant via a delivery handle, the stent has a compressed state and a deployed state. Consequently, the grids deform during the transition between these two states, and depending on a shape of the grids, the deformation of the grids and surrounding struts is uneven. Since the skirt is connected to the struts around the grids, the stress generated within the skirt during grid deformation is also non-uniform, with areas of localized high stress that could even lead to skirt tearing.

The present disclosure employs PU skirts, which offer the advantages of high elongation at break and thinness, and can avoid the aforementioned risk of skirt tearing. Furthermore, based on the advantage of high elongation, PU skirts can adapt to grids of various shapes and areas, such as small-area, low-deformation, rhombic grids, or large-area, high-deformation, fishbone-shaped grids.

Some embodiments of the present disclosure further provide a prosthetic implant, including a stent 1, a plurality of leaflets 7, a skirt 3, and a sealing assembly 4, which may incorporate the various embodiments described above. The skirt includes an inner skirt 31 and an outer skirt 32, with reference to their radial position relative to the stent. The inner skirt 31 and the outer skirt 32 are both made of PU and connected to form a one-piece structure at the inflow side of the stent. The inner skirt 31 and the outer skirt 32 form a pouch structure 33a. At least a part of the stent and at least a part of the sealing assembly are located within the pouch structure. That is, as shown in FIG. 11c, the pouch structure encloses a leg 14 of the stent therein, and can either fully encapsulate the sealing assembly 41a or partially encapsulate the sealing assembly 41b.

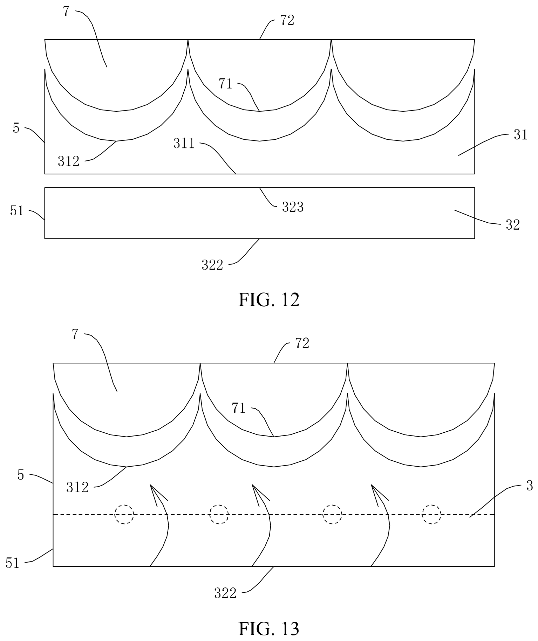

Referring to FIG. 12 (obtained by flattening the inner skirt, the outer skirt, and the plurality of leaflets of FIG. 9), the inner skirt 31 has an inflow edge 311 and an outflow edge 312, and the outer skirt 32 has an inflow edge 323 and an outflow edge 322. The outflow edge 312 of the inner skirt is sutured to the fixed edges 71 of the plurality of leaflets. The inflow edge 311 of the inner skirt and the inflow edge 323 of the outer skirt are substantially aligned. These two inflow edges may be connected either as a one-piece structure or as separate components to seal the blood flow channel. When the inner skirt and the outer skirt are separate components, after being connected, they may either envelop the stent or be spaced by the stent.

The inner skirt is formed from a first sheet 5, and the outer skirt is formed from a second sheet 51. The division of the inner skirt and the outer skirt is based on their final radial position relative to the stent after the skirt and the stent are sutured. The first sheet 5 and the second sheet 51 originate from integral pieces before suturing, and are described herein as distinct components based on this division. Preferably, when the first sheet and/or the second sheet is made of PU, its thickness ranges from 0.02mm to 0.2 mm; when the first sheet and/or the second sheet is made of a biological material, the thickness of the biological material ranges from 0.10mm to 0.50 mm. Fixation between sheets, and between sheets and the stent, may be accomplished by manners such as suturing or bonding:

For example, a sheet made of PU may be sutured or bonded to the stent, and bonding refers to fixation using a thin coating of biological glue, PU solution, or solvent; for example, the two sheets are both made of PU, and the two sheets may be connected by suturing or bonding; for example, one sheet made of biological material may be sutured to the stent or the other sheet; Sheets of the same material (both PU or both biological material) may also be directly formed as one-piece.

Additionally, one skirt may also be bonded to the sealing assembly attached to the other skirt. Bonding refers to fixation using a thin coating of biological glue, PU solution, or solvent. Furthermore, the bonding may be achieved by everting and wrapping followed by bonding, by partial bonding through the hollowed portion of the grid of the stent, or by bonding a part of the skirt that passes through grids of the stent. The bonding is not limited to sheet-to-sheet but may also be sheet-to-foam.

Based on the aforementioned first and second sheets, there are multiple ways to configure the inner skirt and the outer skirt. Only a few are listed below, but the configurations are not limited to the following examples.

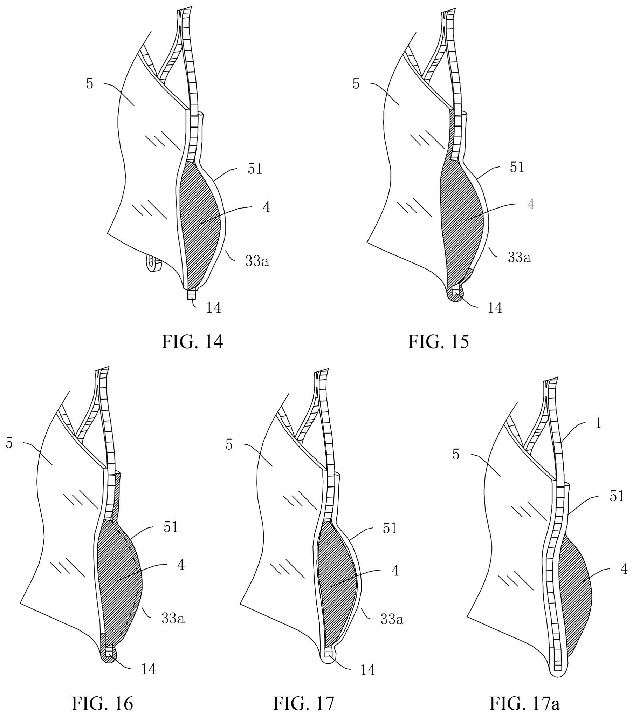

Referring to FIG. 14, the first sheet 5 is fixed to the inner side of the stent 1, and the second sheet 51 is fixed to the outer side of the stent. After suturing, neither the first nor the second sheet is folded over the stent ends. The inflow end of the stent is fully exposed, meaning the legs 14 are fully exposed outside the pouch structure formed by the two sheets (e.g., an end surface of the inflow end of the stent is not wrapped by the sheets, the inflow edges of the first and second sheets may be connected to each other to prevent blood from entering an interlayer between the first and second sheets).

Referring to FIG. 15, the first sheet 5 is fixed to the inner side of the stent. At the inflow side of the stent, the first sheet 5 is everted outward and wraps around the inflow end of the stent. The second sheet 51 is fixed to the outer side of the stent and is connected to the everted portion of the first sheet. This wrapping may be understood as fully wrapping (as shown in FIG. 3) or partial wrapping. Partial wrapping refers to a configuration where a part of leg of the stent exposed from the pouch structure between the two sheets (not shown in the figures).

Referring to FIG. 16, the second sheet 51 is fixed to the outer side of the stent. At the inflow side of the stent, the second sheet 51 is inverted inward and wraps around the inflow end of the stent. The first sheet 5 is fixed to the inner side of the stent and is connected to the inverted portion of the second sheet 51. (The understanding of wrapping here is the same as above).

Referring to FIG. 17, the first sheet and the second sheet are a one-piece structure and fully wrap the inflow end of the stent. (The understanding of wrapping here is the same as above). Additionally, several cut-out hollowed regions may be defined in the sheet(s) to expose a portion of the leg of the stent.

In each of the aforementioned configurations of the inner skirt and the outer skirt, the sealing assembly may be distributed in various ways. For example, the sealing assembly and either the first sheet or the second sheet may be formed as a one-piece structure; or the sealing assembly may be bonded to at least one of the first sheet or the second sheet; or the sealing assembly may be sandwiched between the two sheets. In the radial direction, the sealing assembly may be disposed at the inner side or the outer side of the second sheet.

Referring to FIG. 14, the sealing assembly 4 may be bonded to either the first sheet or the second sheet; Referring to FIG. 15, the sealing assembly 4 and the first sheet 5 are formed as a one-piece structure. Referring to FIG. 16, the sealing assembly 4 and the second sheet 51 are formed as a one-piece structure; Referring to FIG. 17, the sealing assembly 4 is sandwiched between the first sheet and the second sheet. In all the configurations illustrated in the above figures, in the radial direction, the sealing assembly is disposed at the inner side of the second sheet.

In some embodiments, referring to FIG. 17a, in the radial direction, the sealing assembly 4 may be disposed at the outer side of the second sheet. In this case, the sealing assembly 4 may also be connected to the second sheet by bonding or may be integrally formed with the second sheet.

Some embodiments of the present disclosure further provide a prosthetic implant, including a stent 1, a plurality of leaflets 7, a skirt 3, and a sealing assembly 4, which may incorporate the various embodiments described above. Referring to FIG. 17a, the sealing assembly 4 and the outer skirt are formed as a one-piece structure (i.e., the sealing assembly 4 and the second sheet are integrally formed) and the sealing assembly 4 is located on the outer side of the outer skirt.

In some embodiments, the inflow edge 311 of the inner skirt and the inflow edge 323 of the outer skirt are substantially aligned. These two inflow edges are connected either as a one-piece structure or as separate components (as shown in FIG. 12 or FIG. 13) to seal the blood flow channel. Correspondingly, in the radial direction, the sealing assembly may be disposed at the inner side or the outer side of the outer skirt.

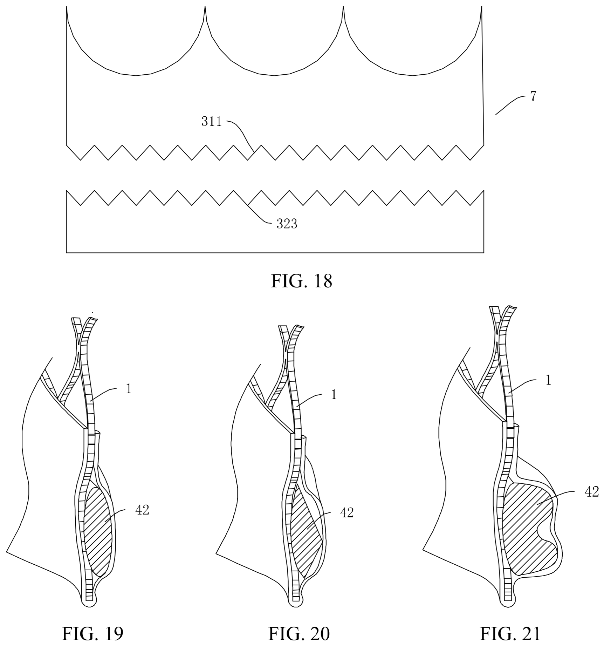

The shapes of the inflow edges of the inner and outer skirts are independent of each other. The inflow edges of the inner and outer skirts may each have a smooth inflow edge (as shown in FIG. 12) or a serrated inflow edge that conforms to the stent grids. As shown in FIG. 11a and FIG. 18, the first sheet and/or the second sheet may be cut into a serrated shape matching the stent grids and embedded within the stent grids, reducing crimping profile challenges. From a circumferential perspective of the stent, it may also be understood that the outflow edge of the outer skirt in FIG. 12 extends smoothly along the stent circumference, or the outflow edge of the outer skirt in FIG. 11a has axial undulations along the stent circumference. Preferably, the degree of axial undulation of the outflow edge 322 of the outer skirt matches the shape of the struts of the stent grids or matches the shape of the axial edge of the sealing assembly 4 (not shown in the figures).

In some embodiments, the inner skirt and the outer skirt are fixed to each other by at least one of bonding or suturing. For example, when both the skirts are made from sheets of PU or a bondable material, fixation between the outflow side of the outer skirt and the inner skirt may be achieved by applying a thin coating of biological glue or PU solution; for example, when both skirts are made of biological material sheets, the outflow side of the outer skirt and the inner skirt may be fixed by suturing.

The inner skirt and the outer skirt may be fixed to each other continuously along the stent circumference, or at intervals, to accommodate different scenarios where the outer skirt fully or partially encloses the sealing assembly. For example, in the scenario shown in FIG. 11a and FIG. 17, the sealing assembly is sandwiched between the first sheet and the second sheet, the outflow sides of the inner skirt and the outer skirt are fixed continuously along the stent circumference, resulting in the sealing assembly being fully encapsulated within the pouch structure; further for example, in FIG. 11b, the outer skirt only partially encapsulates the sealing assembly, the outflow sides of the inner skirt and the outer skirt are fixed at intervals along the stent circumference to accommodate the position of the sealing assembly.

Additionally, the corresponding portions of the outflow side of the outer skirt and the inner skirt may also be fixed using the aforementioned methods, that is, continuously or at intervals along the stent circumference.

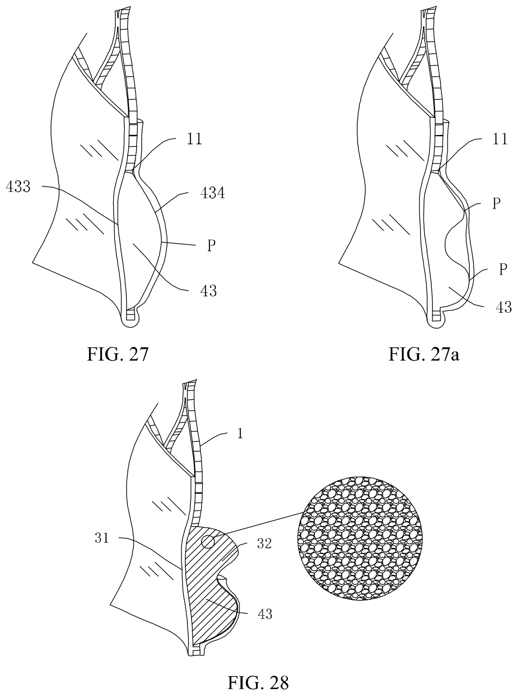

In some embodiments, the material of the sealing assembly is PU with internal voids, which may be understood as a PU foam structure (as shown in FIG. 28). An internal pore size ranges from 5mm to 500 micrometers, and a porosity may be 40% to 95%. The sealing assembly is made of water-swellable material, i.e., the sealing assembly expands upon absorbing water or bodily fluids in in-vitro simulated test environments or in-vivo environments. The swellable material, which deforms upon absorbing water in the body, may further utilize a self-radiopaque material, meaning it can be directly detected by imaging equipment based on its own chemical or physical characteristic.

The sealing assembly 4 is made of deformable PU foam, which may be designed into various shapes to adapt to the stent grids of different stents. Furthermore, due to the compressibility and self-expandability of PU foam, the height and thickness of the PU foam can be automatically adjusted under uneven external forces from the surroundings. Simultaneously, the PU foam absorbs blood and self-expands, thereby conforming tightly to the annulus and preventing perivalvular leakage. Additionally, the sealing assembly made of PU foam does not significantly increase the profile of the delivery system and can even adapt to smaller radial dimensions. Therefore, prosthetic implants utilizing sealing assemblies made of PU foam are also suitable for patients with severe calcification or narrow vasculature. Moreover, the porous structure of PU foam offers good compressibility, allowing it to disperse stress points through deformation during contact and friction with the delivery catheter components or bodily tissues, reducing the risk of tearing.

When the sealing assembly and the skirt are made of the same PU (not requiring strictly identical molecular weight or softness/hardness ratio when processed separately), the sealing assembly is bonded to or integrally formed with the PU skirt to obtain high peel strength. Consequently, when the sealing assembly is exposed, even if the sealing assembly experiences friction with the delivery catheter components or bodily tissues, it is less likely to detach, avoiding the safety risks associated with sealing assembly separation.

The bonding agent may be an identical type of PU solution. The solvent in the solution dissolves the PU at the interface to be bonded, causing the two interfaces to interdiffuse. After the solvent evaporates, the PU precipitates, achieving the bonding purpose. This method does not introduce new bonding agent; bonding identical materials produces a tight, strong connection at the interfaces.

In some embodiments, the sealing assembly may be arranged in various manners; the following examples are set forth without limitation:

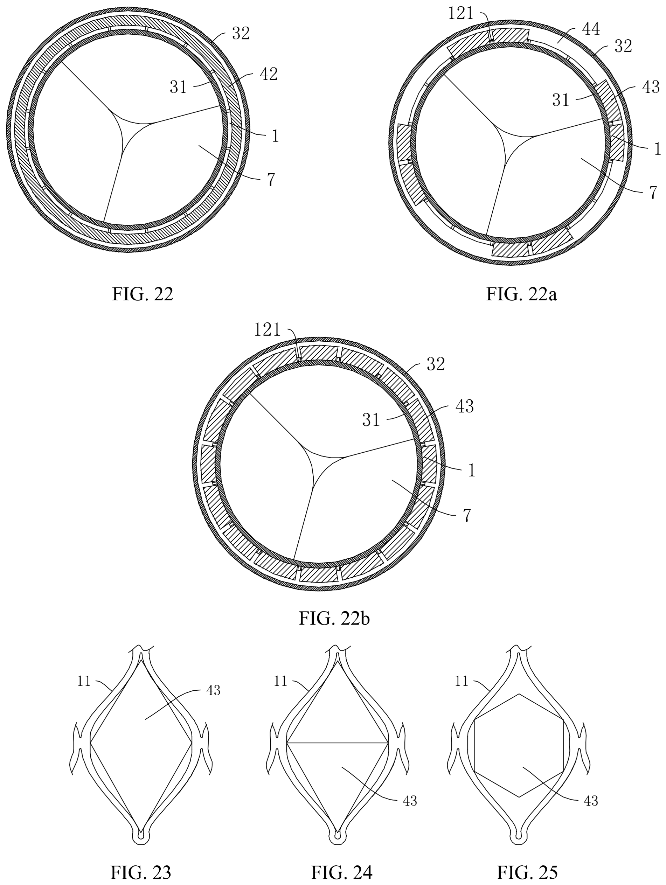

The sealing assembly may include a plurality of sealing blocks arranged circumferentially along the stent (as shown in FIG. 8), wherein "a plurality of" may be understood as two or more;
or, the sealing assembly is generally an annular member 42 located around the outer periphery of the stent, and the sealing assembly is directly sleeved over the outer periphery of the stent (as shown in FIG. 22);
or, the sealing assembly is directly sleeved over the outer periphery of the outer skirt (as shown in FIG. 7).

Some embodiments of the present disclosure further provide a prosthetic implant, including a stent 1, a plurality of leaflets 7, a skirt 3, and a sealing assembly 4, which may incorporate the various embodiments described above. The sealing assembly includes a plurality of sealing blocks arranged circumferentially along the stent. The plurality of sealing blocks are arranged in multiple groups spaced apart circumferentially along the stent, with at least one sealing block 43 in each group. Spacing regions 44 exist between adjacent groups, with all the sealing blocks located away from these spacing regions along the stent circumference. That is, the distribution regions of all the sealing blocks 43 are spaced apart circumferentially along the stent.

The sealing blocks are arranged in two to nine groups circumferentially along the stent, for example, three to six groups. Each group includes 1-10 sealing blocks. The distribution regions of the sealing blocks within the same group are continuously arranged along the stent circumference. Wherein "continuously arranged" may be understood that interruptions by struts or nodes of the grids (e.g., grid node 121 in the figure) within the same group of sealing blocks are not considered, and these interrupted regions can generally be closed after the sealing blocks adaptively deform under tissue compression. As shown in FIG. 22a, the sealing blocks are arranged in 4 groups spaced circumferentially along the stent, the groups are evenly distributed circumferentially, and each group includes two continuously distributed sealing blocks.

In some embodiments, as shown in FIG. 22b, the distribution regions of all the sealing blocks are continuous along the stent circumference, indicating the absence of spacing regions 44 along the stent circumference.

In some embodiments, in the radial direction, the sealing blocks are disposed at the inner side of the outer skirt. Each sealing block is embedded within a corresponding stent grid, and each sealing block may be connected to the inner skirt and/or the outer skirt (e.g., in FIG. 22b, each sealing block is bonded to the inner skirt); or each sealing block may be randomly filled between the two skirts (not shown in the figures).

In some embodiments, within a single grid, the sealing block(s) may also have various distribution manners. For example, shown in FIG. 23, the stent grid 11 contains only one continuously distributed sealing block 43; or, as shown in FIG. 24, the stent grid 11 includes multiple spaced sealing blocks 43.

The stent grids are arranged in multiple rows, including the aforementioned open-grids 111 and closed-grids 112. All the sealing blocks may be distributed within a single circumferential row, or in multiple circumferential rows. As shown in FIG. 8, the sealing blocks 41a are distributed in one single circumferential row of closed-grids 112 on the stent.

As shown in FIG. 9, the sealing blocks are distributed in two circumferential rows, within the open-grids 111 and the closed-grids 112 respectively. The sealing blocks in adjacent stent grids are arranged in an axially offset manner along the stent.

Some embodiments of the present disclosure further provide a prosthetic implant, including a stent 1, a plurality of leaflets 7, a skirt 3, and a sealing assembly 4, which may incorporate the various embodiments described above. In these embodiments, the sealing blocks in circumferentially adjacent stent grids are arranged in an axially offset manner along the stent.

Along the axial direction of the stent, opposite sides of the sealing block itself are a wider side and a narrower side, respectively. The sealing blocks in two adjacent stent grids are positioned such that their wider sides face each other.

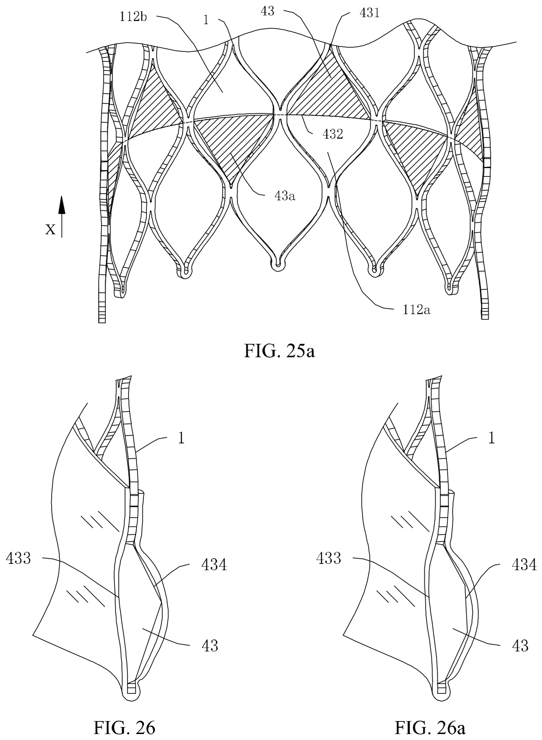

Referring to FIG. 25a, along the axis of the stent (X-direction in the figure), the two sides of the sealing block itself (taking sealing block 43 as an example) are the wider side 431 and the opposite narrower side 432. The sealing blocks in adjacent stent grids are arranged with their wider sides facing each other. That is, the wider sides of the corresponding sealing blocks 43 and 43a in adjacent closed-grids 112a and 112b face each other. Preferably, the wider sides of all the sealing blocks abut each other and extend continuously along the stent circumference (disregarding interruptions by struts or nodes; these interrupted regions may generally be closed after the sealing blocks adaptively deform under tissue compression).

In some embodiments, from a radial perspective of the stent, the shape of the sealing block 43 may be circular, elliptical, rectangular, rhombic (FIG. 23), triangular (FIG. 24), or hexagonal (FIG. 25).

Each sealing block is embedded in a corresponding stent grid 11. From a radial perspective, the sealing block may either fill the entire stent grid or fill only a portion of the stent grid 11. The sealing block may be located in a central region of the stent grid (the sealing block 43 as shown in FIG. 25), or adjacent to one axial end of the stent grid (the sealing block 43 as shown in FIG. 25a).

In some embodiments, a single stent grid 11 contains two sealing blocks spaced apart axially, with each sealing block located adjacent to one axial end of the stent grid.

In longitudinal cross-section, the sealing block 43 has an inner side 433 facing the stent, and an opposite outer side 434. The inner side 433 abuts the inner skirt 31. An edge of the outer side 434 has a broken line shape, a smooth curved shape, or a combination of both.

When the outer side edge of the sealing block 43 is a broken line, the longitudinal cross-section of the sealing block is generally triangular (as shown in FIG. 26), rectangular, or trapezoidal (as shown in FIG. 26a).

As shown in FIG. 27, when the outer side edge of the sealing block 43 is a smooth curve and has a radially highest point (point P in the figure, roughly where the sealing block has its maximum thickness). Preferably, along the axial direction, the highest point P within the stent grid 11 is closer to the inflow side.

As shown in FIG. 27a, the outer side edge of the sealing block 43 has a shape formed by a combination of a broken line and a smooth curved line. The sealing block 43 has a generally B-shaped longitudinal cross-section having at least two highest points P.

Since the sealing blocks of different shapes can adapt to the annulus shape under force after implantation, and the porous material absorbs blood, the sealing blocks fill gaps around the annulus and achieve the effect of preventing perivalvular leakage. Particularly, the B-shaped sealing block, with its central concave area, can effectively cradle the annulus. This enhances the sealing effect and also contributes to anchoring the valve.

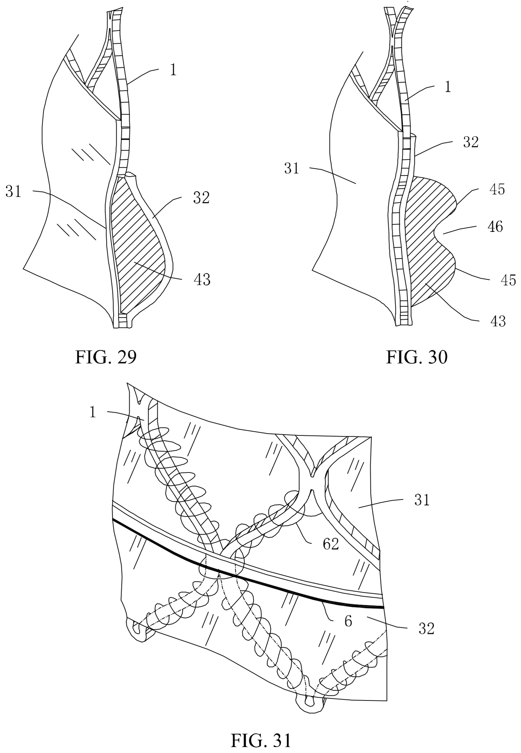

Some embodiments of the present disclosure further provide a prosthetic implant, including a stent 1, a plurality of leaflets 7, a skirt 3, and a sealing assembly 4, which can be combined with the various embodiments described above. Referring to FIG. 27a and FIG. 30, the sealing assembly forms at least two circles of peaks 45 around the outer periphery of the stent. These two circles of peaks 45 are arranged along the axial direction of the stent and define troughs 46 therebetween, wherein the troughs 46 are capable of accommodating the native valve annulus. The peaks in the same circle are constituted by either a plurality of sealing blocks spaced apart circumferentially (as shown in FIG. 27a, where a portion of the plurality of sealing blocks may be embedded within the stent grids and their outer periphery is enveloped by the outer skirt), or a sealing ring continuously distributed circumferentially (as shown in FIG. 30).

Preferably, viewed along the radial direction of the stent, the thickness of the sealing block may be uniform or non-uniform, with its maximum thickness being 0.5-5 mm.

Along the axial direction of the stent, the sealing assembly is generally located closer to the inflow end. The sealing assembly overall spans 0.5-3 stent grids, for example, 0.5-2 grids, along the axial direction of the stent.

In some embodiments, when the sealing assembly is an annular member 42, the annular member sleeves on the stent and is located within the gap between the outer skirt and the inner skirt (i.e., within the pouch structure). The longitudinal cross-section of the annular member 42 may adopt various shapes. That is, when the stent is in a fully expanded state, the shape of the longitudinal cross-section (outer contour) of the annular member is approximately circular, truncated circular (e.g., the semicircle shown in FIG. 17), trapezoidal, rectangular, elliptical, B-shaped, triangular, etc. FIG. 19 to FIG. 21 illustrate annular members with some of these cross-sectional shapes.

When the sealing assembly 4 employs a plurality of separate sealing blocks 43, the sealing assembly may be positioned in various installation locations. Several examples are listed below, but the possibilities are not limited to these.

In some embodiments, the outer skirt surrounds the outside of the sealing assembly and may envelop all or part of the sealing assembly. Referring to FIG. 28, the sealing assembly is fixed to the inner skirt 31 and protrudes from the radial inside of the stent through the corresponding grids towards the radial outside of the stent. The outer skirt 32 surrounds the outside of the sealing assembly and envelops a portion of the sealing assembly.

As another example, as shown in FIG. 29, the sealing assembly is fixed to the inner side of the outer skirt 32 and is located within the interlayer between the outer skirt 32 and the inner skirt 31. The outer skirt 32 fully envelops the sealing assembly.

In both of the aforementioned installation configurations, the sealing assembly is enveloped within the radial gap between the inner skirt and the outer skirt. Thereby, after the prosthesis implant is delivered or positioned in the body, contact and friction between the sealing assembly and the delivery catheter components or bodily tissues are avoided, resulting in higher safety.

Referring to FIG. 30, the sealing assembly is fixed to the outer side of the outer skirt 32, thus leaving the sealing assembly exposed. After the prosthesis implant is delivered or positioned in the body, it directly contacts the bodily tissues, thus allowing the sealing assembly to have a better shape adaptability and facilitating assembly.

The sealing assembly and the skirt may be formed as a one-piece structure, or as separate components. For example, the sealing assembly may be integrally formed with the PU skirt, or the sealing assembly may be bonded with thinly applied biological glue or PU solution.

In some embodiments, as shown in FIG. 28 and FIG. 30, in the longitudinal cross-section of the sealing assembly, a shape of the outer edge of the sealing assembly 4 has two peaks 45 and a trough 46 located between the two peaks (e.g., the aforementioned B-shape). Correspondingly, the outflow edge of the outer skirt can be located at the position of the trough.

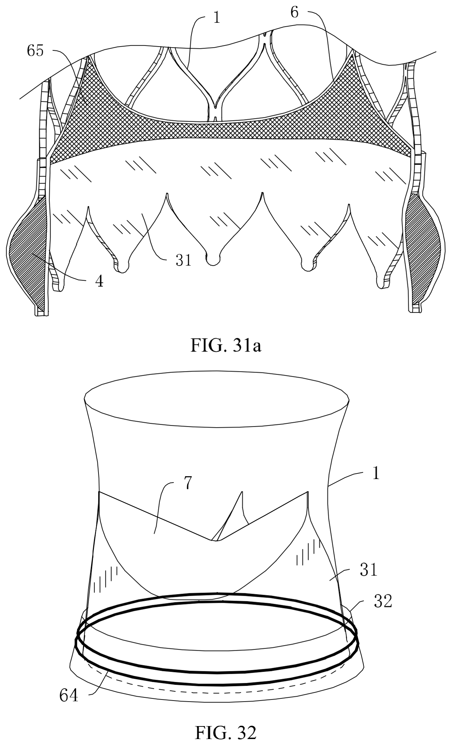

During the manufacturing process of the prosthesis implant, referring to FIG. 31 to FIG. 31a, the outer skirt 32 and the inner skirt 31 are connected to the stent 1 by sutures 62. At least one of the outer skirt and the inner skirt incorporates a reinforcement member 6 for partial or overall reinforcement. Part of the suture 62 is routed through the reinforcement member, thus resolving the issue of weak suture holding strength. Preferably, the material of the reinforcement member is PU or PET.

The reinforcement member may be configured in a variety of shapes, such as a sheet, a strip, a ring. The reinforcement member may also be fixed in various ways, such as being pre-embedded in or bonded to the inner skirt and/or the outer skirt.

Preferably, the reinforcement member may be a pre-embedded suture reinforcement wire 64 or reinforcement fabric 61 within the skirt, or the reinforcement member may be a reinforcement piece 65 directly attached to the outer skirt and/or the inner skirt.

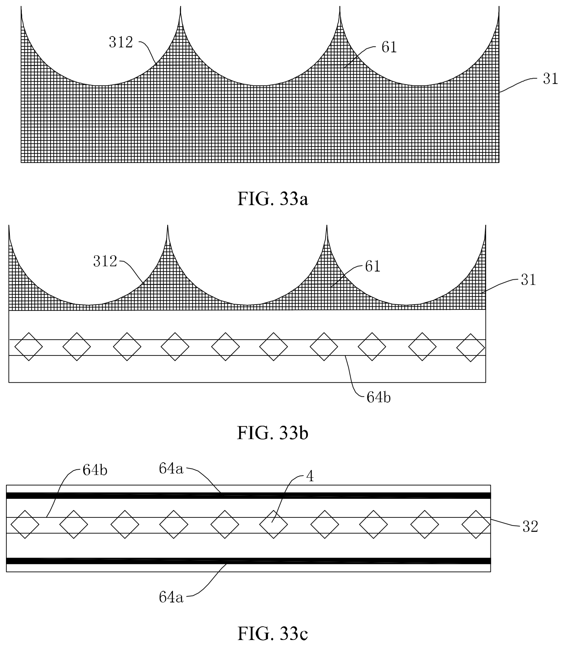

The reinforcement member may be distributed entirely or locally. For example, as shown in FIG. 33a, the reinforcement member is distributed over the entire inner skirt, thereby providing overall strength enhancement for the inner skirt; or, the reinforcement member is only located at the outflow side of the inner skirt and connected to the fixed edge of the leaflet (as shown in FIG. 31a and FIG. 33d), i.e., an edge of the reinforcement member aligns with the outflow edge of the inner skirt, thus providing strength enhancement only for the outflow side of the inner skirt.

In some embodiments, the reinforcement piece 65 may be located on the outer side of the outer skirt, or on the inner side of the inner skirt, or within the radial gap between the outer skirt and the inner skirt. The reinforcement piece 65 and the skirt to which it is attached are configured as a one-piece structure or separate components, with separate fixation employing adhesion or suture stitching, or a combination thereof. For example, in FIG. 31a, the reinforcement piece 65 is located within the radial gap between the outer skirt and the inner skirt and is directly bonded to the inner skirt.

In some embodiments, the suture reinforcement wire 64 may be pre-embedded within the skirt during its producing process. Preferably, the reinforcement wire is pre-embedded at the outflow edge of the outer skirt to enhance the connection strength between the outflow edge of the outer skirt and the stent, thereby achieving higher connection strength when the outer skirt contacts and rubs against bodily tissues.

As shown in FIG. 32, the suture reinforcement wire 64 extends along the stent circumference to form a wire loop. The loop may be a single ring or multiple rings. Multiple rings are arranged spaced apart or extend continuously in a spiral manner, achieving local reinforcement of the skirt.

Viewed from the circumferential direction of the stent, the suture reinforcement wire 64 extends along the stent circumference to form a wire loop. As shown, the wire loop may be a single ring or multiple rings. Multiple rings are arranged spaced apart or extend continuously in a spiral manner.

In some embodiments, the reinforcement fabric 61 may be pre-embedded within the skirt during its manufacturing process, or pre-formed separately and then fixed together by overlapping (e.g., fixed by bonding). For instance, as shown in FIG. 33a, the inner skirt 31 has reinforcement fabric 61 either entirely pre-embedded or fixed by overlapping. In FIG. 33b, the inner skirt 31 primarily has reinforcement fabric 61 disposed at the outflow side of the inner skirt 31, thereby enhancing the suture holding strength between the outflow edge 312 and the leaflet attachment edge, reducing the risk of tearing. Combining the material of the skirts mentioned above and the fixing manner between the sealing assembly and the skirts, the arrangement of the reinforcement member also has various scenarios. The reinforcement member may avoid or pass through the sealing assembly, and the sutures may avoid or pass through the sealing assembly 4.

The inner skirt 31 may be a PU membrane, corresponding to the flexible substrate in the following embodiments. The reinforcement fabric 61 may be a PET fabric, corresponding to the reinforcement component in the following embodiments. The PET fabric may provide overall or local reinforcement for the PU membrane. The PET fabric and the PU membrane may overlap and be fixed as separate sheets, with the overlapping region constituting the reinforced portion. Fixation may be achieved by bonding, thus forming a perivalvular leakage preventing skirt. The following embodiments relating to the perivalvular leakage preventing skirt provide specific improvements in this regard.

When the sealing assembly 4 is provided, it can be configured as a plurality of sealing blocks arranged spaced apart. The multiple sealing blocks may be adjacent to each other. The sealing blocks may be made of PU and fixed to the inner skirt 31.

The sealing blocks employ a deformable structure and are connected to the inner skirt 31, for example, via curing of a polyurethane solution. The following embodiments relating to the self-adaptive perivalvular leakage preventing component provide corresponding improvements regarding the materials of various parts and the connection process.

For example, in FIG. 33b, the sealing assembly 4 is located on the inner skirt 31 in a region exposed from the reinforcement fabric 61, generally adjacent to the inflow side. The sealing assembly 4 may be interconnected by reinforcement wires 64b to further enhance strength.

Some embodiments of the present disclosure further provide a prosthesis implant, including a stent 1, a plurality of leaflets 7, a skirt 3, and a sealing assembly 4, which can be combined with the various embodiments described above. The skirt has a reinforcement member and is connected to the stent by sutures that pass around the reinforcement member. The reinforcement member may be arranged to avoid the sealing assembly.

As shown in FIG. 33c, the outer skirt 32 may be utilized in conjunction with the sealing assembly 4 to reduce perivalvular leakage. The outer skirt 32 is made of PU and is integrally formed with the sealing assembly 4. The position of the reinforcement wire 64a avoids the sealing assembly, allowing for local reinforcement for the outflow side and/or the inflow side of the outer skirt. Accordingly, the sutures passing around the reinforcement wire 64a also avoid the sealing assembly.

The position of the reinforcement wire 64b may pass through the sealing assembly 4, acting as a pulling wire between multiple sealing blocks. This configuration may also be used in combination with the pulling wire 22 shown in FIGS. 48 and 49 and the associated embodiments, the pulling wire connects the individual sealing blocks. Sutures passing around the reinforcement wire 64b may also pass through the sealing assembly, not only strengthening the connection but also serving as a pre-connection of the sealing assembly to the stent before final attachment, which facilitates manufacturing.

Correspondingly, the inner skirt may also be reinforced overall or locally by reinforcement fabric. Local reinforcement may be distributed at the outflow edge and/or the inflow edge. For example, in the embodiment of FIG. 33d, the inner skirt 31 is provided with the reinforcement fabric 61 at its outflow side. The outflow edges of both the inner skirt 31 and the reinforcement fabric 61 are aligned. In other embodiments, outflow edges of the inner skirt 31 and the reinforcement fabric 61 may be offset from each other; for instance, the reinforcement fabric 61 may extend beyond the outflow edge of the inner skirt, forming a structure as shown in FIG. 87.

The inner skirt 31 and the outer skirt 32 may be configured as either a one-piece structure or as separate, fixed components. Referring to FIG. 34a and FIG. 34b, the inner skirt 31 and the outer skirt 32 are made of integral PU material and are formed as a one-piece structure with the sealing assembly. The inner skirt 31 is located on the inner side of the stent 1 and is sutured to the leaflets. The outer skirt 32 is everted to the outer side of the stent 1. The dashed line position (in FIG. 33d) between the inner skirt 31 and the outer skirt 32 generally corresponds to the inflow edge of the stent 1. In the figures, the reinforcement wire 64a avoids the sealing assembly, and the sutures pass around the reinforcement member and avoid the sealing assembly 4, providing local reinforcement for the outflow side of the outer skirt 32. Similarly, the reinforcement wires 64c and 64d provide local reinforcement for the inflow sides of the inner skirt 31 and the outer skirt 32, respectively. A reinforcement wire 64b passing through the sealing assembly 4 may be provided, as shown in the figure.

In other embodiments, the outer skirt may be made of PET fabric. After the connection of the outer skirt to the inner skirt, a structure as shown in FIG. 90 is formed.

In FIG. 34a, the outer skirt 32 is folded to the outer side of the stent 1 and fixed thereto, the sealing assembly 4 is located on the outer side of the outer skirt 32. In FIG. 34b, the sealing assembly 4 is located on the inner side of the outer skirt 32.

In the prior art, radiopaque markers (such as gold marker wires) are typically embedded around the stent circumference by operator to observe a deployment status of the stent in the body via imaging equipment. To facilitate observation of the deployment position and morphology of the prosthesis implant within the body, at least one of the outer skirt, the inner skirt, and the sealing assembly in the present disclosure has pre-embedded radiopaque markers 63.

Some embodiments of the present disclosure further provide a prosthetic implant, including a stent 1, a plurality of leaflets 7, a skirt 3, and a sealing assembly 4, which can be combined with the various embodiments described above. Radiopaque markers are pre-embedded within the skirt and/or the sealing assembly. The joining regions between adjacent leaflets at the stent are joint portions 73. The circumferential positions of the radiopaque markers correspond to the respective joint portions. At least one group of markers is provided, positioned adjacent to the inflow side of the stent in the axial direction. An axial distance between the group of radiopaque makers and the leaflets is sufficient to at least accommodate the native annulus.

Referring to FIG. 35 to FIG. 41, along the axis of the stent, the radiopaque markers 63 are adjacent to the inflow side of the stent. The formation of the radiopaque markers includes at least one of the following manners:
the radiopaque marker is pre-formed and acts as a pre-embedded component during a combining process;
the radiopaque marker is mixed into at least one raw material used in the combining process.

Particularly when the outer skirt or the inner skirt is made of PU, the radiopaque marker may be pre-embedded in a mold during combining PU. Notches corresponding to the marker shape may be defined in the mold, if necessary, to facilitate placement and positioning of the radiopaque marker; or the radiopaque marker may be mixed into the PU solution.

Preferably, the radiopaque marker may be a pre-embedded component in the form of a strip, a sheet, or a block, or may be in powder form mixed into at least one raw material.

In some embodiments, the radiopaque marker is pre-embedded within the skirt (which may be the inner skirt, the outer skirt, or a one-piece skirt). As shown in FIG. 35, the radiopaque marker 63 is strip-shaped and extends along the axial or circumferential direction of the stent. The radiopaque marker 63a extends circumferentially and can indicate an orientation of the stent. The radiopaque marker 63b extends axially and, particularly, to the outflow edge of the outer skirt, thereby accurately indicating the circumferential position of the stent. The radiopaque markers 63a and 63b may avoid each other or intersect. As shown in FIG. 36, the radiopaque markers 63 are block-shaped and arranged at intervals along both the axial and circumferential directions of the stent. The circumferential positions of the radiopaque markers correspond to the respective joint portions, enabling accurate indication of the orientation and the circumferential position of the stent.

As shown in FIG. 37, the radiopaque markers 63 are powdered. During the combining process of the PU skirt, the radiopaque markers are mixed into and dispersed within one of the raw materials. The circumferential position of the radiopaque markers may correspond to the lowest points of the fixed edge of the leaflet.

In some embodiments, the radiopaque marker may be pre-embedded within the sealing assembly. For example, as shown in FIG. 38, for the sealing assembly configured as the annular member 42, a strip-shaped radiopaque marker 63c is arranged circumferentially along the annular member to form one or more circles around the stent. Alternatively, block-shaped radiopaque markers 63d are arranged at intervals circumferentially along the annular member.

As shown in FIG. 39, when the sealing assembly is configured as a plurality of sealing blocks 43, at least two of the sealing blocks (e.g., three to six or even more) are provided with radiopaque markers 63 (which may be strip-shaped, block-shaped, or powdered).

Referring to the longitudinal cross-section of a sealing block 43 in FIG. 40, the radiopaque marker is located substantially in the central region (central point or central line) of the sealing block.

In some embodiments, as shown in FIG. 41, when the sealing assembly is configured as a plurality of B-shaped sealing blocks 43, the radiopaque marker includes a first marker 631 and a second marker 632. The first and second markers are respectively located at the corresponding peaks of the B-shape.

When the sealing block 43 is positioned in the body near the native annulus, the two peaks are positioned on two sides of the annulus. After self-expansion, the B-shaped sealing block clamps the annulus, providing a better perivalvular leakage preventing effect. Furthermore, the radiopaque markers located within the peaks can accurately indicate the position of the annulus.

In some embodiments, when the sealing assembly is configured as a plurality of B-shaped sealing blocks 43, the radiopaque marker may also be located at the trough of the B-shape, corresponding to the position of the native annulus.

The present disclosure further provides methods for producing the aforementioned PU skirt, PU foam, and integrally formed composite thereof, as well as methods for producing the PU skirt and the PU foam with locally or entirely reinforced structure, and methods for producing the PU skirts, the PU foam materials, or integrally formed composites thereof having radiopaque markers. The PU skirt is cut from a PU membrane; therefore, only the producing method for the overall PU membrane is provided below.

A producing method for PU membrane is as follows:
A1: dissolving implantable PU pellets (i.e., polyurethane) in a dimethylformamide (DMF) solvent to obtain a PU solution (i.e., polyurethane solution), wherein the polyurethane content in the polyurethane solution is 5%-20% (w/v). During dissolution of the PU pellets, a mechanical stirrer is employed to increase the dissolution rate, at room temperature or with heating (18-90°C);
A2: pouring a certain amount of the PU solution into a flat mold, and placing the flat mold in an oven at 30-70°C for 7-24 hours to obtain the PU membrane. A thickness of the membrane may be adjusted based on the solution concentration and the height of the solution in the mold, with a minimum thickness achievable of 0.02 mm.

A producing method for PU Foam is as follows:
B1: dissolving implantable PU pellets in a DMF solvent to obtain a PU solution, and Mixing the PU solution with sodium chloride to form a pre-mix for foam preparation. Wherein the sodium chloride solid particles have a diameter of approximately 5-500 micrometers, and the mass fraction of sodium chloride in the pre-mix is 50%-80%; or, a gas-foamed foam structure may be used;
B2: filling the pre-mix into a mold for shaping, curing, and cooling, and placing the mold in water (at room temperature or warm water). Wherein the mold is designed according to the shape of the sealing assembly;
B3: demolding the sample after completion; thereafter, washing the sample in water; and then drying the sample.

Regarding producing method for the foam, existing techniques in the industry for producing foam via chemical, gas, or solid methods may be employed, including but not limited to the above method (provided here merely as an illustrative example).

A producing method for integrally formed PU Membrane and PU foam is as follows:
C1: dissolving implantable PU pellets in a DMF solvent to obtain a PU solution (PU pellet content: 5%-20% (w/v)), and mixing the PU solution with sodium chloride to form a pre-mix for foam preparation. Wherein the sodium chloride particles have a diameter of approximately 5-500 micrometers, and the mass fraction of sodium chloride in the pre-mix is 50%-80%;
C2: filling the pre-mix into a mold and compact the pre-mix. The shape of the mold may be designed according to the shape of the sealing assembly;
C3: heating (40-80°C) the PU membrane obtained in step A2 above, then laying the PU membrane flat on the surface of the mold; and then letting the PU membrane stand or place the PU membrane in an oven for a specified time;
C4: demolding the sample after completion; thereafter, washing the sample in water to dissolve the salt particles; and then drying the sample to obtain integrally formed PU foam and PU membrane material.

A producing method for PU skirt with radiopaque marker is as follows:
D1: dissolving implantable PU pellets in a DMF solvent (PU pellet content: 5%-20% (w/v)) to obtain a PU solution;
D2: laying a radiopaque gold wire flat in a mold, then pouring a certain amount of the PU solution into the mold; and then placing the mold in an oven at 30-70°C for 7-24 hours to obtain a PU film incorporating the radiopaque gold wire. The number of gold wires may be adjusted according to imaging requirements.

A producing method for integrally formed PU Membrane and PU Foam material with radiopaque marker is as follows:
E1: dissolving implantable PU pellets in a DMF solvent to obtain a PU solution (PU pellet content: 5%-20% (w/v));
E2: mixing the PU solution with sodium chloride to form a pre-mix for foam preparation. Wherein the sodium chloride particles have a diameter of approximately 5-500 micrometers, and the mass fraction of sodium chloride in the pre-mix is 50%-80%;
E3: filling the pre-mix into a mold with a radiopaque metal or gold wire embedded therein, and then compacting the pre-mix. The shape of the mold may be designed according to the shape of the sealing assembly;
E4: bringing the PU membrane obtained in step A2 above to room temperature or preheating it to 30-70°C; then laying the PU membrane flat on the mold surface, and covering it with a flexible silicone sheet; and then letting the PU membrane stand or placing it in an oven for a specified time;
E5: removing the mold and cooling it to room temperature; and then placing the mold in water (at room temperature or warm water);
E6: after completion, demolding the sample after completion; and then placing the sample in water to dissolve the salt particles;
E7: drying the sample to obtain an integrally formed material of PU foam and PU membrane.

### Producing Example A

A producing method for PU membrane is as follows:
A1: dissolving implantable PU pellets (i.e., polyurethane) in a dimethylformamide (DMF) solvent to obtain a PU solution (i.e., polyurethane solution). The polyurethane content in the polyurethane solution is 8%-14% (w/v);
A2: pouring a certain amount of the PU solution into a flat mold, and then placing the flat mold in an oven at 70°C for 12 hours to obtain a PU membrane with a thickness of 0.03-0.05 mm.

### Producing Example B

A producing method for integrally formed PU membrane and PU foam is as follows:
C1: dissolving implantable PU pellets in a DMF solvent to obtain a PU solution (PU pellet content: 8%-14% (w/v)), and then mixing the PU solution with sodium chloride to form a pre-mix for foam preparation. The sodium chloride particles have a diameter of approximately 500 micrometers, and the mass fraction of sodium chloride in the pre-mix is 80%;
C2: filling the pre-mix into a mold and compacting the pre-mix. The mold has a rhombic shape;
C3: heating the PU membrane obtained in step A2 of producing Example A (80°C), then laying the PU membrane flat on the mold surface; and then covering the PU membrane with a flexible silicone sheet and letting it stand or placing it in an oven for a specified time;
C4: demolding the sample after completion; thereafter, washing the sample in water to dissolve the salt particles; and then drying the sample to obtain integrally formed PU foam and PU membrane material.

### Performance Characterization 1

According to the producing method of Producing Example A, five samples were obtained under identical conditions, designated as PU Membrane Sample 1 to PU Membrane Sample 5. Tensile strength and elongation tests were conducted on the PU membranes. The tensile strength test method was as follows:

The load value of a universal material testing machine was set to zero. A sample was clamped in the fixtures of the tensile machine, carefully and symmetrically adjusted to ensure uniform force distribution across the cross-section of the sample. An elongation measurement system reading was zeroed, and the tensile speed was set to 5 mm/min. And then the tensile machine was started until the specimen ruptured. The maximum load during the stretching process was recorded and subsequently converted to tensile strength. The recorded data are shown in Table 1 below:

**Table 1**

| Sample ID | Thickness (mm) | Tensile Strength (MPa) | Elongation (%) |
|---|---|---|---|
| PU Membrane Sample 1 | 0.03-0.05 | 42.60 | 296.239 |
| PU Membrane Sample 2 | 0.03-0.05 | 43.05 | 361.705 |
| PU Membrane Sample 3 | 0.03-0.05 | 44.37 | 313.171 |
| PU Membrane Sample 4 | 0.03-0.05 | 44.62 | 342.500 |
| PU Membrane Sample 5 | 0.03-0.05 | 45.43 | 294.371 |
| Average | 0.03-0.05 | 44.01 | 321.597 |
| Porcine Pericardium Material | 0.10-0.50 | 10-20 | 20-40 |

From this, it can be concluded that the PU membrane material exhibits good elasticity and high elongation. During crimping of the valve, the PU membrane can conform to the deformation of the stent, avoiding application of undue force on the stent and thus mitigating the risk of stent buckling. Furthermore, the tensile strength of the PU membrane produced in this disclosure is significantly greater than that of porcine pericardium material. Therefore, for equivalent requirements of strength and elongation, the skirt in this disclosure may employ a relatively thinner PU membrane, which is beneficial for the crimping, retrieval, and passage through human blood vessels of the interventional valve.

### Performance Characterization 2

According to the producing method of Producing Example B, five samples were obtained under identical conditions, designated as integrally formed PU Membrane/PU Foam Sample 1 to integrally formed PU Membrane/PU Foam Sample 5. The samples consist of PU membrane and PU foam, with the PU membrane thickness being 0.02-0.2mm and the PU foam height being 0.5-5mm. Tensile strength was tested, and the recorded data are shown in Table 2 below:

**Table 2**

| Sample ID | Tensile Strength (MPa) |
|---|---|
| Integrally Formed PU Membrane/PU Foam Sample 1 | 0.33 |
| Integrally Formed PU Membrane/PU Foam Sample 2 | 0.41 |
| Integrally Formed PU Membrane/PU Foam Sample 3 | 0.34 |
| Integrally Formed PU Membrane/PU Foam Sample 4 | 0.22 |
| Integrally Formed PU Membrane/PU Foam Sample 5 | 0.34 |
| Average | 0.33 |

The PU membrane substrate and the PU foam are of the same material, resulting in high bonding strength therebetween. Since the foam itself is porous and discontinuous, the actual fracture point during tensile testing of the sample occurs within the PU foam.

Furthermore, to more intuitively test the bonding strength between PU foam and PU membrane, samples as shown in FIGS. 42a to 42c were prepared for a comparative assessment against the bonding strength between PET membrane and PU foam. These samples include a PET membrane 100, a PU membrane 200, and an interlayer 300 made of the same material as the PU foam. A force F1 and an opposing force F2 of equal magnitude were applied to the PET membrane 100 and the PU membrane 200, respectively, for a peel test. The final test result was that the PET membrane 100 was separated from the interlayer 300, while the PU membrane 200 remained fixed to the interlayer 300.

This demonstrates that the PU membrane substrate and the PU foam, being the same material, exhibit a significantly higher bonding strength compared to a bonding strength between PU foam and PET membrane.

Conventional transcatheter aortic valve implantation (TAVI) technology generally employs a delivery system to position a prosthetic implant (this disclosure takes a prosthetic heart valve as an example) at the site of diseased native tissue, to replace the native valve and restore normal valve function. A conventional prosthetic heart valve includes a stent 162 and leaflets 165. Two ends of the stent 162 are the inflow side 1621 and the outflow side 1622, respectively. An interior of the stent 162 forms a blood flow channel 1623. A plurality of leaflets 165 are provided and located within the blood flow channel 1623, and the leaflets 165 are configured to cooperate with one another to selectively open or close the blood flow channel 1623.

Referring to FIG. 43, after the prosthetic implant 16 is substantially positioned in the body, with the valve open, blood flows along direction A from the inflow side 1621 through the blood flow channel 1623 towards the outflow side 1622. When the valve is closed, a pressure differential between the inflow and outflow sides that exceeds the valve's competence may cause blood to regurgitate along direction B around the outer periphery of the stent 162, leading to abnormal blood flow phenomena.

To mitigate leakage between the outer periphery of the stent 162 and the native valve tissue, a perivalvular leakage preventing device, such as a skirt or a water-swellable material attached around the outside of the stent, is sutured to the inflow side of the stent of some conventional prosthetic implants to achieve effect for perivalvular leakage preventing. However, these approaches will lead to issues such as an excessively large radial profile of the prosthetic implant after crimping.

Referring to FIG. 44 to FIG. 47, an embodiment of the present disclosure provides a perivalvular leakage preventing device for a prosthetic implant. The prosthetic implant 16 includes a tubular stent 162 with a grid structure 161. The interior of the stent 162 forms a blood flow channel. A plurality of leaflets 165 within the stent are configured to cooperate with one another to open or close the blood flow channel.

The perivalvular leakage preventing device 2 includes a plurality of sealing blocks 21 and one or more pulling wires 22. The plurality of sealing blocks 21 are arranged circumferentially around the stent 162 and are embedded into the corresponding grid structure 161. The plurality of sealing blocks 21 are gathered to encircle the stent 162 circumferentially. As shown in FIG. 46, viewed from the cross-sectional direction A-A in FIG. 43, all grid structures 161a in the same circumferential row are filled and embedded with sealing blocks 21.

The stent 162 is substantially tubular, that is the stent 162 has a corresponding axial direction (a direction of an axis), a radial direction perpendicular to the axial direction, and a circumferential direction around the axis. The grid structure may be understood as hollowed regions in the tubular sidewall, and the edges of the hollowed regions are the struts enclosing the grid. There are no strict limitations on the grid size or specific configuration. Along the axial direction of the stent, defined by the normal blood flow direction in the body, are the inflow side 1621 and the outflow side 1622, respectively. The stent itself may be manufactured by tube cutting or weaving, and the type of materials utilized depends on its deployment manner in the body (e.g., self-expanding or balloon-expandable).

The pulling wire 22 of the present disclosure may be selected from suitable materials and diameters to ensure sufficient strength for connecting the individual sealing blocks. The sealing blocks and the pulling wire may be connected by means such as bonding, suturing, or integral molding. All the sealing blocks 21 are arranged spaced apart sequentially along the stent circumference. Under the action of the pulling wire, the individual sealing blocks 21 are connected by the pulling wire to form an elongated, chain-like integral structure. The sealing blocks employ a deformable structure, for instance, relying on their own elasticity or a loose porous structure, facilitating a small outer diameter during crimping and compressing, while expanding upon deployment in the body to conform to the surrounding tissue, ensuring an effective perivalvular leakage preventing effect.

As shown in FIG. 47, after the sealing blocks 21 are embedded into corresponding grid structures 161a and deployed in the body, at least a portion of each sealing block 21 protrudes radially outward from the stent forming a protruding region 21a, while the portion within the grid forms a non-protruding region 21b. The sealing blocks employ a deformable structure, enabling the thickness of the protruding region 21a itself to be variable, or allowing the overall thickness, including the non-protruding region 21b, to be variable.

By using a pulling wire, the improved perivalvular leakage preventing device of the present disclosure can release the mutual constraint between the individual sealing blocks during the suturing process of the perivalvular leakage preventing device. This eliminates machining errors, particularly minimizing the accumulation of circumferential matching errors, and significantly reduces suturing difficulty. After the prosthetic implant of the present disclosure is delivered into the body and reaches the predetermined position, the protruding regions 21a of the perivalvular leakage preventing device are compressed against the surrounding tissue, and produce an adaptive conforming and positioning effect, adequately filling the gaps therebetween and resolving the perivalvular leakage issue.

Referring to FIG. 48 to FIG. 50, the sealing blocks 21 are arranged in multiple rows according to the correspondence of the sealing blocks 21 with the grid structures. The rows are arranged along the axis of the stent, and adjacent rows are staggered relative to each other. This staggered arrangement offers significant advantages during both the crimping phase of the prosthetic implant and the expansion phase after positioning in the body. As shown in FIG. 48, the compression directions of adjacent sealing blocks are circumferentially offset and not in the same plane, significantly reducing the crimping preload force. During the expansion stage of the sealing blocks, the protruding regions of the sealing blocks have a greater range of expansion. This enhances self-adaptability to fill gaps in a larger range, and ensures the perivalvular leakage preventing effect.

As shown in FIG. 49, which shows the sealing blocks of FIG. 48 in a flattened view, the sealing blocks within the same row are connected only by the pulling wire 22. This not only meets the requirement for circumferential connection and positioning of the sealing blocks but also minimizes the profile of the prosthetic implant after crimping.

Wherein the sealing blocks and the pulling wire may be connected by various methods such as bonding, suturing, or integral molding. However, considering that bonding and suturing processes still involve potential manual operational errors (e.g., suturing error, bonding error) and aiming to simplify the molding process, connecting strips 23, integrally formed with the sealing blocks, are provided between adjacent sealing blocks 21 in the same row. This allows for the positioned, spaced arrangement of the sealing blocks via the connecting strips 23 during their molding stage. The connecting strips may be used concurrently with the pulling wire(s) or may serve as the pulling wire(s). Both configurations allow the sealing blocks to be linked forming a sealing block ring, further enhancing the connection strength and facilitating subsequent suturing.

Analyzing individual structure of the grid structures, the grid structures near the inflow side 1621 of the stent are divided into two types: closed-grids 1611 and open-grids 1612. According to the correspondence between the sealing blocks and the grid structures, the sealing blocks 211 are fully embedded in and fill the closed-grids 1611, and the sealing blocks 212 are partially embedded in and fill the open-grids 1612.

Analyzing the overall structure of the stent, the row of grids closest to the inflow side 1621 on the stent is an open-grid layer composed of open-grids 1612. The row adjacent to the open-grid layer is a closed-grid layer composed of closed-grids 1611. According to the correspondence between the sealing blocks and the grid structures, two rows of sealing blocks are arranged at the inflow side 1621. The first row of the two rows is defined by sealing blocks 211 that fully fill corresponding grid structures (i.e., fully embedded in the closed-grid layer), and the second row of the two rows is defined by sealing blocks 212 that partially fill corresponding grid structures (i.e., the sealing block ring is partially embedded in the open-grid layer). In the first row, spaced portions 241 are present between every two adjacent sealing blocks 211, and the sealing blocks 212 of the second row are located at these corresponding spaced portions, presenting the aforementioned staggered arrangement.

Referring to FIG. 51 to FIG. 55, to enhance the adaptive conforming and positioning effect of the sealing blocks, the present disclosure divides the grid structures 161 (including closed-grids 1611 and open-grids 1612) into functional zones. Specifically, along the axial direction of the stent, the grid structure 161 is divided into a central region 1613 and edge regions 1614 disposed on two sides of the central region. Different zones exhibit different sealing effects after being filled with the sealing block.

Regarding the shape of the sealing blocks, they may be triangular pyramids, quadrangular pyramids, ellipsoids, partial ellipsoids, spheres, partial spheres, truncated pyramids, other three-dimensional shapes, or combinations of various 3D solids. When the sealing blocks are formed by molding, a mold cavity of a mold has a corresponding shape as the sealing blocks.

For example, as shown in FIG. 51 and FIG. 54, along the axial direction of the stent (e.g., direction Z in the figure), a thickness h1 of the sealing block 211 decreases from the central region 1613 towards the edge regions 1614 of the closed-grid 1611. Along the stent circumferential direction, the width t1 of the sealing block 211 decreases from the central region 1613 towards the edge regions 1614 of the closed-grid 1611.

For example, in FIG. 51 and FIG. 55, along the radial direction of the stent (e.g., direction Z in the figure), a thickness h2 of the sealing block 212 decreases from the central region 1613 towards the edge regions 1614 of the open-grid 1612. Along the stent circumferential direction, a width t2 of the sealing block 212 decreases from the central region 1613 towards the edge regions 1614 of the open-grid 1612. The maximum thickness of the sealing blocks is about 3 mm to 1 cm.

The aforementioned structural improvements regarding the thickness and width of the sealing blocks cause the material of the sealing block to be concentrated towards the central region. The portion of the sealing block located in the central region of the grid structure 161 has optimal deformation capability, further enhancing the ability to fill gaps. Additionally, reduced material accumulation in the edge regions, combined with the advantage of the staggered arrangement, ensures that the central regions of adjacent rows are completely offset, further reducing the preload force during crimping of the prosthetic implant and its radial profile after crimping.

The sealing blocks may be made of PU (polyurethane) with a porous structure, having a porosity of 50-85, for example, 60%-80%, or specifically 75%. In the production of the sealing blocks, solid particles may be added to a polymer solution. After curing, the solid particles are removed, thereby forming a porous foam structure. The solid particles are generally water-soluble but not readily soluble in the polymer solution. (PU foam may also be prepared using gas).

Water washing manner may be used to remove the solid particles, potentially combined with vibration or kneading. The temperature of water is not strictly limited; to improve washing efficiency, a washing temperature is 30-60°C.

The solid particles have a particle size of approximately 5-500 micrometers(µm), for example, 5-250 micrometers. The solid particles may be sodium chloride, potassium chloride, or sugar. The corresponding pore size is approximately 5-200 micrometers. By grading and adjusting the particle size of the solid particles, uniform or variable pore sizes can be obtained. The concentration of the polymer solution may be 0.5%-20.0% (w/v), for example, 5.0%-15.0% (w/v). When polyurethane is employed as the raw material, the solvent may be DMSO (dimethyl sulfoxide), etc.

Due to the porous structure, the obtained sealing blocks have good elasticity and elongation, allowing the sealing blocks to adapt to the in-vivo environment ensuring sealing effectiveness, and also facilitating compression, and transcatheter delivery into the body. Compared to the compressed state, the volume of the sealing blocks after self-expansion can reach 2 to 30 times, for example, 3 to 20 times, or at least 5 times.

The position and number of connections between the pulling wire(s) and the sealing blocks affect the connection strength and positioning effect. The following provides some improved configurations.

Referring to FIG. 56 to FIG. 60, along the radial direction of the stent (i.e., the X direction in FIG. 56), the perivalvular leakage preventing device 2 has an inner side 2a and an opposite outer side 2b. The position of the pulling wire(s) 22 is adjacent to the inner side 2a.

After the sealing blocks and pulling wire(s) of the same row are sutured to the stent, viewed in the entire structure, the pulling wire(s) 22 reside on the inner side of the stent 162, or even conforms to the grid structure, facilitating suturing. The sealing blocks 21 protrude outward from the outer peripheral surface of the stent 162, forming the aforementioned protruding region 21a and non-protruding region 21b.

The perivalvular leakage preventing device 2 has a coiled state, wound around the stent 21, and a relatively unfolded state. The coiled state corresponds to the annular form of the perivalvular leakage preventing device 2 sutured onto the tubular stent (as shown in FIG. 56). The unfolded state refers to the linear chain configuration when unsutured (as shown in FIG. 58), which may also be understood as the direct form of the perivalvular leakage preventing device after the raw material is cured and formed. A plurality of pulling wires are arranged along an extending direction thereof.

Regarding to above-described multiple pulling wires, each pulling wire corresponds to at least one row of sealing blocks. As shown in FIG. 58, the pulling wire 22a corresponds to one row of sealing block ring 24a, the pulling wire 22b corresponds to two rows of sealing block ring 24, and the pulling wire 22c corresponds to the rows of sealing block rings 24b and 25.

To enhance circumferential positioning and ensure the effectiveness of the staggered arrangement, at least one pulling wire passes through all the sealing blocks in two adjacent rows. When multiple rows of sealing blocks are provided, the rows arranged along the axial direction of the stent and at least one pulling wire passes through all the sealing blocks in one of these rows. As shown in the figures, the pulling wires 22b and 22c pass through all the sealing blocks in two adjacent rows, not only ensuring positioning for sealing blocks within the same row but also ensuring positioning for sealing blocks in the staggered adjacent rows.

As shown in FIG. 59, the multiple pulling wires may be arranged parallel or interlaced. The pulling wires may be arranged along the circumferential or axial direction of the stent, both serving to adequately position the sealing blocks in the staggered adjacent rows. In the figure, along the stent circumference, both the pulling wire 22a and the pulling wire 22b pass through all the sealing blocks of the same row and are parallel to each other. Along the axial direction of the stent, both the pulling wire 22e and the pulling wire 22d pass through adjacent sealing blocks in adjacent rows. In three adjacent sealing block rings (sealing block rings 24a, 24b, 25), the pulling wire 22d passes through adjacent sealing blocks 211b, 211a, and 212a in the axial direction, and the pulling wire 22d intersects locally with the pulling wires 22a, 22b, and 22c.

For the pulling wires arranged parallel within the same row, at least one pulling wire extends along the length direction of the perivalvular leakage preventing device. The length direction of the perivalvular leakage preventing device 2 corresponds to the circumferential direction of the stent in the coiled state (e.g., the Y direction in the figure). That is, from the perspective of the unfolded state, the sealing block ring 24a is unfolded along the Y direction (i.e., the length direction of perivalvular leakage preventing device 2), and both the pulling wire 22a and the pulling wire 22b extend along the Y direction in the figure. For the sealing block ring 25, only one pulling wire, i.e., the pulling wire 22c, extends along the Y direction in the figure.

In scenarios with multiple rows, at least two pulling wires extend along the length direction of the perivalvular leakage preventing device in the unfolded state. The sealing block rings 24a and 24b are both unfolded along the Y direction, and the pulling wires 22a, 22b, and 22c also extend along the Y direction.

Whether for the sealing blocks in closed-grids or open-grids, the pulling wires connected thereto may be arranged in one group or more than two groups. Each group consists of multiple parallel pulling wires. One group extends along the length direction Y of the perivalvular leakage preventing device, and another group extends along the width direction of the perivalvular leakage preventing device (corresponding to the axial direction of the stent, i.e., the Z direction shown in FIG. 59). As shown, the sealing block 212a is provided with 2 groups of the pulling wires: one group consists of the pulling wire 22c extending in the Y direction, and the other group consists of the pulling wires 22e and 22d extending in the Z direction. The sealing block 211b is also provided with 2 groups of the pulling wires: one group consists of the pulling wires 22a and 22b extending in the Y direction, and the other group consists of the pulling wires 22e and 22d extending in the Z direction.

The pulling wires with different extension directions may be sections of the same single wire that is arranged in a meandering way. As shown in FIG. 60, the pulling wire 22a extending in the Y direction and the pulling wire 22d extending in the Z direction may be formed from the same single pulling wire.

Since the thickness and width of the sealing blocks increase from the edge regions towards the central region, and material of the sealing block concentrating towards the central region, The connection position and manner between the pulling wires and the sealing blocks are critical to the final positioning. This is because the thickness and width of the sealing blocks increase from the edge regions towards the central region, concentrating the material of the sealing blocks at the central region. Therefore, some improvements regarding the connection position and manner between the pulling wires and the sealing blocks are provided below.

Referring to FIG. 61 and FIG. 62, according to the correspondence with the grid structures, the sealing blocks are arranged in one or multiple rows. At least two pulling wires passe through the sealing blocks in the same row and extend along the length direction of the perivalvular leakage preventing device in the unfolded state. As shown, when the sealing blocks 211 are arranged in only one row, two pulling wires (the pulling wires 22a, 22b) pass through the edge regions of all the sealing blocks 211. When the sealing blocks 211 and 212 are arranged in two rows, at least 2 pulling wires may be set to pass through the edge regions of all the sealing blocks 212, with one pulling wire 22c passing through the edge regions of all the sealing blocks 211 and 212.

Preferably, referring to FIG. 63, when the sealing blocks 211 and 212 are arranged in two rows, along the width direction of the perivalvular leakage preventing device in the unfolded state, at least one of the pulling wires - which passes through the sealing blocks of the same row - is positioned adjacent to the central region of the sealing blocks. The figure shows 2 pulling wires, and the pulling wire 22b passes through the central region of the sealing block 211. Furthermore, along the width direction of the sealing block (the Y direction in the figure), the overlapping length L1 of the pulling wire 22b and the sealing blocks 211 relative to the maximum width L2 of the sealing block 211 is 1/3-2/3. This connection position avoids the region with the greatest deformation on the sealing block, preventing issues at the pulling wire connection points after the sealing block deforms, while still maintaining connection strength.

Referring to FIG. 64, a limiting component is fixed to the pulling wire and is embedded within the sealing block. The limiting component may be a knot 221. The limiting component may be embedded into the sealing block before the sealing block is cured, to obtain a good embedding effect after the sealing block fully cured and thus preventing movement of the sealing block along the smooth surface of the pulling wire under force.

The limiting component may further include a meandering structure 222 connected to the knot 221, further ensuring the limiting effect. When multiple pulling wires are provided, all the pulling wires are connected via limiting components, forming an interconnected integral whole.

The pulling wire(s) may be made of materials such as Nylon or PTFE.

Referring to FIG. 65 and FIG. 66, in another embodiment, the perivalvular leakage preventing device 2 further includes an inner membrane 26. In the coiled state, the inner membrane 26 is disposed on the inner side, and the sealing blocks are fixed to the outer side of the inner membrane 26.

In other embodiments, only the inner membrane 26 may be provided, and the pulling wire(s) may be omitted. Spaced portion 241 is defined between two adjacent sealing blocks in the same row. Along its extending path, a portion of the pulling wire 22a is located within the spaced portion 241, and this portion is attached and fixed to the inner membrane 26.

Conventional inner membranes generally made of PET. While PET generally meets performance needs, it has high cost, low elasticity. During attachment to the stent, PET creates larger needle holes with high stress concentration at the suture line, which leads to difficulty in self-closing. During radial compression and deploying of the prosthetic implant, local bending and folding might cause the PET inner membrane to crack. Additionally, deformation of the stent grid might pull and tear the PET membrane.

Materials for the inner membrane may include, but are not limited to, the following biocompatible and biostable materials: PET, organosilicon, polyurethane (PU), PEEK, PTFE, Nylon, PP, PE (LDPE/HDPE), polyamide, polyester, Ultra-High Molecular Weight Polyethylene (UHMWPE), silicone, POM, PPSU, PSU, PVDF, etc. Preferably, in this embodiment, both the inner membrane and the sealing blocks are made of the same material, e.g., both made of PU. The inner membrane and the sealing blocks may be made of PU but with different specific physical and chemical properties (e.g., soft/hard segment ratio, elastic modulus, porosity, etc.). Alternatively, the inner membrane and the sealing blocks may be made of the same raw material, i.e., having identical specific physical and chemical properties. The thickness of the inner membrane is 0.1-0.5 mm, for example, 0.15-0.4 mm, or specifically 0.2-0.3 mm. Preferably, the inner membrane 26 is made of non-foamed PU.

After the sealing blocks and the inner membrane are manufactured separately, they may be bonded together using processes like adhesion, for instance, forming an adhesive layer.

Referring to FIG. 67 to FIG. 68, an embodiment of the present disclosure further provides a prosthetic implant 16, including a stent 162 and a perivalvular leakage preventing device 2 disposed around the stent. The stent 162 is tubular and has a grid structure 161. The grid structure may be understood as hollowed region in the tubular sidewall, the edges of hollowed region are the struts enclosing the grids. There are no strict limitations on the grid size or specific shape. Along the axial direction stent, defined by the normal blood flow direction in the body, are the inflow side 1621 and the outflow side 1622, respectively. The stent may be manufactured employing the methods described previously.

The perivalvular leakage preventing device 2 is disposed around the inflow side 1621 of the stent and primarily includes sealing blocks 21 and pulling wire(s) 22. A plurality of sealing blocks 21 are arranged circumferentially around the prosthetic implant 16. The sealing blocks employ a deformable structure and are embedded into corresponding grid structures. The sealing blocks are interconnected by the pulling wire(s) 22 to form one or more rows of sealing block rings. Furthermore, after being embedded into the grid structures 161, at least a portion of each sealing block 21 protrudes radially outward from the stent, forming the protruding region 21a.

When the prosthetic implant 16 is deployed in the body and the crimping force is released, the sealing blocks 21, relying on their own elasticity or loose porous structure, fully expand and deform. The protruding regions 21a of the sealing blocks are compressed against the surrounding tissue and can produce an adaptive conforming and positioning effect, thereby circumferentially matching and filling the gap between the stent and the native valve annulus, solving the problem of leakage around the periphery of the prosthetic heart valve.

Subsequent embodiments also provide methods for producing the prosthetic implant, used to produce the prosthetic implant described above.

An embodiment of the present disclosure provides a method for producing a prosthetic implant. The employed stent 162 is tubular and has a grid structure 161. The employed perivalvular leakage preventing device 2 includes sealing blocks 21 and one or more pulling wires 22. A plurality of sealing blocks 21 are arranged circumferentially around the prosthetic implant. Each sealing block employs a deformable structure, and the sealing blocks are interconnected by the pulling wire(s). The stent and perivalvular leakage preventing device mentioned in this embodiment may be combined with the various embodiments described above.

Referring to FIG. 69 to FIG. 71, a producing method of this embodiment includes the following steps:
A1: providing the aforementioned stent 162 and perivalvular leakage preventing device 2. At this stage, the stent 162 is in an expanded state, and the sealing blocks 21 are connected by the pulling wire(s) 22 to form an elongated, chain-like perivalvular leakage preventing device;
A2: then, coiling the perivalvular leakage preventing device 2 and to form a coiled state in which the ends are connected. The sealing blocks, connected by the pulling wire(s), form sealing block rings (e.g., the sealing block ring 24 and the sealing block ring 25 in the figures);
A3: fixing the perivalvular leakage preventing device 2 in the coiled state to the stent 162. During fixation, each sealing block is embedded into a corresponding grid structure.

If the perivalvular leakage preventing device consists of multiple layers of sealing block rings, the multiple layers of sealing block rings may be preconnected via multiple groups of pulling wires arranged in parallel and intersecting patterns to form a complete sealing block mesh.

Subsequent embodiments provide another method for producing a prosthetic implant, used to produce the prosthetic implant described above.

An embodiment of the present disclosure provides a method for producing a prosthetic implant. The employed stent 162 is tubular and has a grid structure 161. The employed perivalvular leakage preventing device 2 includes sealing blocks 21 and a pulling wire 22. A plurality of sealing blocks 21 are arranged circumferentially around the prosthetic implant. Each sealing block employs a deformable structure, and the sealing blocks are interconnected by the pulling wire(s). The stent and perivalvular leakage preventing device mentioned in this embodiment may be combined with the various embodiments described above.

Referring to FIG. 72 to FIG. 74, a producing method of this embodiment includes the following steps:
B1: providing the aforementioned stent 162 and a perivalvular leakage preventing device 2. At this stage, the stent 162 is in an expanded state, and the sealing blocks 21 are connected by the pulling wire(s) 22 to form an elongated sealing block strip 28;
B2: placing a portion of the perivalvular leakage preventing device along a length direction thereof inside or outside the stent and fixing the portion of the perivalvular leakage preventing device to the stent 162;
B3: gradually fixing the remaining portion of the perivalvular leakage preventing device 2 to the stent 162 until the perivalvular leakage preventing device 2 is coiled to a coiled state in which the ends are connected. During fixation, each sealing block is embedded into a corresponding grid structure.

The producing method of this embodiment can be better adapted to stents of different radial sizes. The perivalvular leakage preventing device is progressive embedded into the grid structures facilitates adjustment of the sealing block positions.

The following embodiments further provide a mold for forming the perivalvular leakage preventing device, configured to produce the aforementioned perivalvular leakage preventing device.

Referring to FIG. 75 to FIG. 78, an embodiment of the present disclosure provides a mold 8 for forming a perivalvular leakage preventing device. The mold incudes a main body 81, and the main body 81 defines a working surface 811 at one side. A plurality of mold cavities 82 and wire grooves 83 are defined on the working surface 811. Wherein, the plurality of mold cavities 82 are configured to form the sealing blocks. The shape of the mold cavities is determined according to the specific shape of the grid structure of the stent. The number of mold cavities may be determined according to the number of grid structures at the inflow side of the stent. The wire grooves 83 are for receiving the pulling wire(s). The formed sealing blocks are connected by the pulling wire(s) to constitute the perivalvular leakage preventing device 2, so that the perivalvular leakage preventing device 2 corresponds one-to-one with the grid structure of the corresponding stent, simplifying subsequent processing steps. The plurality of wire grooves may be arranged in parallel or intersecting patterns, facilitating the formation of a sealing block mesh or a sealing block strip after the sealing blocks are cured. The stent, the sealing blocks, the pulling wire(s), the sealing block mesh, and sealing block strip mentioned in this embodiment may be combined with the various embodiments described above.

Regarding the structure and arrangement of the wire grooves, the wire grooves 83 may be open to the working surface 811 to facilitate laying the pulling wire(s). The wire grooves 83 may extend to an edge of the main body 81, so that after the sealing blocks are cured, the operator can directly pull the pulling wire(s) to get all the sealing blocks at once. The wire grooves 83 extend linearly. The arrangement of the wire grooves and the sealing blocks matches the perivalvular leakage preventing device in its unfolded state.

According to previous embodiments, an inner membrane may be provided in the perivalvular leakage preventing device, with the sealing blocks fixed to the outer side of the inner membrane 26. To integrally form the inner membrane, the mold 8 further includes a forming frame 84. The forming frame is disposed on the working surface 811 and surrounds the periphery of openings of the mold cavity. The entire working surface needs to be covered by the forming frame to ensure that all sealing blocks are connected via the inner membrane and the pulling wire(s). Additionally, a hollowed region 841 is defined in the central portion of the forming frame. This hollowed region needs to expose all of the mold cavities so that the finally formed inner membrane can cover all the sealing blocks.

Wherein the forming frame 84 and the main body 81 may be separate structures or an integral structure. Both mold configurations are suitable for forming the perivalvular leakage preventing device with an inner membrane. The following describes improvements for both mold structures.

For the separate structure mold, the forming frame 84 may be stacked onto the working surface 811. The hollowed region 841 in the center of the forming frame exposes all mold cavities, so that after raw material of the inner membrane and the sealing block are simultaneously cured, all the sealing blocks are interconnected via the inner membrane and the pulling wire(s).

For the integral structure mold, referring to FIG. 78, the forming frame 84 is a recessed region 842 of the working surface 811. The mold cavities 82 and wire grooves 83 are defined within this recessed region. In the subsequent process of forming the perivalvular leakage preventing device, the raw material of the sealing block and the raw material of the inner membrane are filled sequentially. All the sealing blocks are cured simultaneously to be interconnected via the inner membrane and the pulling wire(s).

The following embodiments further provide a method for producing the perivalvular leakage preventing device using a mold. The employed mold includes a main body 81, a working surface 811 is defined in one side of the main body 81. A plurality of mold cavities 82 and wire grooves 83 are defined on the working surface 811. The mold may be a silicone mold, Teflon mold, aluminum mold, stainless steel mold, or glass mold, and may be prepared employing methods such as 3D printing, traditional machining, or casting.

The perivalvular leakage preventing device may specifically be combined with the various embodiments described above. Referring to FIG. 79, this embodiment includes the following steps:
C1: adding raw material of the sealing block into the multiple mold cavities 82 of the mold 8. At this stage, the raw material of the sealing block still has some fluidity. The raw material of sealing block is preferably a PU DMSO solution with a concentration of 0.5%-20.0% (w/v). This PU solution may be formulated according to the actual performance requirements of the foam body to obtain different parameters such as elastic modulus, expansion coefficient, porosity, etc. The PU DMSO solution contains solid particles with a particle diameter of approximately 5-500 micrometers, e.g., sodium chloride with a particle diameter of 5-250 micrometers;
C2: laying the pulling wire(s) 22 with an extending path of the pulling wire(s) intersecting the mold cavities 82. The pulling wire(s) may extend to an edge of the mold, that is, an extension direction of the pulling wire(s) follows the arrangement direction of the mold cavities. In case of the pulling wire(s) having knots for limiting position, place the knots completely within the sealing block material in the mold cavities at this stage to achieve an embedding effect;
C3: curing the raw material of the sealing block under predetermined conditions. A curing temperature is 10-60°C, for example, 20-50°C, or specifically 30-40°C. A curing time is 1-24 hours, for example, 1.5-10 hours, or specifically 2-4 hours. In this embodiment, curing is performed at 37°C for 2-4 hours, allowing the raw material of the sealing block to cure and form a stable shape;
C4: demolding the formed, interconnected sealing blocks via the pulling wire(s) to obtain the perivalvular leakage preventing device 2. Since the pulling wire(s) extend beyond the edge of the mold, all sealing blocks can be demolded and removed by directly pulling the pulling wire(s). Subsequently, water washing may be performed to remove the solid particles, obtaining the corresponding porous structure.

In the aforementioned molding method, the steps of adding the raw material of the sealing block and laying the pulling wire(s) may be performed in any order, or alternately performed.

The raw material of the sealing block is fluid or paste-like and is leveled or scraped flat to be flush with the working surface.

According to some embodiments above, the perivalvular leakage preventing device further includes the inner membrane 26. In the coiled state, the inner membrane 26 is on the inner side, and the sealing blocks are fixed to the outer side of the inner membrane. To integrally form a perivalvular leakage preventing device with an inner membrane, the mold 8 further includes a forming frame 84 for forming the inner membrane. The forming frame and the main body 81 may be a separate mold structure or an integral mold structure, as combined with the embodiments above.

Regarding the separate mold structure example, before curing the raw material of the sealing block, a raw material of the inner membrane is applied to a top side of the raw material of the sealing block to form a coating. Then, the raw material of the inner membrane and the raw material of the sealing block material are cured simultaneously. Specifically, the aforementioned step C3 in the molding method shall be replaced by the following steps:
C3-1: covering the main body 81 with the corresponding forming frame 84, aligning the mold cavities on the working surface with the hollowed region of the forming frame;
C3-2: applying a non-foamed PU coating within the hollowed region of the assembled mold;
C3-3: proceeding with curing as described in step C3 above.

In step C2 mentioned above, the number of the pulling wires may be one or multiple, evenly distributed over the raw material of the sealing block. The arrangement of the pulling wires may be combined with the various embodiments described above.

Referring to FIG. 80 to FIG. 81, some embodiments of the present disclosure provide a perivalvular leakage preventing device for a prosthetic implant. The prosthetic implant includes a generally tubular stent 162 with grid structures. The perivalvular leakage preventing device 2 includes a plurality of sealing blocks 21 and an inner membrane 26. The plurality of sealing blocks are arranged circumferentially around the stent. Each sealing block employs a deformable structure and is embedded into a corresponding grid structure. The plurality of sealing blocks are fixed to one side of the inner membrane. In a preferred approach, the inner membrane and the plurality of sealing blocks are made of the same material to enhance adhesion. Other specific features and processes may be combined with the descriptions above. This embodiment primarily omits the pulling wire(s).

An embodiment of the present disclosure further provides a perivalvular leakage preventing skirt. At least a portion of the perivalvular leakage preventing skirt has a flexible substrate, and at least a portion has a reinforcement component in the form of a fabric. The flexible substrate and the reinforcement component have overlapping regions.

The perivalvular leakage preventing skirt is a flexible sheet. Different regions of the sheet may have a material composition chosen from one of the following:
a) the flexible substrate only;
b) the reinforcement component only; or
c) both the flexible substrate and the reinforcement component.

The reinforcement component is used to increase the suturing strength of the perivalvular leakage preventing skirt. In case with only the flexible substrate, the flexible substrate is prone to tearing for sutures passing through the flexible substrate. By providing the reinforcement component, which bears the tensile force from the sutures, the suture strength at the sutured areas of the perivalvular leakage preventing skirt is increased, preventing damage to the skirt from suture forces.

The reinforcement component may be continuously distributed or distributed discretely only in regions requiring suturing or where sutures might pass through. It is sufficient for the reinforcement component to reinforce the region around the needle holes created by the sutures.

Regions where sutures pass through may be regions where the flexible substrate and the reinforcement component overlap, or may be regions where only the reinforcement component exists.

Referring to FIG. 84 and FIG. 87, a perivalvular leakage preventing skirt, the perivalvular leakage preventing skirt includes:
a flexible substrate 92, made of polyurethane membrane;
a reinforcement component 95, in the form of a fabric. At least a portion of the reinforcement component and at least a portion of the flexible substrate are fixedly overlapped.

The perivalvular leakage preventing skirt is formed from a polyurethane membrane with a smaller thickness as the flexible substrate and replaces the porcine pericardium material. The reinforcement component is in fabric form, which does not significantly increase the thickness of the flexible substrate while effectively reinforcing the sutured regions.

In this disclosure, the flexible substrate, as a pre-formed sheet, is fixedly connected to the reinforcement component. Alternatively, the flexible substrate is prepared on the reinforcement component by mold casting to achieve a fixed connection between the flexible substrate and the reinforcement component. This excludes the scenario in which a polyurethane solution is coated onto the reinforcement component, and the cured polyurethane solution forms the polyurethane membrane.

Polyurethane membranes formed by coating are typically thin, and a uniform thickness is difficult to control. For this disclosure, the flexible substrate, as a part of the skirt, demands both a small thickness and a high degree of thickness uniformity. Otherwise, regions with thinner thickness are highly susceptible to damage due to stress concentration. The regions of the perivalvular leakage preventing skirt consisting solely of the flexible substrate require the polyurethane membrane to have a specific and uniform thickness, which is difficult to achieve via coating methods, particularly in terms of uniformity.

At least a portion of the reinforcement component extends beyond the flexible substrate. That is, at least a portion of the reinforcement component is not fixedly overlapped with the flexible substrate. From another perspective, the portion of the reinforcement component extending beyond the flexible substrate is neither fixed to nor overlapping with the flexible substrate.

Referring to FIG. 88 and FIG. 89, the perivalvular leakage preventing skirt 90 further includes a sealing assembly 91 fixed to the flexible substrate 92. The sealing assembly employs a deformable structure.

In this disclosure, the flexible substrate, the sealing assembly, and the reinforcement component are all made from medical-grade raw materials, such as polyurethane, etc. The perivalvular leakage preventing skirt exhibits excellent biocompatibility, thereby avoiding thrombogenicity.

The elongation at break of the flexible substrate is not less than 200%. Optionally, the elongation at break of the flexible substrate is not less than 300%. Optionally, the elongation at break of the flexible substrate is 200% to 500%.

The tensile strength of the flexible substrate is 30-100 MPa. Preferably, the tensile strength of the flexible substrate is 40-100 MPa. Preferably, the tensile strength of the flexible substrate is 50-100 MPa. Preferably, the tensile strength of the flexible substrate is 50-90 MPa. Preferably, the tensile strength of the flexible substrate is 50-80 MPa.

The sealing assembly is made of polyurethane with an open-pore structure.

The sealing assembly is composed of polyurethane with an open-pore structure, imparting excellent compression resilience and a very high specific surface area to the material. During compression and deploying, the sealing assembly may be compressed to a small size, thereby minimizing its overall delivery profile.

Due to the open-pore structure, after implantation in the body, the sealing assembly absorbs blood and expands into an arbitrary shape according to the physiological environment. Furthermore, the internal pore channels of the sealing assembly provide sites for cell adhesion, facilitating tissue regeneration.

By controlling the pore structure within the sealing assembly, and combining multidisciplinary technical principles such as mechanics, bionics, and finite element analysis, the stress distribution on the sealing assembly is analyzed to optimize its structural design. By regulating the pore structure and the pore size within the sealing assembly, it is made to meet acceptable standards for tensile strength, compression strength, and bonding strength, while also benefiting cell attachment and tissue growth. This promotes the apposition of the skirt to the perivalvular tissue, enabling the preparation of a perivalvular leakage preventing skirt with good mechanical properties and biocompatibility that meets implantability requirements, thereby achieving an active perivalvular leakage preventing function.

The sealing assembly and the flexible substrate are made of the same polyurethane.

The polyurethane employed in both the sealing assembly and the flexible substrate needs to have good mechanical properties, biostability, hemocompatibility, and environmental stress crack resistance, making it suitable for long-term cardiovascular implantation and ensuring safety. The sealing assembly and the flexible substrate are made of the same polyurethane, with the only distinction being that the flexible substrate is formed by direct film formation of polyurethane, whereas the sealing assembly possesses a porous structure.

Both the sealing assembly and the flexible substrate employ the same implantable polyurethane materials, including but not limited to Elast-Eon, LifePolymer, POSS-PCU, and Chronosil, etc.

The pores within the sealing assembly are communicated with each other and have uniform pore size. The pore sizes within the sealing assembly are relatively uniform, and the pores are communicated with each other, imparting the sealing assembly with excellent compression resilience and a very high specific surface area.

The porosity of the sealing assembly is 50%-95%. For example, the porosity is 60%-95%. As another example, the porosity is 70%-90%.

The porosity of the sealing assembly determines its compression resilience to a certain extent. Higher porosity generally leads to better compressibility but may compromise resilience and strength. An appropriate porosity can balance all aspects of performance, ensuring suitable compressibility, resilience, and strength.

The fabric is a flexible fabric. Although the fabric serves as a reinforcement component, it still needs to possess good flexibility. At a minimum, after being combined with the flexible substrate, the fabric does not significantly reduce the flexibility of the flexible substrate.

A thickness of the fabric ranges from 0.04 mm to 0.2 mm. A pore size of the fabric is not less than 0.03 mm*0.03 mm. When punctured at a distance of 5 mm from an edge of the fabric using a suture needle with a diameter of 0.4 mm and PTFE suture, the measured suture retention force value shall be not less than 2.5 N.

The raw material of the fabric is one of polyester (PET), polyamide (PA), or polyetheretherketone (PEEK). For example, the raw material of the fabric is polyester. The fabric may be a weft-knitted fabric.

Defined relative to the intended blood flow direction in the physiological environment, the perivalvular leakage preventing skirt includes an inflow end and an outflow end. The portion of the reinforcement component that extends beyond the flexible substrate is located at the outflow end.

The portion of the reinforcement component extending beyond the flexible substrate is located at the outflow end; that is, this portion of the reinforcement component extending beyond the flexible substrate is positioned between the inflow and outflow ends, closer to the outflow end. The reinforcement component is located at the outflow end, and the outflow end is mainly configured for attachment to the stent following sutured to the leaflets. The reinforcement component mainly serves to increase suture strength.

The sealing assembly is located at the inflow end of the perivalvular leakage preventing skirt. That is, the sealing assembly is positioned between the inflow and outflow ends, closer to the inflow end.

Along the axial direction of the perivalvular leakage preventing skirt, the sealing assembly is spaced apart from the reinforcement component. A distance between the sealing assembly and the reinforcement component is 3-20 mm. Preferably, the distance between the sealing assembly and the reinforcement component is 10-15 mm.

Since the shapes of both the sealing assembly and the reinforcement component are irregular, the definition of the distance D is as shown in FIG. 88 and FIG. 89. The distance D, measured along the axial direction of the stent between the nearest points of the sealing assembly 130 and the reinforcement component 120, is 3-20 mm. Preferably, the distance between the sealing assembly and the reinforcement component is 10-15 mm.

The sealing assembly and the reinforcement component are located in different regions of the flexible substrate and do not contact each other, thus not interfering with one another.

The sealing assembly and the reinforcement component may be located on the same side of the flexible substrate, or the sealing assembly and the reinforcement component may be located on different sides of the flexible substrate. That is, when laid flat, the flexible substrate has two opposite sides. The sealing assembly and the reinforcement component may be on the same side or on different sides.

The flexible substrate has a first edge and an opposite second edge. The first edge corresponds to the position of the inflow end of the perivalvular leakage preventing skirt. The second edge is configured for attachment to the stent after being sutured to the leaflets, and the second edge is closer to the outflow end of the stent relative to the first edge.

The reinforcement component has a third edge and an opposite fourth edge. The fourth edge of the reinforcement component has the same shape as the second edge of the flexible substrate.

Referring to FIG. 82, the flexible substrate 92 has a first edge 921 and a second edge 922. Under physiological conditions, the first edge 921 is closer to the inflow end of the stent, and the second edge 922 is closer to the outflow end of the stent. The second edge 922 has a curved portion adapted to the shape of the stent and is fixed to the stent after being sutured to the leaflets. The shape of the flexible substrate is not limited to that shown in FIG. 82; the specific shape varies depending on design of the stent. FIG. 82 primarily illustrates the relative positional relationship between the first edge and the second edge.

Referring to FIG. 83 and FIG. 86, the reinforcement component 200 has a third edge 951 and a fourth edge 952. The shape of the fourth edge 952 of the reinforcement component is the same as the shape of the second edge 922 of the flexible substrate 92. The shape of the reinforcement component is not limited to that shown in FIG. 83 and FIG. 86; the specific shape is selected as needed. FIG. 83 and FIG. 86 primarily distinguish the relative positional relationship between the third edge and the fourth edge.

The shape of the third edge and the fourth edge of the reinforcement component are the same. The reinforcement component is generally wavy and has the same width throughout.

Referring to FIG. 83, the depiction of the reinforcement component 95 as generally wavy and having a constant width W is for illustrative purposes only to describe a general shape and should not be construed as a limiting feature. Particularly in the regions where the reinforcement component forms turns, the width is designed to vary.

The third edge of the reinforcement component is a smooth arc.

Referring to FIG. 86, the third edge 951 of the reinforcement component 95 is a smooth arc. When the third edge is in use, its ends are connected to form a ring-like structure. When the third edge 951 of the reinforcement component 95 is configured as an arc, the component 95 has a larger area, facilitating its handling during fixed attachment to the flexible substrate.

The fourth edge of the reinforcement component is aligned with the second edge of the flexible substrate.

Referring to FIG. 84, the second edge 922 of the flexible substrate 92 shown in FIG. 82 and the fourth edge 122 of the reinforcement component 95 shown in FIG. 83 are aligned and fixedly joined to each other. The overlapping region of the reinforcement component 95 and the flexible substrate 92 (indicated by the hatched area in the figure) is adapted to permit the passage of sutures. The presence of the reinforcement component increases the strength of the flexible substrate, preventing tearing by the sutures.

The flexible substrate has a first edge and an opposite second edge, in which the second edge is closer to the outflow end of the stent relative to the first edge.

The reinforcement component has a third and an opposite fourth edge, and the fourth edge of the reinforcement component extends beyond the second edge of the flexible substrate, forming an extension part for suturing.

Referring to FIG. 87, the fourth edge 952 of the reinforcement component 95 extends beyond the second edge 922 of the flexible substrate 92, forming an extension part 953 (the hatched area in the figure). That is, the extension part 953 consists only of the reinforcement component 95 and is devoid of the flexible substrate 92. The extension part 953 is adapted to permit the passage of sutures. Forming The extension part 953 is formed solely from the reinforcement component 95. Therefore, on one hand, the profile in the compressed state is reduced, and on another hand, sutures pass through the reinforcement component 95 without penetrating the flexible substrate, making suturing easier. This also avoids backside protrusions after needle passage, prevents suture abrasion, and does not affect valve durability.

The sealing assembly consists of at least one protrusion fixed onto the flexible substrate. The sealing assembly may be either sealing blocks arranged at intervals or a ring structure extending continuously in the circumferential direction.

Using a stent with rhombic grids as an example, and referring to FIG. 88 and FIG. 89, the protrusion is shaped to correspond to the rhombic-shaped stent grid. Other shapes for the protrusion are also applicable.

The reinforcement component and the flexible substrate are connected by at least one of the following methods: solvent bonding, solution bonding, thermal fusion bonding, or suture stitching.

The solvent used for solvent bonding is a good solvent for polyurethane that is miscible with water.

The solvent used for solvent bonding is at least one of dimethyl sulfoxide (DMSO), N,N-Dimethylformamide (DMF), and N,N-Dimethylacetamide (DMAC).

The solution used for solution bonding is a polyurethane solution with a concentration of 5-20 wt.%. The solvent for the polyurethane solution is at least one of dimethyl sulfoxide (DMSO), N,N-Dimethylformamide (DMF), and N,N-Dimethylacetamide (DMAC). Preferably, the concentration of the polyurethane solution is 5-10 wt.%.

When using a solution or solvent for bonding, it is preferable to apply it locally using a syringe followed by finger pressing, or to use tools like a silicone rod or cotton swab for application. After the bonding region is fully wetted by the solvent or solution, it is dried at 50-70°C to complete the bonding.

The temperature for thermal fusion bonding is 50-70°C, and the heating time is 0.5-2 hours. Preferably, the temperature for thermal fusion bonding is 50-70°C, and the heating time is 1-2 hours.

The sealing assembly and the flexible substrate are connected via a bonding region. The bonding region is formed by contacting adjacent parts of the flexible substrate and the protrusion in a solution state, allowing for mutual interpenetration, followed by curing.

The order of preparing the sealing assembly and the flexible substrate is not limited. The sealing assembly may be prepared first, followed by the flexible substrate, or vice versa.

The raw material for preparing the flexible substrate is a polyurethane solution. The raw material for preparing the sealing assembly is a pre-mix of solid particles and polyurethane solution. Both the raw materials for the flexible substrate and the sealing assembly contain a solvent for polyurethane. If the flexible substrate is prepared first, when the raw material of the sealing assembly contacts the flexible substrate, the polyurethane solvent in the raw material of the sealing assembly will dissolve part of the flexible substrate. Curing then simultaneously achieves the formation of the sealing assembly and the bonding between the flexible substrate and the sealing assembly. Similarly, if the sealing assembly is prepared first, when the raw material of the flexible substrate contacts the sealing assembly, the solvent in the flexible substrate raw material will dissolve part of the sealing assembly. Curing then simultaneously achieves the formation of the flexible substrate and the bonding between the flexible substrate and the sealing assembly.

The boundary of the bonding region is relatively blurry but can still be distinguished from the more uniform texture of the flexible substrate 92 and the sealing assembly 91. Unlike the flexible substrate 92 and the sealing assembly 91, the distinct transition region between the flexible substrate and the sealing assembly is the bonding region. The flexible substrate 92 and the sealing assembly 91 may be either pre-cured formed parts, or may be in solution form within a mold. The solutions of the adjacent portions of the flexible substrate 92 and the sealing assembly 91 interpenetrate and cure to form the bonding region.

The bonding region is located at the interface between the flexible substrate (or the polyurethane solution filling the mold in the shape of the flexible substrate) and the sealing assembly (or the pre-mix filling the mold in the shape of the sealing assembly). A part of the flexible substrate and the sealing assembly each interpenetrate via solution contact and, upon curing, yield the bonding region. As both belong to the polyurethane material, bonding interface of the flexible substrate and the sealing assembly forms a polyurethane bond within the junction of the sealing assembly and the flexible substrate. However, this interface, being of the same material and sufficiently bonded, does not form a clear, flat bonding interface but is rather blurry, uneven, and fully integrated. This indicates that the formation of this interface results in good bonding strength and a stable bonded structure.

The thickness T1 of the flexible substrate is 0.03 mm - 0.05 mm. The thickness T2 of the reinforcement component is 0.04 mm - 0.2 mm. The thickness T3 at the location where the flexible substrate and the reinforcement component are overlapped and fixed is 0.05 mm - 0.24 mm, and T1, T2, T3 satisfy the constraint: 0.05mm≥T1+T2-T3≥0.01 mm.

The thickness of the flexible substrate is significantly less than that of porcine pericardium. When the flexible substrate is bonded to the reinforcement component, part of the flexible substrate dissolves. The polyurethane molecular chains of the flexible substrate embed into the pore structure of the fabric of the reinforcement component, followed by curing. The successful formation of the composite (i.e., bonding) can be evaluated by the final thickness after the components are joined. If the thickness at the bonded region of the reinforcement component and the flexible substrate is the sum of their individual thicknesses minus 0.01-0.05 mm, the bonding can be considered to meet an acceptable standard. The acceptable range for the thickness after bonding the reinforcement component and the flexible substrate is 0.05 mm-0.24 mm.

An embodiment of the present disclosure further provides a method for producing a perivalvular leakage preventing skirt, including:
attaching the reinforcement component and the sealing assembly respectively to the flexible substrate; and
cutting the reinforcement component and the flexible substrate into a predetermined shape.

The attachment of the reinforcement component and the sealing assembly respectively to the flexible substrate is performed in any one of the following sequences:
a) attaching the reinforcement component and then the sealing assembly sequentially to the flexible substrate;
b) attaching the sealing assembly and then the reinforcement component sequentially to the flexible substrate.

The cutting of the reinforcement component and the flexible substrate into the predetermined shape is performed in any one of the following sequences:
1) cutting the flexible substrate and the reinforcement component separately, before attaching the reinforcement component to the flexible substrate;
2) cutting the flexible substrate and the reinforcement component simultaneously, after attaching the reinforcement component to the flexible substrate.

The producing method of the sealing assembly includes:
mixing a polyurethane solution with solid particles to obtain a pre-mix;
curing and washing the pre-mix, thereby obtaining the sealing assembly having a porous structure.

The solid particles have a particle size of 80-120 mesh. The solid particles are insoluble in the solvent of the polyurethane solution but soluble in water. A porous structure may be obtained by removing the solid particles via water washing.

The solid particles have a relatively uniform particle size, and the content of solid particles in the pre-mix is high, resulting in a state similar to quicksand. The solid particles contact each other and are immersed in the polyurethane solution. A soft 3D porous framework structure with communicated internal channels, formed by solvent casting/particulate leaching, which provides the material with excellent compression resilience and a very high specific surface area.

An embodiment of the present disclosure further provides a prosthetic implant, including:
a stent, being a deformable tubular structure with a sidewall having hollowed region, the interior of the stent forming a blood flow channel;
leaflets, connected to the stent for controlling the blood flow channel; and
the perivalvular leakage preventing skirt according to any embodiment of the present disclosure. The perivalvular leakage preventing skirt is located substantially inside the stent. The outflow end of the perivalvular leakage preventing skirt is connected to the leaflets. The sealing assembly protrudes outward from the stent via the hollowed region of the stent.

The sealing assembly is located at the inflow end of the stent. The height of the sealing assembly along the axial direction corresponds to 1-2 rows of grids at the inflow end of the stent.

After the perivalvular leakage preventing skirt is fixed to the stent, the height of the sealing assembly along the axial direction covers 1-2 rows of grids at the inflow end of the stent. This ensures that the sealing assembly matches the position of the annulus after implantation, maximizing the along the perivalvular leakage preventing effect.

An embodiment of the present disclosure further provides a method for producing a prosthetic implant, including:
connecting the leaflets and the perivalvular leakage preventing skirt according to any embodiment of the present disclosure to form a tubular pre-assembly; and
assembling the pre-assembly into the stent of the prosthetic implant.

The following examples further illustrate the structural characteristics of the perivalvular leakage preventing skirt and the procedural details of the producing method.

### Example 1

A method for producing a perivalvular leakage preventing skirt includes the following steps:
① preparing PU solution (i.e., polyurethane solution, solvent: dimethyl sulfoxide) with a concentration of 10.0 wt.% and preheating the solution to 60°C;
② spreading the preheated PU solution evenly in a PU membrane molding mold, placing the mold in an air drying oven at 60°C for 7 hours to form, and demolding to obtain the PU membrane (i.e., polyurethane membrane, also the flexible substrate);
③ mixing PU solution with sodium chloride of 80-120 mesh in a proportion (sodium chloride accounting for 75 wt.%) to prepare a pre-mix;
④ filling the mold with the pre-mix and screeding the pre-mix, then covering the mold with the PU membrane. After curing, the sealing assembly (hereinafter referred to as PU foam) is obtained, and the sealing assembly is connected to the PU membrane, yielding the skirt;
⑤ cutting the skirt into the shape shown in FIG. 82 after formed, cleaned, and dried (the shape is determined according to the specific stent shape);
⑥ cutting the fabric according to FIG. 83 (the fabric is cut to form the reinforcement component; the shape of the fabric fabricated by cutting is determined according to the shape of the stent and requirements of the reinforcement region);
⑦ laying the PU membrane with the sealing assembly flat on a glass plate (preferably with the PU foam facing down; facing up is an alternative embodiment); covering the fabric on the PU membrane with an edge of the fabric aligned with the top edge of the PU membrane (as shown in FIG. 84); and applying dimethyl sulfoxide (DMSO) onto the fabric for adhesion;
⑧ after the bonding region is fully wetted by DMSO, placing the entire assembly into an air drying oven at 60°C and heating for 1 hour to obtain the perivalvular leakage preventing skirt.

### Example 2 (the fabric bonded with the flexible substrate before cutting)

A method for producing a p perivalvular leakage preventing skirt includes the following steps:
① preparing PU solution (i.e., polyurethane solution, solvent: dimethyl sulfoxide) with a concentration of 10.0 wt.% and preheating the solution to 60°C;
② spreading the preheated PU solution evenly in a PU membrane molding mold, placing the PU solution in an air drying oven at 60°C for 7 hours to form, and demolding to obtain the PU membrane (i.e., polyurethane membrane, also the flexible substrate);
③ mixing PU solution with 80-120 mesh sodium chloride in a proportion (sodium chloride accounting for 75 wt.%) to prepare a pre-mix;
④ filling the mold with the pre-mix, screeding the pre-mix, and then covering the pre-mix with the PU membrane. After curing, the sealing assembly (hereinafter referred to as PU foam) is obtained, and the sealing assembly is connected to the PU membrane, yielding the skirt;
⑤ laying the PU membrane with the sealing assembly flat on a glass plate (preferably with the PU foam facing down; facing up is an alternative embodiment), placing the fabric to cover an area at least 3mm above the rhombic grid foam;
⑥ after the bonding area is fully wetted by DMSO, applying dimethyl sulfoxide (DMSO) to the fabric, placing the entire assembly into an air drying oven at 60°C to heat for 1 hour, and bonding the fabric to the polyurethane membrane;
⑦ cutting the entire assembly from step ⑥ according to the drawing in FIG. 82, meaning the flexible substrate and the reinforcement component are cut to have identical shapes, to obtain a perivalvular leakage preventing skirt with a stitched-on fabric reinforcement.

Compared to Example 1, this embodiment involves bonding before cutting the fabric. The larger fabric area makes handling easier and increases the success rate of bonding.

### Example 3 (finally producing the sealing assembly)

A method for producing a perivalvular leakage preventing skirt includes the following steps:
① preparing PU solution (i.e., polyurethane solution, solvent: dimethyl sulfoxide) with a concentration of 10.0 wt.% and preheating the solution to 60°C;
② spreading the preheated PU solution evenly in a PU membrane molding mold, placing the PU solution in an air drying oven at 60°C for 7 hours to form, and demolding to obtain the PU membrane;
③ cutting the PU membrane according to FIG. 82 for later use, and cutting the fabric according to FIG. 83 for later use;
④ laying the PU membrane flat on a glass plate, covering the PU membrane with the fabric, aligning an edge of the fabric with the top edge of the PU membrane (as shown in FIG. 84), and applying dimethyl sulfoxide (DMSO) onto the fabric for adhesion;
⑤ after the bonding region fully wetted by DMSO, placing the entire assembly into an air drying oven at 60°C to heat for 1 hour, thereby obtaining a fabric-reinforced polyurethane membrane;
⑥ mixing PU solution with 80-120 mesh sodium chloride (sodium chloride accounting for 75 wt.%) in proportion to prepare a pre-mix;
⑦ filling the mold for forming the sealing assembly with the pre-mix and screeding the pre-mix, then covering the fabric-reinforced polyurethane membrane prepared in step ⑤. After curing, cleaning, and drying, the perivalvular leakage preventing skirt is obtained.

In this embodiment, when bonding the fabric, the sealing assembly is not yet attached to the polyurethane membrane. This makes the bonding process between the fabric and the membrane easier to perform, increases the bonding success rate, and avoids potential damage to the sealing assembly during bonding.

### Example 4

A method for producing a perivalvular leakage preventing skirt includes the following steps:
① preparing PU solution (i.e., polyurethane solution, solvent: dimethyl sulfoxide) with a concentration of 10.0 wt.% and preheating the solution to 60°C;
② spreading the preheated PU solution evenly in a PU membrane molding mold, placing the PU solution in an air drying oven at 60°C for 7 hours to form, and demolding to obtain the PU membrane (i.e., polyurethane membrane, also the flexible substrate);
③ mixing PU solution with 80-120 mesh sodium chloride in a proportion (sodium chloride accounting for 75 wt.%) to prepare a pre-mix;
④ filling the mold with the pre-mix, screeding the pre-mix, and then covering the pre-mix with the PU membrane. After curing, the sealing assembly (hereinafter referred to as PU foam) is obtained, and the sealing assembly is connected to the PU membrane, yielding the skirt;
⑤ after the skirt is formed, cleaned, and dried, cutting the skirt according to FIG. 85;
⑥ cutting the fabric (PET fabric) according to FIG. 86;
⑦ laying the cut PU membrane flat on a glass plate (PU foam facing up or down), covering the PU membrane with the fabric, and aligning the 1.5mm gap reserved on the left and right sides (the gaps are set for aligning the fabric and the PU membrane);
⑧ dropping an appropriate amount of DMSO onto the fabric, pressing with a finger or other tool to laminate, and after the bonding region fully wetted by DMSO, placing the entire assembly **in** an air drying oven at 60°C to heat for 1 hour, thereby obtaining the perivalvular leakage preventing skirt.

In this example, the region for suturing to the leaflets is composed exclusively of the PET fabric (with a width of 1.5mm), and the remaining suturing region is composed solely of the PU membrane. There is no need for sutures to pass through the fabric-reinforced area (the composite region of PU membrane and PET fabric). This makes suturing easier, avoids backside protrusions after needle passage, prevents suture abrasion, and does not affect valve durability.

### Example 5

A method for producing a perivalvular leakage preventing skirt includes the following steps:
① preparing PU solution (i.e., polyurethane solution, solvent: dimethyl sulfoxide) with a concentration of 10.0 wt.% and preheating the solution to 60°C;
② spreading the preheated PU solution evenly in a PU membrane molding mold, placing the PU solution in an air drying oven at 60°C for 7 hours to form, and demolding to obtain the PU membrane (i.e., polyurethane membrane, also the flexible substrate);
③ cutting the PU membrane according to FIG. 85 for later use, and cutting the fabric (PET fabric) according to FIG. 86 for later use;
④ laying the cut PU membrane flat on a glass plate, covering the PU membrane with the fabric, and aligning the 1.5mm gap reserved on the left and right sides;
⑤ dropping an appropriate amount of DMSO onto the fabric, pressing with a finger or other tool to laminate, and after the bonding region fully wetted by DMSO, placing the entire assembly **in** an air drying oven at 60°C to heat for 1 hour, thereby obtaining a fabric-reinforced polyurethane membrane;
⑥ mixing PU solution with 80-120 mesh sodium chloride in a proportion (sodium chloride accounting for 75 wt.%) to prepare a pre-mix;
⑦ filling the mold for forming the sealing assembly with the pre-mix, screeding the pre-mix, and then covering the fabric-reinforced polyurethane membrane prepared in step ⑤. After curing, cleaning, and drying, the perivalvular leakage preventing skirt is obtained.

In this example, the region for suturing to the leaflets is composed exclusively of the PET fabric (with a width of 1.5mm), and the remaining suturing region is composed solely of the PU membrane. There is no need for sutures to pass through the fabric-reinforced area (the composite region of PU membrane and PET fabric). This makes suturing easier, avoids backside protrusions after needle passage, prevents suture abrasion, and does not affect valve durability.

In another embodiment, the perivalvular leakage preventing skirt is not only located inside the stent; at least a portion of the perivalvular leakage preventing skirt may be folded over the inflow end of the stent to the outside of the stent and fixed thereto.

For example, the flexible substrate is substantially located inside the stent, while the reinforcement component is substantially located outside the stent. The overlapping region of the flexible substrate and the reinforcement component is approximately at the inflow end of the stent, for instance, wrapped around an end of the stent.

### Example 6

A method for producing a perivalvular leakage preventing skirt includes the following steps:
preparing PU solution (i.e., polyurethane solution, solvent: dimethyl sulfoxide) with a concentration of 10.0 wt.% and preheating the solution to 60°C;
spreading the preheated PU solution evenly in a PU membrane molding mold, placing the PU solution in an air drying oven at 60°C for 7 hours to form, and demolding to obtain the PU membrane (i.e., polyurethane membrane, also the flexible substrate);
cutting the PU membrane according to FIG. 82 for later use, and cutting the fabric (PET fabric) according to FIG. 90 for later use;
bonding the edges of the PU membrane and the fabric, e.g., using DMSO as the adhesive. Alternatively, PU solution may be used as the adhesive, or thermal compression bonding or direct suturing may be employed to join the PU membrane and the fabric, obtaining the fabric-reinforced polyurethane film as shown in FIG. 91;
mixing PU solution with 80-120 mesh sodium chloride in a proportion (sodium chloride accounting for 75 wt.%) to prepare a pre-mix;
filling the mold for forming the sealing assembly with the pre-mix, screeding the pre-mix, and then covering above-described fabric-reinforced polyurethane film, then cover it with the aforementioned fabric-reinforced polyurethane film, proceeding with the skirt forming process. After cleaning and drying, the fabric-reinforced perivalvular leakage preventing skirt is obtained.

After the fabric-reinforced perivalvular leakage preventing skirt is connected to the stent of the prosthetic implant, the PU membrane is located on the inner side of the stent. The sealing assembly faces outward and can protrude through the hollowed region of the stent. The fabric portion is everted to the outside of the stent and fixed thereto. The PU membrane and the fabric form a pouch structure encapsulating the sealing assembly (i.e., the PU foam material).

In the prior art, the skirt is generally fixed to the stent by suturing. Skirts are typically chosen in a fabric form suitable for suturing to achieve good suture strength, but this also increases the size during retrieval and the risk of folding damage. Currently, to further improve the sealing effect, some skirts incorporate deformable materials on the outer side, such as foam materials. However, material selection for valve skirts in the interventional field is stringent, making it difficult to balance the inherent strength of the fabric, the strength of the deformable material, and a strong bond between them.

Based on above, some embodiments of the present disclosure further provide a self-adaptive perivalvular leakage preventing component. The self-adaptive perivalvular leakage preventing component may be directly installed on the stent of a prosthetic implant. It may also be combined with the reinforcement component described in the previous embodiments to enhance strength of a structure, resulting in a perivalvular leakage preventing skirt as shown in FIG. 88 or FIG. 89, which is then installed on the stent of the prosthetic implant.

One embodiment of the present disclosure provides a self-adaptive perivalvular leakage preventing component. The perivalvular leakage preventing component includes: a sheet-like and stretchable flexible substrate, and a sealing assembly attached to the flexible substrate. The sealing assembly consists of one or more protrusions, each having a porous structure. Both the flexible substrate and the protrusions are made of polyurethane material. The connection between the flexible substrate and the protrusions is achieved through the curing of a polyurethane solution.

When applied to a prosthetic implant, such as a prosthetic heart valve, the flexible substrate may serve as the outer skirt and/or the inner skirt. The protrusions may serve as sealing blocks, with at least a portion protruding outward from the stent of the prosthetic heart valve.

The flexible substrate (serving as an inner membrane for fixed to the stent) and the protrusions (serving as sealing blocks for sealing the gap between the valve and surrounding tissue) are made from the same polyurethane material. They may be co-cured from a liquid state, or they may be cured separately and then bonded using a curing polyurethane solution to form a joint region. The flexible substrate and the protrusions contact in solution form, interpenetrate, and then cure to form the join region, thereby creating a bonding interface with the same material. Since both the flexible substrate and the protrusions at the bonding interface are polyurethane material, no distinct phase interface prone to delamination or peeling under external force is formed.

In the prior art, covering materials on the stent, including perivalvular leakage preventing components, are typically connected to the stent by suturing, and the perivalvular leakage preventing components are usually in fabric form. In the present disclosure, the perivalvular leakage preventing component is not in fabric form. Both the flexible substrate and the protrusions are prepared by casting, and they are made of the same polyurethane material, offering at least one of the following beneficial effects:
(a) In the prior art, if a protrusion detaches and migrates within the vessel, it is likely to cause fatal complication, such as thrombosis. In this disclosure, the protrusions and the flexible substrate are made of the same material, ensuring good compatibility and stronger interfacial bonding strength. This eliminates the risk of protrusion detachment, resulting in better safety for the perivalvular leakage preventing component.
(b) In the prior art, the perivalvular leakage preventing components often use fabrics like PET for easier suturing. However, PET material is relatively stiff. During stent deformation, PET exhibits insufficient deformability to conform to an object. The expansion and folding of PET wrinkle positions, as well as the interaction between the PET and the stent, are likely to cause interference; this can easily damage the leaflets or the membrane.
(c) The flexible substrate and the protrusions are made of the same polyurethane material, allowing them to be formed under the same process conditions, which simplifies the process and improves operational convenience.
(d) The polyurethane material employed in both the flexible substrate and the protrusions has a high elongation at break, allowing it to self-adapt to stent deformation. During stent deformation, the flexible substrate and protrusions deform in compliance, without imposing resistance during stent deformation. Consequently, they do not interfere with the stent's crimping and deployment.
(e) The protrusions have a porous structure. During prosthetic valve compression, the protrusions can reduce their occupied volume by compressing the space within their own pore structure, minimizing the increase in delivery system profile. After deployment in the body and release of external force, the protrusions can restore the space within the pore structure, expanding back to their initial size to achieve good perivalvular leakage preventing. Simultaneously, they can facilitate good endothelialization sealing under blood flow across the heart valve.
(f) In the prior art, when adhesives are used to bond different parts of the membrane on the stent, the adhesive is composed of a material (e.g., PET or others) different from the materials of the membrane. The bonding interface tends to be rigid, becoming a stress concentration point. During crimping and deploying the stent, this interface easily becomes the main stress-bearing point. Furthermore, the compliant deformation performance of the interface is poor, potentially causing hindrance during stent compression and expansion and leading to damage to the leaflets or the membrane.
   In this disclosure, both the flexible substrate and the protrusions are made of polyurethane. The polyurethane solution is cured to connect the flexible substrate and the protrusions. The adjacent parts of the flexible substrate and the protrusions contact in solution form, and the solutions at the adjacent parts of the flexible substrate and the protrusions interpenetrate. During the subsequent curing process, the solutions is cured using the same manner as that used for the substrate and the protrusions, which involves solvent removal by evaporation or dissolution. The connection region thus forms a phase structure with properties closer to those of the flexible substrate and protrusions, ensuring relatively uniform overall physical properties of the perivalvular leakage preventing component. This results in good overall compliance of the perivalvular leakage preventing component during stent crimping, loading, and deployment.
(g) In the prior art, sealing is often achieved by adhesive material penetrating through the fabric mesh of the skirt to form protrusions. Although the fabric offers high strength, its elasticity and softness are poor; it is prone to tearing and deformation under force. In contrast, the integrated polyurethane material possesses better softness and resilience, offering better adaptability to the motion of the valve within the heart.

The elongation at break of the flexible substrate is not less than 200%. Preferably, the elongation at break of the flexible substrate is not less than 300%. Further preferably, the elongation at break of the flexible substrate is 200%-500%.

The pore diameter of the protrusions is 5-500 micrometers. Preferably, the pore diameter of the protrusions is 5-450 micrometers. Preferably, the pore diameter of the protrusions is 5-400 micrometers. Preferably, the pore diameter of the protrusions is 5-350 micrometers. Preferably, the pore diameter of the protrusions is 5-300 micrometers. Preferably, the pore diameter of the protrusions is 5-250 micrometers.

The protrusions have a controllable porosity. The protrusions have a uniform porosity. The protrusions have an adjustable porosity.

The porosity of the protrusions is 50%-95%. Preferably, the porosity of the protrusions is 60% - 95%. Preferably, the porosity of the protrusions is 70%-90%.

The protrusions have an open-pore structure and/or a closed pore structure. Preferably, the protrusions have an open-pore structure.

The average thickness of the flexible substrate is 0.02-0.20 mm. Preferably, the average thickness of the flexible substrate is 0.02-0.15 mm. Preferably, the average thickness of the flexible substrate is 0.02-0.12 mm.

The tensile strength of the flexible substrate is 30-100 MPa. Preferably, the tensile strength of the flexible substrate is 40-100 MPa. Preferably, the tensile strength of the flexible substrate is 50-100 MPa. Preferably, the tensile strength of the flexible substrate is 50-90 MPa. Preferably, the tensile strength of the flexible substrate is 50-80 MPa.

The flexible substrate is relatively thin and possesses higher tensile strength, allowing it to follow any deformation of the stent without being easily damaged.

An embodiment of the present disclosure provides a self-adaptive perivalvular leakage preventing component. The perivalvular leakage preventing component includes: a sheet-like and stretchable flexible substrate, and one or more protrusions attached to the flexible substrate. Each protrusion has a porous structure. Both the flexible substrate and the protrusions are made of polyurethane material. The flexible substrate and the protrusions are connected via a bonding region. The adjacent parts of the flexible substrate and the protrusions contact in solution form, interpenetrating, and then curing to form the bonding region.

Referring to FIG. 97 and FIG. 98, the thickness d of the bonding region is 0.01-0.03 mm. As shown in FIG. 97, the boundary of the bonding region is relatively blurry but can still be distinguished from the more uniform texture of the flexible substrate 92 and the sealing assembly 91. The sealing assembly 91 constitutes the protrusions. Alternatively, unlike the flexible substrate 92 and the sealing assembly 91, the distinct transition region between the flexible substrate and the sealing assembly is the bonding region. The flexible substrate 92 and the sealing assembly 91 may be either pre-cured formed parts, or may be in solution form within a mold. The solutions of the adjacent portions of the flexible substrate 92 and the sealing assembly 91 interpenetrate and cure to form the bonding region.

The bonding region is located at the interface between the flexible substrate (or the casting material filling the mold in the shape of the flexible substrate) and the protrusions (or the casting material filling the mold in the shape of the protrusions). Parts of each interpenetrate via solution contact and, upon curing, yield the bonding region. Because the flexible substrate and protrusions are made of the same polyurethane material, their bonding interface is essentially a polyurethane-to-polyurethane bond. However, this interface, being of the same material and sufficiently bonded, does not form a clear, flat bonding interface but is rather blurry, uneven, and fully integrated. This indicates that the formation of this interface results in good bonding strength and a stable bonded structure.

An embodiment of the present disclosure provides a self-adaptive perivalvular leakage preventing component made of polyurethane. The perivalvular leakage preventing component includes: a sheet-like and stretchable flexible substrate, and one or more protrusions connected to the flexible substrate. Both the flexible substrate and the protrusions are formed by a casting process. The casting liquid for the protrusions is a polyurethane solution containing solid particles. The casting liquid for the flexible substrate is a polyurethane solution or a polyurethane mixture containing solid particles.

The casting process involves filling a mold with the casting liquid and then curing it. Details of the casting process are provided in subsequent preparation methods and are not repeated here.

The casting liquids for both the protrusions and the flexible substrate are polyurethane mixtures containing solid particles.

An embodiment of the present disclosure provides a self-adaptive perivalvular leakage preventing component. The perivalvular leakage preventing component includes a flexible substrate and protrusions. The porosity of the protrusions is not lower than that of the flexible substrate. The adjacent parts of the flexible substrate and the protrusions contact in solution form, interpenetrating, and curing to form interface between the flexible substrate and the protrusions.

The protrusions and the flexible substrate are made of the same material. "Same material" means all physical and chemical properties are identical; if any property differs, they are considered different materials.

The protrusions and the flexible substrate are both made of polyurethane material.

Both the protrusions and the flexible substrate have a porous structure, and the porosity of the protrusions is not lower than that of the flexible substrate.

Alternatively, the protrusions have a porous structure, while the flexible substrate is a non-porous, dense structure.

An embodiment of the present disclosure provides a method for producing a self-adaptive perivalvular leakage preventing component to obtain the adaptive perivalvular leakage preventing component of the various embodiments of this disclosure. Wherein the producing method includes the following steps performed in any order:
filling a mold with a polyurethane mixture containing solid particles, curing it, and then washing to remove the solid particles, thereby obtaining the protrusions;
utilizing a polyurethane mixture containing solid particles, curing it, and washing it to obtain the flexible substrate. The connection between the flexible substrate and the protrusions is achieved through the curing of a polyurethane solution.

Since both the protrusions and the flexible substrate are made of the same polyurethane material, they can be cured under the same process conditions, resulting in a simple process flow, high efficiency, and ease of implementation.

The protrusion(s) and the flexible substrate may be made one first and then the other, or they may be prepared simultaneously and integrally formed.

Since the protrusions and the flexible substrate are made of the same polyurethane material, there is no distinct phase interface at their connection point, resulting in stronger bonding strength, no risk of protrusion detachment, and better safety.

The polyurethane mixture is prepared by dispersing solid particles in a polyurethane solution. The polyurethane solution used for preparing the polyurethane mixture and the polyurethane solution used for preparing the flexible substrate may be the same or different. In this disclosure, when specifying the polyurethane solution, it applies both to the polyurethane solution used in the polyurethane mixture and to the polyurethane solution used for preparing the flexible substrate. Similarly, when specifying fixed conditions for the polyurethane solution, it applies to both.

In this disclosure, since the polyurethane mixture contains a large amount of insoluble solid particles, the polyurethane mixture exhibits a state similar to quicksand. When the polyurethane mixture contacts a polyurethane solution, the polyurethane solute and solvent within the mixture form a solution phase (the solid particles in the mixture do not affect curing and are dis-considered), which then contacts, interpenetrates with, and cures together with the polyurethane solution. When a polyurethane mixture contacts another polyurethane mixture, the same understanding applies: the polyurethane solute and solvent from each mixture form solution phases (the solid particles are disregarded), which then contact, interpenetrate, and cure together.

An embodiment of the present disclosure provides a method for producing a self-adaptive perivalvular leakage preventing component, including the following steps performed sequentially:
Step 1: curing a polyurethane solution to obtain a flexible substrate;
Step 2: filling a mold with a polyurethane mixture containing solid particles;
Step 3: covering the mold with the flexible substrate, allowing the flexible substrate to contact the polyurethane mixture containing solid particles, and curing the polyurethane mixture containing solid particles, and then washing to remove the solid particles, thereby obtaining the self-adaptive perivalvular leakage preventing component.

This method first prepares the flexible substrate, then prepares the protrusions on the flexible substrate. After the flexible substrate is cured and contacts the polyurethane mixture, the surface of the flexible substrate in contact with the polyurethane mixture is partially dissolved. The dissolved part cures together with the adjacent polyurethane mixture, forming the bonding region.

After the flexible substrate is cured and contacts the polyurethane mixture, and the polyurethane mixture is cured, the connection between the flexible substrate and the protrusions is achieved. Since the flexible substrate and the protrusions are made of the same material, their interfacial bonding strength is better.

An embodiment of the present disclosure provides a method for producing a self-adaptive perivalvular leakage preventing component, including the following steps performed sequentially:
Step 1: filling a mold with a polyurethane mixture containing solid particles, curing it, and washing to remove the solid particles, thereby obtaining the protrusions;
Step 2: bringing the protrusions into contact with a polyurethane solution, and curing the polyurethane solution to obtain the self-adaptive perivalvular leakage preventing component.

This method first prepares the protrusions, then prepares the flexible substrate on the protrusions. After the protrusions are cured and contact the polyurethane solution, the surface of the protrusions in contact with the polyurethane solution is partially dissolved. The dissolved part cures together with the adjacent polyurethane solution, forming the bonding region.

After the protrusions are cured and contact the polyurethane solution, and the polyurethane solution is cured, the connection between the flexible substrate and the protrusions is achieved. Since the flexible substrate and the protrusions are made of the same material, their interfacial bonding strength is better.

An embodiment of the present disclosure provides a method for producing a self-adaptive perivalvular leakage preventing component, including the following steps performed sequentially:
Step 1: curing a polyurethane solution to obtain a flexible substrate;
Step 2: filling a mold with a polyurethane mixture containing solid particles, curing it, and washing to remove the solid particles, thereby obtaining the protrusions;
Step 3: bonding the flexible substrate and the protrusions using an adhesive, to obtain the self-adaptive perivalvular leakage preventing component.

The adhesive is a polyurethane solution or a good solvent for polyurethane. The concentration of the polyurethane solution used as the adhesive is 8%-14% (w/v).

The good solvent for polyurethane used as the adhesive is at least one of dimethyl sulfoxide (DMSO), tetrahydrofuran (THF), dimethylformamide (DMF), dimethylacetamide (DMAc), dichloromethane, chloroform, and hexafluoroisopropanol (HFIP).

The solvent of a polyurethane solution used as the adhesive evaporates to bond the flexible substrate and the protrusions together.

When a good solvent for polyurethane is used as the adhesive and the solvent evaporates, bonding the flexible substrate and the protrusions together. The good solvent partially dissolves the contacting areas of the already cured flexible substrate and protrusions, reforming a polyurethane solution. After the solvent in the polyurethane solution evaporates and the polyurethane solution cures, the flexible substrate and the protrusions are connected together.

This method involves separately preparing the flexible substrate and the protrusions first, then bonding them using a polyurethane solution or a good solvent for polyurethane as the adhesive to obtain the self-adaptive perivalvular leakage preventing component.

The polyurethane solution or good solvent used as the adhesive dissolves the adjacent parts of the flexible substrate and the protrusions, i.e., the contacting areas of the flexible substrate and the protrusions are dissolved by the adhesive, and then cured to form the bonding region.

An embodiment of the present disclosure provides a method for producing a self-adaptive perivalvular leakage preventing component, including the following steps performed sequentially:
Step 1: filling a mold with a polyurethane mixture containing solid particles;
Step 2: bringing the polyurethane mixture containing solid particles into contact with a polyurethane solution, then curing and washing to remove the solid particles, thereby obtaining the self-adaptive perivalvular leakage preventing component.

This method involves co-curing the polyurethane mixture and the polyurethane solution to prepare the self-adaptive perivalvular leakage preventing component. Both the flexible substrate and the protrusions are in solution form and are cured together, forming a bonding region at their adjacent parts.

The flexible substrate and the protrusions are cured simultaneously, allowing them to be formed under the same process conditions, resulting in a simpler process flow and more convenient operation.

An embodiment of the present disclosure provides a method for producing a self-adaptive perivalvular leakage preventing component, including:
Step 1: filling a mold with a polyurethane mixture containing solid particles for forming the protrusions, and bringing this mixture into contact with a polyurethane solution for forming the flexible substrate;
Step 2: curing and washing to remove the solid particles, thereby obtaining the self-adaptive perivalvular leakage preventing component.

The polyurethane mixture containing solid particles for forming the protrusions and a polyurethane mixture containing solid particles for forming the flexible substrate are co-cured to prepare the self-adaptive perivalvular leakage preventing component. In this case, both the flexible substrate and the protrusions in the perivalvular leakage preventing component have a porous structure.

The polyurethane molecular chain includes hard segments and soft segments. The soft segment content is 40-70%, and the remainder is hard segments. The soft segment is at least one of polyether diol, polycarbonate diol, polyester diol, and polysiloxane diol. The hard segment is an isocyanate. The R-value of the isocyanate is 1.0-1.1. The soft segment content in the polyurethane molecular chain is 50%-65%.

The isocyanate is at least one of TDI, HDI, MDI, NDI, PPDI, IPDI, and XDI.

The polyurethane molecular chain further includes a chain extender. The chain extender is at least one of ethylene glycol, butanediol, hexanediol, octanediol, and ethylenediamine.

The solvent for the polyurethane solution is at least one of dimethyl sulfoxide (DMSO), tetrahydrofuran (THF), dimethylformamide (DMF), dimethylacetamide (DMAc), dichloromethane, chloroform, and hexafluoroisopropanol (HFIP).

The solvent is selected to be easily volatile, water-miscible, and capable of dissolving polyurethane. Easy volatility facilitates subsequent solvent evaporation and polyurethane curing under heating conditions. Water miscibility aids in the later stage where the solvent dissolves in water, allowing the polyurethane to form.

The solvent for the polyurethane solution is a mixture of two selected from dimethyl sulfoxide (DMSO), tetrahydrofuran (THF), dimethylformamide (DMF), dimethylacetamide (DMAc), dichloromethane, chloroform, and hexafluoroisopropanol (HFIP). The volume ratio of the two solvents may be 1-99: 1. The volume ratio of the two solvents may be 5-95:1. The volume ratio of the two solvents may be 10-95:1. The volume ratio of the two solvents may be 15-95:1. The volume ratio of the two solvents may be 20-95:1. The volume ratio of the two solvents may be 25-95:1. The volume ratio of the two solvents may be 10-90:1. The volume ratio of the two solvents may be 10-80:1. The volume ratio of the two solvents may be 15-75:1. The volume ratio of the two solvents may be 20-75:1. The volume ratio of the two solvents may be 25-75:1.

The concentration of polyurethane in the polyurethane solution is 0.25%-25% (w/v). Preferably, the polyurethane concentration is 0.5%-20% (w/v). Preferably, the polyurethane concentration is 0.5%-15% (w/v). Preferably, the polyurethane concentration is 0.5%-10% (w/v). Preferably, the polyurethane concentration is 5%-25% (w/v). Preferably, the polyurethane concentration is 1%-25% (w/v). Preferably, the polyurethane concentration is 2%-25% (w/v). Preferably, the polyurethane concentration is 3%-25% (w/v). Preferably, the polyurethane concentration is 4% - 25% (w/v). Preferably, the polyurethane concentration is 5%-20% (w/v). Preferably, the polyurethane concentration is 5%-15% (w/v). Preferably, the polyurethane concentration is 8%-14% (w/v).

The solid particles are at least one of sodium chloride, potassium chloride, and sucrose.

In this disclosure, the solid particles are insoluble in the polyurethane solution. After the polyurethane solution cures, the solid particles remain in solid state. Subsequently, a cleaning agent is used to wash away the solid particles, and the spaces occupied by the solid particles form the porous structure of the protrusions.

The cleaning agent used to wash the solid particles can dissolve the solid particles; the cleaning agent is water.

To ensure uniformity of the pore structure, mixing equipment such as a multi-tube vortex mixer is used to uniformly disperse the solid particles in the polyurethane solution. The number-average particle diameter of the solid particles is 5-500 micrometers. Preferably, the number-average particle diameter is 5-450 micrometers. Preferably, the number-average particle diameter is 5-400 micrometers. Preferably, the number-average particle diameter is 5-350 micrometers. Preferably, the number-average particle diameter is 5-300 micrometers. Preferably, the number-average particle diameter is 5-250 micrometers.

The particle size of the solid particles determines the pore size within the protrusions. The pore size in the protrusions may be adjusted by varying the particle size of the solid particles.

The polyurethane mixture containing solid particles, the mass fraction of the solid particles determines the porosity of the protrusions. The porosity of the protrusions affects their strength. A higher mass fraction of solid particles results in higher porosity but lower strength of the protrusions. Therefore, the porosity of the protrusions, and consequently their strength, can be adjusted by varying the mass fraction of solid particles in the polyurethane solution. Lower porosity results in higher protrusion strength. However, excessively low porosity may lead to an still overly large volume after compression of the protrusions, which is not conducive to loading within the delivery system.

In the polyurethane mixture containing solid particles, the mass fraction of the solid particles is 50%-80%. Preferably, the mass fraction of the solid particles is 55%-80%. Preferably, the mass fraction of the solid particles is 60%-80%. Preferably, the mass fraction of the solid particles is 60%-75%.

The material for the mold used to prepare the protrusions may be silicone, Teflon, aluminum, stainless steel, glass, etc. Any mold material that does not interfere with polyurethane formation and has thermal conductivity is acceptable. The specific structure of the mold is designed according to the requirements for the protrusions.

Referring to FIG. 99, the mold 93 includes a plurality of mold cavities 94. Each mold cavity 94 corresponds to one protrusion, and the distribution of the mold cavities 94 corresponds to the distribution of the protrusions.

The shape of the mold cavities 94 is not limited. The mold has millimeter-level precision. The shape of the mold cavities 94 is at least one of a triangular pyramid, quadrangular pyramid, ellipsoid, partial ellipsoid, sphere, partial sphere, parallelogram, rhombus, or partial rhombus. Preferably, the shape of the mold cavity matches the shape of the protrusions.

A curing temperature for the protrusions and the flexible substrate is 20-90°C. Preferably, the curing temperature for the protrusions and the flexible substrate is 20-70°C. Preferably, the curing temperature for the protrusions and the flexible substrate is 20-50°C.

A curing time for the protrusions and the flexible substrate is not less than 4 hours. Preferably, the curing time for the protrusions and the flexible substrate is not less than 6 hours. Preferably, the curing time for the protrusions and the flexible substrate is not less than 12 hours.

The curing temperature for the protrusions and the flexible substrate is 20-90°C, and a curing time for the protrusions and the flexible substrate is at least 6 hours. Preferably, the curing temperature for the protrusions and the flexible substrate is 20-70°C, and the curing time for the protrusions and the flexible substrate is at least 6 hours. More preferably, the curing temperature for the protrusions and the flexible substrate is 20-50°C, and the curing time for the protrusions and the flexible substrate is at least 6 hours.

The curing temperature for the protrusions and the flexible substrate is 20-90°C, and the curing time for the protrusions and the flexible substrate is at least 12 hours. Preferably, the curing temperature for the protrusions and the flexible substrate is 20-70°C, the curing time for the protrusions and the flexible substrate is at least 12 hours. More preferably, the curing temperature for the protrusions and the flexible substrate is 20-50°C, and the curing time for the protrusions and the flexible substrate is at least 12 hours.

The curing temperature for the protrusions and the flexible substrate is 50-70°C, and the curing time for the protrusions and the flexible substrate is 4-48 hours. Preferably, the curing temperature for the protrusions and the flexible substrate is 50-70°C, and the curing time for the protrusions and the flexible substrate is 4-24 hours.

The preparation of the protrusions and the flexible substrate further includes a post-treatment step after curing. The post-treatment includes: placing the protrusions and the flexible substrate in water at 20-40°C for at least 2 hours.

During the post-treatment step, the solvent of the polyurethane solution dissolves in the water, while the polyurethane is insoluble, allowing the polyurethane to finalize its formation.

Specifically, the post-treatment may include: placing the protrusions and the flexible substrate in water at 20-40°C for 2-4 hours.

The preparation of the protrusions and the flexible substrate further include a pre-curing step performed before the main curing step. Pre-curing conditions is: pre-curing temperature of 20-70°C, pre-curing time of 20-60 minutes.

Preferably, pre-curing conditions is: pre-curing temperature of 30-60°C, pre-curing time of 20-40 minutes.

When preparing the protrusions and the flexible substrate, performing a pre-curing treatment on the polyurethane mixture containing solid particles can prevent the solid particles from floating up within the polyurethane solution, which would lead to uneven distribution of solid particles in the polyurethane solution.

The pre-curing time is relatively short. After pre-curing, the polyurethane mixture is not fully formed; the primary purpose is to prevent the solid particles from floating.

Washing the protrusions and the flexible substrate includes: immersing the protrusions in a cleaning agent and letting them stand for at least 4 hours.

Alternatively, washing the protrusions and the flexible substrate includes: immersing the protrusions in the cleaning agent and letting them stand for 4-12 hours.

Alternatively, washing the protrusions and the flexible substrate includes: treating the protrusions in an agitated cleaning agent for at least 4 hours.

Alternatively, Washing the protrusions and the flexible substrate includes: treating the protrusions in an agitated cleaning agent for 4-12 hours.

The polyurethane solution that contacts the protrusions may be at room temperature or preheated. Room temperature means no heating or cooling is applied. Preheating treatment means that increases the temperature of the polyurethane solution. Preheating treatment can improve its fluidity and facilitate handling. If room temperature already provides sufficient fluidity, preheating is unnecessary.

The preheating temperature for the polyurethane solution is 30-70°C. Preferably, the preheating temperature for the polyurethane solution is 40-70°C. Preferably, the preheating temperature for the polyurethane solution is 50-70°C.

The preparation of the protrusions and the flexible substrate further include an initial curing step before the pre-curing step. Initial curing may be performed using one of the following methods:
a) blowing hot air over the surface of the polyurethane mixture containing solid particles;
b) spraying hot steam onto the surface of the polyurethane mixture containing solid particles;
c) adding water, ethanol, or isopropanol dropwise into the polyurethane mixture containing solid particles.

The main purpose of initial curing is to solidify the surface of the polyurethane mixture. The surface of the polyurethane mixture can solidify due to solvent evaporation caused by heating (e.g., hot air or steam), or due to the solvent dissolves in the added water/ethanol/isopropanol while the polyurethane remains insoluble in water/ethanol/isopropanol. Solidifying the surface of the polyurethane mixture prevents the solid particles from floating and potentially breaching the interface between the protrusions and the flexible substrate.
For step a): Hot air temperature is 40-100°C, blowing time is 0.5-2 min.
For step b): Steam temperature is 40-100°C, spraying time is 0.5-2 min.
For step c): Add 1-2.5 mL.

An embodiment of the present disclosure further provides a valve (e.g., a prosthetic heart valve, specifically such as an artificial aortic valve). The valve includes:
a stent, defining a blood flow passage in the interior. The stent has an inflow side and an opposite outflow side, and the inflow side has a grid structure;
one or more leaflets;
a perivalvular leakage preventing component connected at the inflow side (positioned on the inflow side relative to the leaflets). The flexible substrate of the perivalvular leakage preventing component is located inside the stent, and the protrusions are embedded within the grid structures; or, the flexible substrate of the perivalvular leakage preventing component is located outside the stent, and the protrusions are located on the side of the flexible substrate facing away from the stent.

Both the flexible substrate and the protrusions are made of polyurethane material, resulting in strong interfacial bonding strength therebetween and a high elongation at break. When the stent deforms, both the flexible substrate and the protrusions can adapt well to the deformation of the stent, contracting as the stent contracts and expanding as the stent expands. Even under maximum stent deformation, the flexible substrate and protrusions can maintain good mechanical properties.

The grid structure includes a number of grids. The plurality of protrusions correspond one-to-one with the positions of the grids. When the flexible substrate is located inside the stent, each protrusion extends from the inside to the outside of the stent through the corresponding grid.

When the flexible substrate is inside the stent, the protrusions extend from the inside to the outside through the corresponding grids, filling the perivalvular gaps. This achieves an excellent perivalvular leakage preventing effect without needing to increase the thickness of the flexible substrate, thereby avoiding an increase in the delivery system profile. Even if the valve is retrieved multiple times, damage is less likely to occur.

When the perivalvular leakage preventing component is in a flattened state, the cross-section of each protrusion parallel to the flexible substrate corresponds to the size of the respective grid.

The perivalvular leakage preventing component may be used alone or spliced together with other biological materials for use.

The following examples further illustrate the procedural details of the producing methods.

### Example 7

A method for producing a self-adaptive perivalvular leakage preventing component, referring to FIG. 96, includes the following steps:
(1) dissolving PU pellets (i.e., polyurethane) in dimethylformamide (DMF) to obtain a polyurethane solution with a polyurethane content of 15% (w/v);
(2) mixing sodium chloride particles with the polyurethane solution, and uniformly dispersing the sodium chloride solid particles with diameter of approximately 100-200 micrometers in the polyurethane solution using a multi-tube vortex mixer to obtain a pre-mix for forming the protrusions (i.e., the polyurethane mixture containing solid particles). The mass fraction of sodium chloride in the mixture is 75%;
(3) filling a soft silicone mold with the pre-mix and compacting the pre-mix;
(4) spreading the preheated polyurethane solution over the mold surface, defining the boundary of the polyurethane solution (this polyurethane solution is for forming the flexible substrate);
(5) placing the mold with the spread polyurethane solution into an oven for heat setting at 60°C for 4 hours, then removing the mold and cooling the mold to room temperature;
(6) placing the cooled mold into water (warm water, temperature of the warm water is between 25-40°C; 'warm water' mentioned elsewhere may also use this temperature range) for 2 hours;
(7) continue placing the demolded sample in water (room temperature or warm water) to dissolve the sodium chloride solid particles (using magnetic stirring to accelerate dissolution of the sodium chloride solid particles) for 4 hours;
(8) obtaining the self-adaptive perivalvular leakage preventing component with firmly bonded protrusions and polyurethane flexible substrate.

### Example 8

(1) dissolving PU pellets (i.e., polyurethane) in dimethylformamide (DMF) to obtain a polyurethane solution with a polyurethane content of 15% (w/v);
(2) mixing sucrose particles with the polyurethane solution, and uniformly dispersing the sucrose solid particles with diameter approx. 300-500 micrometers using a multi-tube vortex mixer to obtain the pre-mix for protrusions. The mass fraction of sucrose in the mixture is 75%;
(3) filling a soft silicone mold with the pre-mix and compacting the pre-mix;
(4) placing the silicone mold in water (room temperature) to cure for 1 hour, then drying in an oven at 60°C for 0.5 hours;
(5) taking out the mold, spreading the preheated polyurethane solution (the polyurethane solution is configured to form the flexible substrate) over the mold surface, defining a boundary of the polyurethane solution;
(6) placing the mold with the polyurethane solution into an oven for heat setting at 50°C for 3 hours, and taking out the mold and cooling the mold to room temperature;
(7) placing the cooled mold into warm water for 2 hours;
(8) placing the demolded sample in water (room temperature or warm water) to dissolve the sucrose solid particles (using magnetic stirring to accelerate dissolution of the sucrose solid particles) for 2 hours;
(9) obtaining the self-adaptive perivalvular leakage preventing component with firmly bonded protrusions and polyurethane flexible substrate.

### Example 9

(1) dissolving PU pellets in dimethylacetamide (DMAc) to obtain a polyurethane solution with a polyurethane content of 20% (w/v);
(2) mixing sodium chloride particles with the polyurethane solution, and uniformly dispersing the NaCl particles (diameter approx. 100-200 micrometers) using a multi-tube vortex mixer to obtain the pre-mix for protrusions. The mass fraction of NaCl in the mixture is 60%;
(3) spreading a layer of polyurethane solution and drying it at 60°C for 12 hours to obtain the flexible substrate;
(4) filling a soft silicone mold with the pre-mix and compacting the pre-mix;
(5) placing the mold in water (room temperature), then drying and curing at 60°C for 1 hour;
(6) taking out the mold, applying a small amount of preheated polyurethane solution (with a concentration of 8% (w/v)) as an adhesive onto the surface of the formed protrusions in the mold, and placing the pre-prepared flexible substrate on top, ensuring firm and effective adhesion;
(7) placing the mold into an oven for heat setting at 50°C for 6 hours, and removing the mold and cooling the mold to room temperature;
(9) placing the demolded sample in a magnetic stirrer with water (room temperature) to dissolve the NaCl particles for 6 hours.
(10) obtaining the self-adaptive perivalvular leakage preventing component with firmly bonded protrusions and polyurethane flexible substrate.

### Example 10

(1) dissolving PU pellets in dimethylformamide (DMF) to obtain polyurethane solutions with a polyurethane content of 10% (w/v) and polyurethane solution with a polyurethane content of 20% (w/v).
(2) mixing potassium chloride (KCL) particles with the 20% (w/v) polyurethane solution, and uniformly dispersing the potassium chlorideparticles (diameter approx. 50-150 micrometers) using a multi-tube vortex mixer to obtain a pre-mix for preparing the protrusions. The mass fraction of potassium chloride in the mixture solution is 75%.
(3) filling a mold with the pre-mix and compacting the pre-mix.
(4) spreading the preheated 10% (w/v) polyurethane solution (for forming the flexible substrate) over the mold surface, defining a boundary of the polyurethane solution.
(5) placing the mold into an oven for heat setting at 50°C for 12 hours, and then taking out the mold and cooling the mold to room temperature.
(6) placing the cooled mold into a magnetic stirrer with water (room temperature) to accelerate dissolution of the potassium chloride particles (accelerates dissolution of the potassium chloride particles by magnetic stirring) for 12 hours, then demolding.
(7) obtaining the self-adaptive perivalvular leakage preventing component with firmly bonded protrusions and polyurethane flexible substrate.

### Example 11

(1) dissolving PU pellets in dimethylformamide (DMF) to obtain a polyurethane solution with a polyurethane content of 20% (w/v).
(2) mixing sodium chloride (NaCl) particles with the polyurethane solution, and uniformly dispersing the sodium chloride particles (diameter approx. 300-400 micrometers) in the polyurethane solution using a multi-tube vortex mixer to obtain a pre-mix to prepare the protrusions. The mass fraction of sodium chloride in the polyurethane solution is 65%.
(3) filling a silicone mold with the pre-mix and compacting the pre-mix.
(4) treating the surface of the silicone mold with hot air blowing for 3 minutes.
(5) taking out the mold, spreading the preheated polyurethane solution (for the flexible substrate) over the mold surface, defining a boundary of the polyurethane solution.
(6) placing the mold into an oven for heat setting at 40°C for 2.5 hours, and taking out the mold and cooling the mold to room temperature.
(7) placing the cooled mold into water (warm water) for 2 hours.
(8) placing the demolded sample in a magnetic stirrer with water (room temperature) to accelerate dissolution of sodium chloride particles for 12 hours.
(9) obtaining the self-adaptive perivalvular leakage preventing component with firmly bonded protrusions and polyurethane flexible substrate.

### Example 12

(1) dissolving PU pellets in dimethyl sulfoxide (DMSO) to obtain a polyurethane solution with a polyurethane content of 10% (w/v).
(2) mixing sodium chloride (NaCl) particles with the polyurethane solution, and uniformly dispersing the sodium chloride particles (diameter approx. 100-200 micrometers) into the polyurethane solution to obtain the pre-mix for prepare the protrusions. The mass fraction of sodium chloride in the mixture solution is 75%.
(3) filling a silicone mold (with grooves, i.e., mold cavities) with the pre-mix and compacting pre-mix.
(4) treating the surface of the silicone mold by spraying hot steam for 3 minutes.
(5) taking out the mold, spreading the preheated polyurethane solution (for the flexible substrate) over the surface, defining a boundary of the polyurethane solution.
(6) placing the mold into an oven for heat setting at 60°C for 12 hours, and taking out the mold and cooling the mold to room temperature.
(7) placing the cooled mold into water (room temperature) for 6 hours.
(8) placing the demolded sample in a magnetic stirrer with water (room temperature) to accelerate dissolution of sodium chloride particles for 8 hours.
(9) obtaining the self-adaptive perivalvular leakage preventing component with firmly bonded protrusions and polyurethane flexible substrate.

### Example 13

(1) dissolving PU pellets in dimethylformamide (DMF) to obtain a polyurethane solution with a polyurethane content of 12% (w/v).
(2) mixing sucrose particles with the polyurethane solution, and uniformly dispersing the sucrose particles (diameter approx. 100-200 micrometers) into the polyurethane solution to obtain a pre-mix for preparing protrusions. The mass fraction of sucrose in the pro-mix solution is 50%.
(3) filling a silicone mold (with grooves) with the pre-mix and compacting the pre-mix.
(4) adding 2.0 mL of ethanol dropwise onto the surface of the silicone mold.
(5) taking out the mold, spreading the preheated polyurethane solution (for forming the flexible substrate) over the surface, defining a boundary of the polyurethane solution.
(6) placing the mold into an oven for heat setting at 30°C for 12 hours, and taking out the mold and cooling the mold to room temperature.
(7) placing the cooled mold into water (warm water) for 2 hours.
(8) placing the demolded sample in water (room temperature or warm water) to dissolve the sucrose particles (accelerating dissolution of the sucrose particles by magnetic stirring) for 4 hours.
(9) obtaining the self-adaptive perivalvular leakage preventing component with firmly bonded protrusions and polyurethane flexible substrate.

### Example 14

(1) dissolving PU pellets in dimethylformamide (DMF) to obtain a polyurethane solution with a polyurethane content of 15% (w/v).
(2) mixing sodium chloride (NaCl) particles with the polyurethane solution, and uniformly dispersing the sodium chloride particles (diameter approx. 400-500 micrometers) into the polyurethane solution to obtaining a pre-mix for preparing the protrusions. The mass fraction of sodium chloride in the pre-mix solution is 75%.
(3) drying the polyurethane solution at 60°C for 12 hours to obtain the flexible substrate.
(4) filling a soft silicone mold (with grooves with predetermined shape) with the pre-mix and compacting the pre-mix.
(5) taking out the mold, applying a low-concentration (8% (w/v)) polyurethane solution (a small amount as adhesive) onto the surface of the pre-mix filled in the mold, and attaching the pre-prepared flexible substrate, ensuring the two full contact and adhesion.
(6) placing the mold into an oven for heat setting at 55°C for 3.5 hours, and removing the mold from the oven and cooling the mold to room temperature.
(7) placing the cooled mold into water (room temperature or warm water) for 2 hours.
(8) placing the demolded sample in water (room temperature or warm water) to dissolve the solid particles (dissolution of solid particles accelerated by magnetic stirring) for 4 hours.
(9) obtaining the self-adaptive perivalvular leakage preventing component with firmly bonded protrusions and polyurethane flexible substrate.

### Comparative Example 1

(1) dissolving PU pellets in dimethylformamide (DMF) to obtain a polyurethane solution with a polyurethane content of 15% (w/v).
(2) mixing sodium chloride (NaCl) particles with the polyurethane solution, and uniformly dispersing the sodium chloride particles (diameter approx. 100-200 micrometers) into the polyurethane solution to obtain the pre-mix for protrusions. The mass fraction of sodium chloride in the pre-mix solution is 75%.
(3) filling a mold with grooves with the pre-mix and compacting the pre-mix. Before covering the mold with a PET fabric, applying the preheated polyurethane solution as an adhesive onto both the PET fabric and the added pre-mix surface to ensure full contact and effective bonding between the two.
(4) then drying at 60°C for 30 minutes.
(5) cooling at room temperature for more than 20 min, then placing the mold in water for more than 36 hours before demolding.
(6) placing the demolded skirt in water (room temperature or warm water) to dissolve the sodium chloride particles (dissolution of sodium chloride solid particles accelerated by magnetic stirring) for 4 hours.
(7) obtaining the perivalvular leakage preventing component.

### Performance Characterization

Referring to FIG. 92, Comparative Example 1 uses PET fabric as the substrate and polyurethane solution as the adhesive to bond the PET fabric and the protrusions. Since PET and polyurethane are not the same material, their compatibility is poor. A distinct physical interface exists, prone to phase separation. The interfacial bonding strength is weak, making the protrusions susceptible to detachment from the substrate. This poses a significant risk of detachment during valve loading and retrieval, representing a safety concern.

Referring to FIG. 93, both the flexible substrate and the protrusions in Example 7 are made of polyurethane material. The interfacial bonding strength between the same material is better, eliminating the risk of protrusion detachment and ensuring better safety.

Referring to FIG. 94, In the perivalvular leakage preventing component prepared **in** Comparative Example 1, which uses a PET fabric as the substrate, the PET fabric and the protrusions are bonded via a polyurethane (PU) solution as the adhesive. In FIG. 94, the upper part of the drawing shows the overall perivalvular leakage preventing component, and the lower part of the drawing is a partial enlarged view of an area without protrusions. As shown in FIG. 94, the surface of the perivalvular leakage preventing component is non-uniform, and the PU membrane is prone to lifting and peeling.

Referring to FIG. 95, the perivalvular leakage preventing component prepared **in** Example 7 is shown. In FIG. 95, the upper part of the drawing shows the overall perivalvular leakage preventing component, and the lower part of the drawing is a partial enlarged view of an area without protrusions. As shown in FIG. 95, the surface is uniform, lacking weak points in mechanical performance, indicating better mechanical properties.

The elongation at break of the perivalvular leakage preventing components prepared in Example 7 and Comparative Example 1 was tested. Results are shown in Table 1.

**Table 1**

| Sample ID | Comparative Example 1 | Example 7 |
|---|---|---|
| Sample 1 | 36.10% | 311.04% |
| Sample 2 | 36.57% | 314.64% |
| Sample 3 | 37.33% | 357.04% |
| Average | 36.67% | 327.57% |

As shown in Table 1, the perivalvular leakage preventing component from Comparative Example 1 has a small elongation at break. The material feels relatively stiff subjectively. When the stent deforms, this perivalvular leakage preventing component cannot adapt well to the deformation and hinders the deformation of the stent.

As shown in Table 1, the perivalvular leakage preventing component from Example 7 has a large elongation at break. When the stent deforms, both the flexible substrate and the protrusions can adapt well, contracting and expanding with the stent. Even under maximum stent deformation, the flexible substrate and the protrusions maintain good mechanical properties, especially elasticity, self-adapting to any potential configuration of the stent.

Referring to FIG. 42a to FIG. 42c, an interlayer 300, made of the same material as the protrusions, is disposed between a PET material layer 100 and a PU material layer 200. The interlayer 300 applies a force F1 to the PET material layer 100 and a force F2 of to the PU material layer 200, which are equal in magnitude but opposite in direction. The PET material layer 100 separates from the interlayer 300, while the PU material layer 200 remains fixed to the interlayer 300.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same figure, it can be considered that the figure also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. A prosthetic implant, **characterized in that**, comprising:
a stent, being a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a perivalvular leakage preventing structure, arranged circumferentially around the stent, and the perivalvular leakage preventing structure being:
a sealing assembly, at least a portion of the sealing assembly protruding radially outward from the stent, and the sealing assembly comprising a deformable structure; and/or
an outer skirt wrapped around an outer periphery of the stent.

2. The prosthetic implant of claim 1, wherein the prosthetic implant further comprises an inner skirt located radially inside the stent; wherein the sealing assembly and at least one of the inner skirt and the outer skirt are formed as a one-piece structure, or the sealing assembly is wrapped within a radial gap between the inner skirt and the outer skirt.

3. The prosthetic implant of claim 1, wherein the sealing assembly is fixed to the inner skirt through a hollowed region of a stent grid and wraps at least a portion of a strut constituting the stent grid.

4. The prosthetic implant of claim 1, wherein the perivalvular leakage preventing structure is an outer skirt located radially outside the stent; the prosthetic implant further comprises an inner skirt located radially inside the stent; and the inner skirt and the outer skirt are a one-piece structure and are made of polyurethane material.

5. The prosthetic implant of claim 1, wherein the perivalvular leakage preventing structure comprises the sealing assembly and the outer skirt;
the prosthetic implant further comprises an inner skirt located radially inside the stent, the inner skirt and the outer skirt are connected at an inflow side of the stent to form a pouch structure, and at least a portion of the stent and at least a portion of the sealing assembly are located within the pouch structure.

6. A prosthetic implant, **characterized in that**, comprising:
a stent, being a radially deformable tubular structure, the stent having a through blood flow channel;
a plurality of leaflets, each leaflet having a fixed edge and an opposite free edge, wherein the fixed edge is fixed to the stent, and the free edge is configured to control the blood flow channel;
a skirt, comprising an inner skirt located radially inside the stent and/or an outer skirt located radially outside the stent;
a sealing assembly, being a plurality of sealing blocks, made of a deformable structure, and arranged circumferentially around the stent, at least a portion of the sealing assembly radially protruding outward from the stent; and the sealing assembly and at least one of the inner skirt and the outer skirt being a one-piece structure, or the sealing assembly wrapped within a radial gap between the inner skirt and the outer skirt.

7. The prosthetic implant of claim 6, wherein the sealing assembly comprises a plurality of sealing blocks, the plurality of sealing blocks are arranged in multiple groups spaced apart circumferentially around the stent, each group has at least one sealing block, spacing regions exist between adjacent groups, with all sealing blocks located away from the spacing regions along the stent circumference.

8. The prosthetic implant of claim 6, wherein the sealing assembly comprises a plurality of sealing blocks embedded in stent grids; sealing blocks in two circumferentially adjacent stent grids are arranged offset from each other along an axial direction of the stent; and along the axial direction of the stent, opposite sides of a sealing block are a wider side and a narrower side respectively, and the sealing blocks in two adjacent stent grids are positioned with the wider sides facing each other.

9. The prosthetic implant of claim 6, wherein the sealing assembly forms at least two circles of peaks around the outer periphery of the stent, the two circles of peaks are arranged along an axial direction of the stent and define a trough region therebetween for accommodating a native valve annulus, and the peaks in the same circle are a plurality of sealing blocks spaced circumferentially or a sealing ring continuously distributed circumferentially.

10. The prosthetic implant of claim 6, wherein the stent has an inflow side and an opposite outflow side, the outer skirt is arranged on the inflow side; the stent has stent grids, and the axial span of the outer skirt is 0.5 to 2 grids.

11. The prosthetic implant of claim 6, wherein the outer skirt has an inflow edge and an outflow edge; along an axial direction of the stent, the outflow edge of the outer skirt is adjacent to a midpoint position of the fixed edge of the leaflet and does not extend beyond the midpoint position towards the outflow side.

12. The prosthetic implant of claim 6, wherein the stent has a waist portion, and an outflow edge of the outer skirt is located at the waist portion.

13. The prosthetic implant of claim 6, wherein the stent has a waist portion, and a distance between an outflow edge of the outer skirt and the waist portion is 0.5 to 1 grid.

14. The prosthetic implant of claim 6, wherein a pouch structure is defined between the outer skirt and the inner skirt, and the sealing assembly is located within the pouch structure.

15. The prosthetic implant of claim 6, wherein a pouch structure extends continuously along the stent circumference or is arranged at intervals along the stent circumference.

16. The prosthetic implant of claim 14, wherein along a radial direction of the stent, the pouch structure protrudes outward beyond an outer peripheral wall of the stent by 0.5 to 20 mm.

17. The prosthetic implant of claim 14, wherein the pouch structure is a fully enclosed structure; or the pouch structure is a semi-open structure with openings facing an inflow side and/or an outflow side.

18. The prosthetic implant of claim 17, wherein a plurality of the openings are provided at intervals around the stent circumference.

19. The prosthetic implant of claim 6, wherein the inner skirt and the outer skirt are:
both made of polyurethane; or
one made of polyurethane and the other made of a biological material; or
both biological materials; or
the inner skirt made of polyurethane, and the outer skirt made of PET fabric.

20. The prosthetic implant of claim 6, wherein the inner skirt and the outer skirt each have an inflow edge and an outflow edge; the outflow edge of the inner skirt is connected to the fixed edges of the plurality of leaflets; the inner skirt is formed from a first sheet, the outer skirt is formed from a second sheet; and configurations of the inner skirt and the outer skirt is:
the first sheet is fixed to an inner side of the stent, the second sheet is fixed to an outer side of the stent, and the inflow end of the stent is exposed; or
the first sheet is fixed to an inner side of the stent, the first sheet is everted outward at the inflow side of the stent and wraps around the inflow end of the stent, the second sheet material is fixed to an outer side of the stent and is connected to the everted portion of the first sheet; or
the second sheet is fixed to an outer side of the stent, the second sheet is inverted inward at the inflow side of the stent and wraps around the inflow end of the stent, the first sheet is fixed to an inner side of the stent and is connected to the inverted portion of the second sheet; or
the first sheet is fixed to an inner side of the stent, the second sheet is fixed to an outer side of the stent, and the first sheet and the second sheet are a one-piece structure and wrap the inflow end of the stent;
wherein the sealing assembly and one of the first sheet and the second sheet are formed as a one-piece structure; or the sealing assembly is bonded to at least one of the first sheet and the second sheet; or the sealing assembly is sandwiched between the first sheet and the second sheet, and a radial position of the sealing assembly is disposed at the inner side or the outer side of the second sheet.

21. The prosthetic implant of claim 6, wherein the inflow edge of the inner skirt and the inflow edge of the outer skirt are substantially aligned, and are connected as a one-piece structure or as separate components to seal the blood flow channel; in a radial direction, the sealing assembly is disposed at an inner side or an outer side of the outer skirt.

22. The prosthetic implant of claim 6, wherein shapes of inflow edges of the inner skirt and the outer skirt are independent of each other, each of the inflow skirt and the outer skirt has a smooth inflow edge or an inflow edge conforming to a shape of the stent grids.

23. The prosthetic implant of claim 6, wherein an outflow edge of the outer skirt extends smoothly along the stent circumference; or an outflow edge of the outer skirt has axial undulations along the stent circumference.

24. The prosthetic implant of claim 23, wherein a degree of axial undulation matches a shape of struts of the stent or matches a shape of an axial edge of the sealing assembly.

25. The prosthetic implant of claim 20, wherein the first sheet and/or the second sheet are made of polyurethane with a thickness of 0.02-0.2 mm; or
the first sheet is made of polyurethane, and the second sheet is made of PET fabric.

26. The prosthetic implant of claim 6, wherein the inner skirt and the outer skirt are fixed to each other by at least one of bonding or suturing.

27. The prosthetic implant of claim 26, wherein the inner skirt and the outer skirt are fixed to each other continuously or at intervals along the stent circumference.

28. The prosthetic implant of claim 6, wherein the material of the sealing assembly is polyurethane or other polymer materials.

29. The prosthetic implant of claim 20, wherein the material of the sealing assembly is polyurethane and the same material as at least one of the first sheet and the second sheet, and the sealing assembly and at least one of the first sheet and the second sheet are formed as one-piece structure.

30. The prosthetic implant of claim 6, wherein the sealing assembly has internal voids.

31. The prosthetic implant of claim 6, wherein the sealing assembly has a foam structure with a pore size of 5-500 micrometers and a porosity of 40%-95%.

32. The prosthetic implant of claim 6, wherein an arrangement of the sealing assembly is one of the following:
a plurality of sealing blocks arranged circumferentially along the stent; or
the sealing assembly being an annular member located around an outer periphery of the stent, and the sealing assembly directly sleeved over the outer periphery of the stent; or
the sealing assembly directly sleeved over an outer periphery of the outer skirt.

33. The prosthetic implant of claim 32, wherein the plurality of sealing blocks are arranged in multiple groups spaced apart circumferentially around the stent, each group has at least one sealing block, spacing regions exist between adjacent groups, with all sealing blocks located away from the spacing regions along the stent circumference.

34. The prosthetic implant of claim 32, wherein the plurality of sealing blocks are arranged in multiple groups spaced apart circumferentially around the stent, each group comprises 1 to 10 sealing blocks, and distribution regions of the sealing blocks within the same group are continuously arranged along the stent circumference.

35. The prosthetic implant of claim 32, wherein distribution regions of all the plurality of sealing blocks are continuously arranged along the stent circumference.

36. The prosthetic implant of claim 32, wherein in the radial direction, the sealing blocks are disposed at an inner side of the outer skirt; and each sealing block is embedded in a corresponding stent grid or each sealing block is randomly filled between the two skirts.

37. The prosthetic implant of claim 32, wherein one grid contains only one continuously distributed sealing block; or one grid contains multiple spaced sealing blocks.

38. The prosthetic implant of claim 32, wherein the stent has multiple rows of grids, and all sealing blocks are distributed only within a same row.

39. The prosthetic implant of claim 32, wherein the sealing blocks in two adjacent stent grids are arranged offset from each other along an axial direction of the stent.

40. The prosthetic implant of claim 32, wherein along the axial direction of the stent, opposite sides of a sealing block are a wider side and a narrower side respectively, and the sealing blocks in two adjacent stent grids are positioned with the wider sides facing each other.

41. The prosthetic implant of claim 40, wherein the wider sides of all the sealing blocks are connected to each other and extend continuously the stent circumference.

42. The prosthetic implant of claim 32, wherein viewed from a radial perspective of the stent, a shape of the sealing blocks is circular, elliptical, rectangular, rhombic, triangular, or hexagonal.

43. The prosthetic implant of claim 32, wherein each sealing block is embedded in a corresponding stent grid, and viewed from a radial perspective of the stent; the sealing block fills the entire stent grid in which it is located, or occupy only a partial region of the stent grid in which it is located.

44. The prosthetic implant of claim 32, wherein the sealing block is located in a central region of the stent grid in which it is located, or adjacent to one axial end of the stent grid in which it is located.

45. The prosthetic implant of claim 32, wherein a single stent grid contains two sealing blocks spaced apart axially, with each sealing block located adjacent to one axial end of the stent grid.

46. The prosthetic implant of claim 32, wherein in a longitudinal cross-section of the sealing block, the sealing block has an inner side facing the stent and an opposite outer side, and a shape of an edge of the outer side is a broken line, a smooth curved line, or a combination of both.

47. The prosthetic implant of claim 32, wherein an edge of an outer side of the sealing block is a broken line, and the longitudinal cross-section of the sealing block is triangular, rectangular, or trapezoidal.

48. The prosthetic implant of claim 32, wherein an edge of an outer side of the sealing block is a smooth curve and has a radially highest point, and along the axial direction, the highest point within the stent grid is closer to the inflow side.

49. The prosthetic implant of claim 32, wherein along a radial direction of the stent, a thickness of the sealing block is uniform or non-uniform distributed, and a maximum thickness of the sealing block is 0.5-5 mm.

50. The prosthetic implant of claim 32, wherein the sealing assembly overall spans 0.5-3 grids along an axial direction.

51. The prosthetic implant of claim 32, wherein a shape of a longitudinal cross-section of the annular member is circular, truncated circular, trapezoidal, rectangular, elliptical, B-shaped, triangular, etc.

52. The prosthetic implant of claim 6, wherein an installation location of the sealing assembly is one of the following:
the sealing assembly is fixed to the inner skirt and protrudes from a radial inside of the stent towards a radial outside through corresponding grids, and the outer skirt surrounds an outside of the sealing assembly; or
the sealing assembly is fixed to an inner side of the outer skirt and is located within an interlayer between the outer skirt and the inner skirt; or
the sealing assembly is fixed to an outer side of the outer skirt.

53. The prosthetic implant of claim 6, wherein the outer skirt surrounds an outside of the sealing assembly, and the outer skirt wraps all or part of the sealing assembly.

54. The prosthetic implant of claim 6, wherein in a longitudinal section of the sealing assembly, a shape of an edge of an outer side of the sealing assembly has two peaks and a trough between the two peaks, and an outflow side of the outer skirt is located at the position of the trough.

55. The prosthetic implant of claim 6, wherein the prosthetic implant further comprises:
a reinforcement member, embedded in the skirt and connected to the stent via sutures passing through the reinforcement member.

56. The prosthetic implant of claim 55, wherein the reinforcement member avoids or passes through the sealing assembly.

57. The prosthetic implant of claim 55, wherein the outer skirt and the inner skirt are connected to the stent by sutures, at least one of the outer skirt and the inner skirt comprises the reinforcement member, and the sutures pass through the reinforcement member.

58. The prosthetic implant of claim 55, wherein the reinforcement member is a suture reinforcement wire or a reinforcement fabric pre-embedded within the skirt; or the reinforcement member is a reinforcement piece directly attached to the outer skirt and/or the inner skirt.

59. The prosthetic implant of claim 55, wherein the material of the reinforcement member is polyurethane or PET.

60. The prosthetic implant of claim 57, wherein the reinforcement piece is located on an outer side of the outer skirt or on an inner side of the inner skirt, or radially between the outer skirt and the inner skirt.

61. The prosthetic implant of claim 57, wherein the reinforcement piece and the skirt to which the reinforcement piece is attached form a one-piece structure or are fixed as separate parts.

62. The prosthetic implant of claim 57, wherein at a junction of the inner skirt and the leaflet, the reinforcement piece, the leaflet, and the inner skirt are stacked and fixed in any order.

63. The prosthetic implant of claim 55, wherein the reinforcement member is distributed over the entire inner skirt or only located at an outflow side of the inner skirt and connected to the fixed edge of the leaflet.

64. The prosthetic implant of claim 55, wherein the sutures avoid or pass through the sealing assembly.

65. The prosthetic implant of claim 58, wherein the suture reinforcement wire extends along the stent circumference to form a wire loop, the wire loop is a single circle or multiple rings, wherein multiple rings of the wire loops are arranged spaced apart or extend continuously in a spiral manner.

66. The prosthetic implant of claim 6, wherein the prosthetic implant further comprises:
a radiopaque marker, pre-embedded in the skirt and/or the sealing assembly, joining regions between adjacent leaflets at the stent are joint portions, the circumferential position of the radiopaque markers correspond to respective joint portions, at least one group of markers is provided, positioned adjacent to the inflow side of the stent in the axial direction, and an axial distance between the group of the radiopaque makers and the leaflets is sufficient to at least accommodate the native annulus.

67. The prosthetic implant of claim 66, wherein the prosthetic implant further comprises:
a reinforcement member, pre-embedded in the skirt and connected to the stent via sutures passing through the reinforcement member;
the radiopaque marker is pre-embedded in at least one of the outer skirt, the inner skirt, the sealing assembly, and the reinforcement member.

68. The prosthetic implant of claim 66, wherein the radiopaque marker is formed by at least one of the following methods:
the radiopaque marker is pre-formed and acts as a pre-embedded component in a combining process;
the radiopaque marker is mixed into at least one raw material used in a combining process.

69. The prosthetic implant of claim 66, wherein the radiopaque marker is strip-shaped, powdered, sheet-shaped, or block-shaped.

70. The prosthetic implant of claim 66, wherein the radiopaque marker is strip-shaped and extends along an axial direction or a circumferential direction of the stent.

71. The prosthetic implant of claim 66, wherein the sealing assembly is an annular member, and the radiopaque marker is arranged circumferentially along the annular member.

72. The prosthetic implant of claim 66, wherein the sealing assembly is a plurality of sealing blocks, and at least two sealing blocks are provided with the radiopaque marker.

73. The prosthetic implant of claim 72, wherein the radiopaque marker is located in a central region of the sealing block at which the radiopaque marker is located.

74. The prosthetic implant of claim 66, wherein in a longitudinal section of the sealing assembly, a shape of an edge of an outer side of the sealing assembly has two peaks and a trough between the two peaks, and the radiopaque marker comprises a first marker and a second marker, the two markers are located at the corresponding peaks, respectively.

75. The prosthetic implant of claim 66, wherein in a longitudinal section of the sealing assembly, a shape of an edge of an outer side of the sealing assembly has two peaks and a trough between the two peaks, and the radiopaque marker is located at the trough.

76. The prosthetic implant of claim 66, wherein joining regions between two circumferentially adjacent leaflets at the stent are joint portions, the radiopaque marker comprises multiple groups, and along a circumference of the stent, each group corresponds to a position of a respective joint portion.

77. The prosthetic implant of claim 66, wherein along an axial direction of the stent, the radiopaque marker is adjacent to an inflow side of the stent.

78. A perivalvular leakage preventing device for a prosthetic implant, **characterized in that**, the prosthetic implant comprises a stent with a grid structure and being tubular, and the perivalvular leakage preventing device comprises:
a plurality of sealing blocks arranged around a circumference of the stent, each sealing block made of a deformable structure and embedded in a corresponding grid structure;
a pulling wire, the sealing blocks are connected to each other via the pulling wire.

79. The perivalvular leakage preventing device of claim 78, wherein according to the correspondence with the grid structure, the sealing blocks are arranged in multiple rows along an axial direction of the stent, and adjacent rows are staggered relative to each other.

80. The perivalvular leakage preventing device of claim 78, wherein the sealing blocks in the same row are connected only by the pulling wire.

81. The perivalvular leakage preventing device of claim 78, wherein a connecting strip integrally formed with the sealing blocks is provided between adjacent sealing blocks in the same row.

82. The perivalvular leakage preventing device of claim 78, wherein the sealing block fully fills the grid structure in which the sealing block is located.

83. The perivalvular leakage preventing device of claim 78, wherein the sealing block partially fills the grid structure in which the sealing block is located.

84. The perivalvular leakage preventing device of claim 78, wherein the sealing blocks are arranged in two rows, wherein the sealing blocks in the first row fully fill the grid structures where they are located, and the sealing blocks in the second row partially fill the grid structures where they are located.

85. The perivalvular leakage preventing device of claim 84, wherein in the first row, a spaced portion is present between every two adjacent sealing blocks, and the sealing blocks in the second row are located in the corresponding spaced portions.

86. The perivalvular leakage preventing device of claim 78, wherein along an axial direction of the stent, the grid structure has a central region and edge regions disposed on two sides of the central region; along the radial direction of the stent, a thickness of the sealing block decreases from the central region towards the edge regions of the grid where it is located; along a stent circumferential direction, a width of the sealing block decreases from the central region towards the edge regions of the grid where it is located.

87. The perivalvular leakage preventing device of claim 78, wherein the sealing block has a porous structure.

88. The perivalvular leakage preventing device of claim 87, wherein the sealing block is made of foamed polyurethane.

89. The perivalvular leakage preventing device of claim 87, wherein the porosity of the sealing block is 50-85.

90. The perivalvular leakage preventing device of claim 78, wherein along a radial direction of the stent, the perivalvular leakage preventing device has an inner side and an outer side, and a position of the pulling wire is adjacent to the inner side.

91. The perivalvular leakage preventing device of claim 78, wherein the pulling wire is located on the inner side of the stent, and the sealing blocks protrude outward from an outer peripheral surface of the stent.

92. The perivalvular leakage preventing device of claim 78, wherein the perivalvular leakage preventing device has a coiled state wound around the stent and a relatively unfolded state; in the unfolded state, a plurality of pulling wires are arranged along their extension direction.

93. The perivalvular leakage preventing device of claim 92, wherein at least one pulling wire passes through all the sealing blocks.

94. The perivalvular leakage preventing device of claim 92, wherein the sealing blocks are arranged in multiple rows along an axial direction of the stent, and at least one pulling wire passes through all the sealing blocks in one of the rows.

95. The perivalvular leakage preventing device of claim 92, wherein in the unfolded state, two of the pulling wires are parallel to each other.

96. The perivalvular leakage preventing device of claim 92, wherein in the unfolded state, two of the pulling wires intersect.

97. The perivalvular leakage preventing device of claim 92, wherein in the coiled state, at least one pulling wire extends along a length direction of the perivalvular leakage preventing device.

98. The perivalvular leakage preventing device of claim 92, wherein in the unfolded state, at least one pulling wire extends along a length direction of the perivalvular leakage preventing device.

99. The perivalvular leakage preventing device of claim 92, wherein in the unfolded state, at least two pulling wires extend along a length direction of the perivalvular leakage preventing device.

100. The perivalvular leakage preventing device of claim 78, wherein the pulling wires are arranged in at least two groups, each group consists of multiple parallel wires, one of the groups extends along a length direction of the perivalvular leakage preventing device, and another group extends along a width direction of the perivalvular leakage preventing device.

101. The perivalvular leakage preventing device of claim 100, wherein the pulling wires of different extension directions belong to the same pulling wire and are meanderingly arranged.

102. The perivalvular leakage preventing device of claim 78, wherein according to the correspondence with the grid structure, the sealing blocks are arranged in one or more rows; at least one pulling wire passing through the sealing blocks in the same row extends along the length direction of the perivalvular leakage preventing device in the unfolded state.

103. The perivalvular leakage preventing device of claim 78, wherein in the unfolded state, along a width direction of the perivalvular leakage preventing device, at least one of the pulling wires, which pass through the sealing blocks in the same row, is adjacent to a central region of the sealing blocks.

104. The perivalvular leakage preventing device of claim 78, wherein a limiting component is fixed to the pulling wire and is embedded within the sealing block.

105. The perivalvular leakage preventing device of claim 78, wherein the limiting component is a knot.

106. The perivalvular leakage preventing device of claim 105, wherein the limiting component further comprises a meandering structure connected to the knot.

107. The perivalvular leakage preventing device of claim 78, wherein the pulling wires are all connected via the limiting component.

108. The perivalvular leakage preventing device of claim 78, wherein the pulling wire is made of PTFE material.

109. The perivalvular leakage preventing device of claim 78, wherein the perivalvular leakage preventing device further comprises an inner membrane; in the coiled state, the inner membrane is located on the inner side, and the sealing blocks are fixed on an outer side of the inner membrane.

110. The perivalvular leakage preventing device of claim 78, wherein in the same row, a spaced portion is defined between two adjacent sealing blocks; along its extending path, a portion of the pulling wire is located in the spaced portion and is fixed to the inner membrane.

111. The perivalvular leakage preventing device of claim 109, wherein the inner membrane and the sealing blocks are made of the same material.

112. The perivalvular leakage preventing device of claim 78, wherein a thickness of the inner membrane is 0.01-0.5 mm.

113. A perivalvular leakage preventing device for a prosthetic implant, wherein the prosthetic implant has a stent with a grid structure and is tubular, the perivalvular leakage preventing device comprising:
a plurality of sealing blocks arranged around the circumference of the stent, each sealing block made of a deformable structure and embedded in a corresponding cell structure;
an inner membrane, the plurality of sealing blocks fixed to one side of the inner membrane, and the inner membrane and the plurality of sealing blocks are made of the same material.

114. A prosthetic implant, **characterized in that**, comprising:
a stent and a perivalvular leakage preventing device wrapped around the stent, the stent being tubular and having a grid structure, wherein,
the perivalvular leakage preventing device comprises:
a plurality of sealing blocks, the plurality of sealing blocks arranged around the circumference of the prosthetic implant, each sealing block made of a deformable structure and embedded in a corresponding grid structure;
a pulling wire, the plurality of sealing blocks connected to each other via the pulling wire to constitute the perivalvular leakage preventing device;
or
the perivalvular leakage preventing device comprises:
a plurality of sealing blocks arranged around the circumference of the stent, each sealing block made of a deformable structure and embedded in a corresponding grid structure;
an inner membrane, the plurality of sealing blocks fixed to one side of the inner membrane.

115. The prosthetic implant of claim 114, wherein the inner membrane and the plurality of sealing blocks are made of the same material.

116. The prosthetic implant of claim 114, wherein the prosthetic implant is a prosthetic heart valve.

117. A method for producing a prosthetic implant, **characterized in that**, comprising:
providing a stent and a perivalvular leakage preventing device separately, wherein the stent is tubular and has a cell structure, and the perivalvular leakage preventing device comprises:
a plurality of sealing blocks, multiple blocks arranged around the circumference of the prosthetic implant, each sealing block made of a deformable structure;
a pulling wire, the sealing blocks connected to each other via the pulling wire to constitute the perivalvular leakage preventing device;
the processing method further comprising:
curling the perivalvular leakage preventing device and connecting ends of the perivalvular leakage preventing device to form a coiled state;
fixing the perivalvular leakage preventing device in the coiled state to the stent, during fixation, each sealing block embedded in a corresponding grid structure.

118. A method for producing a prosthetic implant, **characterized in that**, comprising:
providing a stent and a perivalvular leakage preventing device separately, wherein the stent is tubular and has a grid structure, and the perivalvular leakage preventing device comprises:
a plurality of sealing blocks, the plurality of sealing blocks arranged around the circumference of the prosthetic implant, each sealing block made of a deformable structure;
a pulling wire, the sealing blocks connected to each other via the pulling wire to constitute the perivalvular leakage preventing device;
the processing method further comprising:
placing one part of the perivalvular leakage preventing device along a length direction of the perivalvular leakage preventing device into an inside or an outside of the stent and fixing the perivalvular leakage preventing device to the stent;
gradually fixing the remaining part of the perivalvular leakage preventing device to the stent until ends of the perivalvular leakage preventing device are connected to form a coiled state, during fixation, each sealing block embedded in a corresponding grid structure.

119. A mold for forming a perivalvular leakage preventing device, **characterized in that**, comprising:
a main body having a working surface on one side, the working surface defining:
a plurality of mold cavities configured to form sealing blocks;
a wire groove intersecting with the mold cavities for receiving a pulling wire, the formed sealing blocks are connected by the pulling wire to constitute the perivalvular leakage preventing device.

120. The mold for forming a perivalvular leakage preventing device of claim 119, wherein the wire groove is open on the working surface.

121. The mold for forming a perivalvular leakage preventing device of claim 119, wherein the wire groove extends to an edge of the main body.

122. The mold for forming a perivalvular leakage preventing device of claim 119, wherein the wire groove extends along a straight line.

123. A mold for forming a perivalvular leakage preventing device, **characterized in that**, comprising:
a main body having a working surface on one side, the working surface defining a plurality of mold cavities configured to form sealing blocks;
a forming frame stacked on the working surface and surrounding a periphery of openings of the mold cavities.

124. The mold for forming a perivalvular leakage preventing device of claim 123, wherein the forming frame and the main body are separate structures or an integral structure.

125. The mold for forming a perivalvular leakage preventing device of claim 124, wherein the working surface has a recessed region, and the plurality of mold cavities are located within the recessed region.

126. The mold for forming a perivalvular leakage preventing device of claim 124, wherein a depth of the recessed region and a thickness of the forming frame both correspond to a thickness of the inner membrane.

127. A method for producing a perivalvular leakage preventing device using a mold, **characterized in that**, comprising:
adding raw material of a sealing block into a plurality of mold cavities of the mold;
laying a pulling wire, an extension path of the pulling wire intersects with the mold cavities;
curing the raw material of the sealing block under predetermined conditions;
demolding the formed sealing blocks connected via the pulling wire to obtain the perivalvular leakage preventing device.

128. The method of claim 127, wherein adding raw material of a sealing block and laying a pulling wire are performed in any order, or alternately.

129. The method of claim 127, wherein the raw material of the sealing block is fluid or paste-like, and is leveled or scraped flat to be flush with the working surface.

130. The method of claim 127, wherein before curing the raw material of the sealing block, raw material of an inner membrane is further used to form a coating on a top side of the raw material of the sealing block, and then the raw material of the inner membrane and the raw material of the sealing block are cured simultaneously.

131. The method of claim 127, wherein at least one of the raw material of the sealing block and raw material of an inner membrane is paste-like.

132. The method of claim 127, wherein the raw material of the sealing block contains solid particles, and the method further comprises removing the solid particles after demolding.

133. The method of claim 127, wherein a curing temperature is 10-60°C, and a curing time is 1-24 hours.

134. A perivalvular leakage preventing skirt, **characterized in that**, at least a portion of the perivalvular leakage preventing skirt has a flexible substrate, and at least a portion is provided with a reinforcement component in the form of a fabric, and the flexible substrate and the reinforcement component have an overlapping region.

135. The perivalvular leakage preventing skirt of claim 134, wherein the perivalvular leakage preventing skirt comprises:
the flexible substrate made of polyurethane membrane;
the reinforcement component in the form of a fabric, wherein at least a portion of the reinforcement component and at least a portion of the flexible substrate overlap and are fixed to each other.

136. The perivalvular leakage preventing skirt of claim 135, wherein at least a portion of the reinforcement component extends beyond the flexible substrate.

137. The perivalvular leakage preventing skirt of claim 135 or 136, wherein the perivalvular leakage preventing skirt further comprises a sealing assembly fixed to the flexible substrate, the sealing assembly is made of a deformable structure.

138. The perivalvular leakage preventing skirt of claim 134, wherein an elongation at break of the flexible substrate is not less than 200%, and a tensile strength of the flexible substrate is 30-100 MPa.

139. The perivalvular leakage preventing skirt of claim 138, wherein the sealing assembly is made of polyurethane with an open pore structure.

140. The perivalvular leakage preventing skirt of claim 139, wherein the pores in the sealing assembly are interconnected and have uniform pore size.

141. The perivalvular leakage preventing skirt of claim 139, wherein the porosity of the sealing assembly is 50%-95%.

142. The perivalvular leakage preventing skirt of claim 135, wherein the fabric is a flexible fabric with a thickness range of 0.04 mm to 0.2 mm.

143. The perivalvular leakage preventing skirt of claim 135, wherein the raw material of the fabric is one of polyester, nylon, or polyetheretherketone.

144. The perivalvular leakage preventing skirt of claim 135, wherein the fabric is a weft-knitted fabric.

145. The perivalvular leakage preventing skirt of claim 136, wherein the perivalvular leakage preventing skirt has an inflow end and an opposite outflow end according to an intended blood flow direction in a physiological environment, and the portion of the reinforcement component extending beyond the flexible substrate is located at the outflow end.

146. The perivalvular leakage preventing skirt of claim 145, wherein the sealing assembly is located at the inflow end of the perivalvular leakage preventing skirt.

147. The perivalvular leakage preventing skirt of claim 146, wherein in the axial direction of the perivalvular leakage preventing skirt, the sealing assembly is spaced apart from the reinforcement component.

148. The perivalvular leakage preventing skirt of claim 147, wherein a distance between the sealing assembly and the reinforcement component is 3-20 mm.

149. The perivalvular leakage preventing skirt of claim 137, wherein the sealing assembly and the reinforcement component are located on the same side of the flexible substrate; or the sealing assembly and the reinforcement component are located on different sides of the flexible substrate.

150. The perivalvular leakage preventing skirt of claim 137, wherein the sealing assembly protrudes relative to the flexible substrate.

151. The perivalvular leakage preventing skirt of any one of claims 134-136, wherein the flexible substrate has a first edge and an opposite second edge, the first edge corresponds to a position of the inflow end of the perivalvular leakage preventing skirt, the second edge is configured to be fixed to the stent after being sutured to a leaflet, and the second edge is closer to the outflow end of the stent than the first edge;
the reinforcement component has a third edge and an opposite fourth edge, and the fourth edge of the reinforcement component has the same shape as the second edge of the flexible substrate.

152. The perivalvular leakage preventing skirt of claim 151, wherein the third edge and the fourth edge of the reinforcement component have the same shape, the reinforcement component is substantially wavy, and has the same width throughout.

153. The perivalvular leakage preventing skirt of claim 151, wherein the third edge of the reinforcement component is a smooth arc.

154. The perivalvular leakage preventing skirt of claim 151, wherein the fourth edge of the reinforcement component is aligned with the second edge of the flexible substrate.

155. The perivalvular leakage preventing skirt of any one of claims 134-136, wherein the flexible substrate has a first edge and an opposite second edge, the second edge is closer to the outflow end of the stent than the first edge;
the reinforcement component has a third edge and an opposite fourth edge, and the fourth edge of the reinforcement component extends beyond the second edge of the flexible substrate to form an extension part for suturing.

156. The perivalvular leakage preventing skirt of claim 137, wherein the sealing assembly consists of at least one protrusion fixed onto the flexible substrate.

157. The perivalvular leakage preventing skirt of claim 135, wherein the reinforcement component and the flexible substrate are connected by at least one of the following methods: solvent bonding, solution bonding, thermal fusion bonding, or suture splicing.

158. The perivalvular leakage preventing skirt of claim 157, wherein the solvent used for solvent bonding is at least one of dimethyl sulfoxide, N,N-dimethylformamide, or N,N-dimethylacetamide.

159. The perivalvular leakage preventing skirt of claim 157, wherein the solution used for solution bonding is a polyurethane solution with a polyurethane content of 5-20 wt.%.

160. The perivalvular leakage preventing skirt of claim 157, wherein when using a solution or solvent for bonding, after the bonding area is fully wetted by the solvent or solution, it is dried at a temperature of 50-70°C.

161. The perivalvular leakage preventing skirt of claim 157, wherein a temperature for thermal fusion bonding is 50-70°C, and a heating time is 0.5-2 hours.

162. The perivalvular leakage preventing skirt of claim 137, wherein the sealing assembly and the flexible substrate are connected via a bonding region, and the bonding region is formed by contacting, penetrating, and curing the portion of the flexible substrate adjacent to the protrusion in a solution form.

163. The perivalvular leakage preventing skirt of claim 135, wherein a thickness T1 of the flexible substrate is 0.03 mm-0.05 mm, a thickness T2 of the reinforcement component is 0.04 mm-0.2 mm, a thickness at a location where the flexible substrate and the reinforcement component are overlapped and fixed is T3, which is 0.05 mm-0.24 mm, and T1, T2, T3 satisfy the constraint: 0.05mm≥T1+T2-T3≥0.01 mm.

164. A method for producing a perivalvular leakage preventing skirt, **characterized in that**, comprising:
attaching a reinforcement component and a sealing assembly respectively to a flexible substrate;
cutting the reinforcement component and the flexible substrate into a predetermined shape.

165. The method for producing a perivalvular leakage preventing skirt of claim 164, wherein connecting the reinforcement component and the sealing assembly to the flexible substrate adopts any one of the following sequences:
a) attaching the reinforcement component and then the sealing assembly sequentially to the flexible substrate;
b) attaching the sealing assembly and then the reinforcement component sequentially to the flexible substrate.

166. The method for producing a perivalvular leakage preventing skirt of claim 164, wherein cutting the reinforcement component and the flexible substrate into the predetermined shape is performed in any one of the following sequences:
1) cutting the flexible substrate and the reinforcement component separately, before connecting the reinforcement component to the flexible substrate;
2) cutting the flexible substrate and the reinforcement component simultaneously, after connecting the reinforcement component to the flexible substrate.

167. The method for producing a perivalvular leakage preventing skirt of claim 164, wherein the preparation of the sealing assembly comprises:
mixing a polyurethane solution with solid particles to obtain a pre-mix;
curing and washing the pre-mix, thereby obtaining the sealing assembly having a pore structure.

168. The method for producing a perivalvular leakage preventing skirt of claim 167, wherein a particle size of the solid particles is 80-120 mesh.

169. A prosthetic implant, **characterized in that**, comprising:
a stent, being a deformable tubular structure with a side wall having hollowed regions, an interior of the stent forming a blood flow channel;
one or more leaflets, connected to the stent for controlling the blood flow channel;
a perivalvular leakage preventing skirt of any one of claims 134-163, wherein the perivalvular leakage preventing skirt is located substantially inside the stent, an outflow end of the perivalvular leakage preventing skirt is connected to the leaflets, and the sealing assembly protrudes outward from the stent via the hollowed region of the stent.

170. The prosthetic implant of claim 169, wherein the sealing assembly is located at an inflow end of the stent, and a height of the sealing assembly along an axial direction corresponds to 1 to 2 rows of grids at the inflow end of the stent.

171. A method for producing the prosthetic implant of claim 169 or 170, comprising:
connecting the leaflets and the perivalvular leakage preventing skirt to each other to form a tubular pre-assembly;
assembling the pre-assembly to the stent.

172. An adaptive perivalvular leakage preventing component, **characterized in that**, the perivalvular leakage preventing component comprises: a sheet-like and stretchable flexible substrate, and one or more protrusions attached to the flexible substrate, wherein each protrusion has a pore structure, the flexible substrate and the protrusions are both made of polyurethane material, and a connection between the flexible substrate and the protrusions is achieved by curing a polyurethane solution.

173. The adaptive perivalvular leakage preventing component of claim 172, wherein an elongation at break of the flexible substrate is not less than 200%.

174. The adaptive perivalvular leakage preventing component of claim 173, wherein an elongation at break of the flexible substrate is not less than 300%.

175. The adaptive perivalvular leakage preventing component of claim 173, wherein an elongation at break of the flexible substrate is 200%-500%.

176. The adaptive perivalvular leakage preventing component of claim 173, wherein a pore size of the protrusions is 5-500 micrometers.

177. The adaptive perivalvular leakage preventing component of claim 176, wherein a pore size of the protrusions is 5-450 micrometers.

178. The adaptive perivalvular leakage preventing component of claim 177, wherein a pore size of the protrusions is 5-400 micrometers.

179. The adaptive perivalvular leakage preventing component of claim 178, wherein a pore size of the protrusions is 5-350 micrometers.

180. The adaptive perivalvular leakage preventing component of claim 179, wherein a pore size of the protrusions is 5-300 micrometers.

181. The adaptive perivalvular leakage preventing component of claim 180, wherein a pore size of the protrusions is 5-250 micrometers.

182. The adaptive perivalvular leakage preventing component of claim 172, wherein a porosity of the protrusions is 50%-95%.

183. The adaptive perivalvular leakage preventing component of claim 182, wherein a porosity of the protrusions is 60%-95%.

184. The adaptive perivalvular leakage preventing component of claim 183, wherein a porosity of the protrusions is 70%-90%.

185. The adaptive perivalvular leakage preventing component of claim 172, wherein the protrusions have an open-pore structure and/or a closed pore structure.

186. The adaptive perivalvular leakage preventing component of claim 172, wherein an average thickness of the flexible substrate is 0.02-0.20 mm.

187. The adaptive perivalvular leakage preventing component of claim 186, wherein an average thickness of the flexible substrate is 0.02-0.15 mm.

188. The adaptive perivalvular leakage preventing component of claim 187, wherein an average thickness of the flexible substrate is 0.02-0.12 mm.

189. The adaptive perivalvular leakage preventing component of claim 172, wherein a tensile strength of the flexible substrate is 30-100 MPa.

190. The adaptive perivalvular leakage preventing component of claim 189, wherein a tensile strength of the flexible substrate is 40-100 MPa.

191. The adaptive perivalvular leakage preventing component of claim 190, wherein a tensile strength of the flexible substrate is 50-100 MPa.

192. The adaptive perivalvular leakage preventing component of claim 191, wherein a tensile strength of the flexible substrate is 50-90 MPa.

193. The adaptive perivalvular leakage preventing component of claim 192, wherein a tensile strength of the flexible substrate is 50-80 MPa.

194. An adaptive perivalvular leakage preventing component, **characterized in that** the perivalvular leakage preventing component comprises: a sheet-like, stretchable flexible substrate, and one or more protrusions attached to the flexible substrate, wherein each protrusion has a pore structure, the flexible substrate and the protrusions are both made of polyurethane material, and the flexible substrate and the protrusions are connected via a bonding region, the bonding region is formed by contacting, penetrating, and curing a portion of the flexible substrate adjacent to the protrusion in a solution form.

195. The adaptive perivalvular leakage preventing component of claim 194, wherein both the protrusions and the flexible substrate have a pore structure.

196. The adaptive perivalvular leakage preventing component of claim 195, wherein a porosity of the protrusions is not lower than that of the flexible substrate.

197. The adaptive perivalvular leakage preventing component of claim 194, wherein the flexible substrate is a dense structure without pores.

198. An adaptive perivalvular leakage preventing component, **characterized in that** the perivalvular leakage preventing component is made of polyurethane material and comprises: a sheet-like, stretchable flexible substrate, and one or more protrusions connected to the flexible substrate, wherein the flexible substrate and the protrusions are both formed by a casting molding process, casting liquid for forming the protrusions is a polyurethane solution containing solid particles, and casting liquid for the flexible substrate is a polyurethane solution or a polyurethane mixture containing solid particles.

199. A method for producing an adaptive perivalvular leakage preventing component comprising the following steps in any order:
filling a mold with a polyurethane mixture containing solid particles, curing the mixture, and then washing the mixture to remove the solid particles to obtain protrusions;
obtaining a flexible substrate by curing a polyurethane solution, or by curing and then washing a polyurethane mixture containing solid particles;
connecting the flexible substrate and the protrusions by curing a polyurethane solution.

200. The producing method of claim 199, wherein a polyurethane molecular chain comprises hard segments and soft segments, the soft segment content is 40-70%, and the remainder is the hard segments; the soft segment is at least one of polyether diol, polycarbonate diol, polyester diol, or polysiloxane diol; the hard segment is isocyanate, and a R value of the isocyanate is 1.0-1.1.

201. The producing method of claim 199, wherein in the polyurethane molecular chain, the soft segment content is 50%-65%.

202. The producing method of claim 199, wherein the isocyanate is at least one of TDI, HDI, MDI, NDI, PPDI, IPDI, or XDI.

203. The producing method of claim 199, wherein the polyurethane molecular chain further comprises a chain extender, and the chain extender is at least one of ethylene glycol, butanediol, hexanediol, octanediol, or ethylenediamine.

204. The producing method of claim 199, wherein the solvent for the polyurethane solution is at least one of dimethyl sulfoxide, tetrahydrofuran, dimethylformamide, dimethylacetamide, dichloromethane, chloroform, or hexafluoroisopropanol.

205. The producing method of claim 199, wherein the solvent for the polyurethane solution is two selected from dimethyl sulfoxide, tetrahydrofuran, dimethylformamide, dimethylacetamide, dichloromethane, chloroform, and hexafluoroisopropanol, and avolume ratio of the two solvents is 1-99:1.

206. The producing method of claim 205, wherein the volume ratio of the two solvents is 5-95:1.

207. The producing method of claim 205, wherein the volume ratio of the two solvents is 10-95:1.

208. The producing method of claim 205, wherein the volume ratio of the two solvents is 15-95:1.

209. The producing method of claim 205, wherein the volume ratio of the two solvents is 20-95:1.

210. The producing method of claim 205, wherein the volume ratio of the two solvents is 25-95:1.

211. The producing method of claim 205, wherein the volume ratio of the two solvents is 10-90:1.

212. The producing method of claim 205, wherein the volume ratio of the two solvents is 10-80:1.

213. The producing method of claim 205, wherein the volume ratio of the two solvents is 15-75:1.

214. The producing method of claim 205, wherein the volume ratio of the two solvents is 20-75:1.

215. The producing method of claim 205, wherein the volume ratio of the two solvents is 25-75:1.

216. The producing method of claim 199, wherein a concentration of polyurethane in the polyurethane solution is 0.25%-25% (w/v).

217. The producing method of claim 199, wherein a concentration of polyurethane is 0.5%-20% (w/v).

218. The producing method of claim 199, wherein a concentration of polyurethane is 0.5%-15% (w/v).

219. The producing method of claim 199, wherein a concentration of polyurethane is 0.5%-10% (w/v).

220. The producing method of claim 199, wherein a concentration of polyurethane is 5%-25% (w/v).

221. The producing method of claim 199, wherein a concentration of polyurethane is 1%-25% (w/v).

222. The producing method of claim 199, wherein a concentration of polyurethane is 2%-25% (w/v).

223. The producing method of claim 199, wherein a concentration of polyurethane is 3%-25% (w/v).

224. The producing method of claim 199, wherein a concentration of polyurethane is 4%-25% (w/v).

225. The producing method of claim 199, wherein a concentration of polyurethane is 5%-20% (w/v).

226. The producing method of claim 199, wherein a concentration of polyurethane is 5%-15% (w/v).

227. The producing method of claim 199, wherein a concentration of polyurethane is 8%-20% (w/v).

228. The producing method of claim 199, wherein a concentration of polyurethane is 10%-20% (w/v).

229. The producing method of claim 199, wherein the solid particles are at least one of sodium chloride, potassium chloride, or sucrose.

230. The producing method of claim 199, wherein a number-average particle size of the solid particles is 5-500 micrometers.

231. The producing method of claim 199, wherein a number-average particle size of the solid particles is 5-450 micrometers.

232. The producing method of claim 199, wherein a number-average particle size of the solid particles is 5-400 micrometers.

233. The producing method of claim 199, wherein a number-average particle size of the solid particles is 5-350 micrometers.

234. The producing method of claim 199, wherein a number-average particle size of the solid particles is 5-300 micrometers.

235. The producing method of claim 199, wherein a number-average particle size of the solid particles is 5-250 micrometers.

236. The producing method of claim 199, wherein in the polyurethane mixture containing solid particles, a mass fraction of the solid particles is 50%-80%.

237. The producing method of claim 199, wherein a mass fraction of the solid particles is 55%-80%.

238. The producing method of claim 199, wherein a mass fraction of the solid particles is 60%-80%.

239. The producing method of claim 199, wherein a mass fraction of the solid particles is 60%-75%.

240. The producing method of claim 199, wherein the mold comprises a plurality of mold cavities, each mold cavity corresponds to one protrusion, and the distribution of the mold cavities corresponds to the distribution of the protrusions.

241. The producing method of claim 199, wherein a curing temperature for the protrusions and the flexible substrate is 20-90°C.

242. The producing method of claim 199, wherein a curing temperature for the protrusions and the flexible substrate is 20-70°C.

243. The producing method of claim 199, wherein a curing temperature for the protrusions and the flexible substrate is 20-50°C.

244. The producing method of claim 199, wherein the preparation of the protrusions and the flexible substrate further comprises a post-treatment after curing, the post-treatment comprising: placing the protrusions and the flexible substrate in water at 20-40°C for at least 1 hour.

245. The producing method of claim 244, wherein the post-treatment comprises: placing the protrusions and the flexible substrate in water at 20-40°C for 2-4 hours.

246. The producing method of claim 199, wherein the preparation of the protrusions and the flexible substrate further comprises a pre-curing step before the curing step, the pre-curing conditions is: a pre-curing temperature of 20-70°C, a pre-curing time of 20-60 minutes.

247. The producing method of claim 246, wherein the pre-curing conditions are: the pre-curing temperature 30-60°C, the pre-curing time of 20-40 minutes.

248. The producing method of claim 199, wherein the washing of the protrusions and the flexible substrate comprises: placing the protrusions in a washing agent and letting the protrusions stand for at least 4 hours.

249. The producing method of claim 199, wherein the washing of the protrusions and the flexible substrate comprises: placing the protrusions in a washing agent and letting the protrusions stand for 4-12 hours.

250. The producing method of claim 199, wherein the washing of the protrusions and the flexible substrate comprises: treating the protrusions in a washing agent under agitation for at least 4 hours.

251. The producing method of claim 199, wherein the washing of the protrusions and the flexible substrate comprises: treating the protrusions in a washing agent under agitation for 4-12 hours.

252. The producing method of claim 199, wherein the polyurethane solution in contact with the protrusions is at room temperature or has been preheated, and a preheating temperature of the polyurethane solution is 30-70°C.

253. The producing method of claim 199, wherein a preheating temperature of the polyurethane solution is 40-70°C.

254. The producing method of claim 199, wherein a preheating temperature of the polyurethane solution is 50-70°C.

255. The producing method of claim 199, wherein the preparation of the protrusions and the flexible substrate further comprises an initial curing step before the pre-curing step, and the initial curing is performed by one of the following methods:
a) blowing hot air over a surface of the polyurethane mixture containing solid particles;
b) spraying hot steam over the surface of the polyurethane mixture containing solid particles;
c) adding water, ethanol, or isopropanol dropwise into the polyurethane mixture containing solid particles;
in step a), a hot air temperature is 40-100°C, and a blowing time is 0.5-2 minutes;
in step b), a steam temperature is 40-100°C, and a spraying time is 0.5-2 minutes;
in step c), 1-2.5 mL is added.

256. A prosthetic heart valve, comprising:
a stent, having a blood flow channel inside, the stent having an inflow side and an opposite outflow side, and the inflow side having a grid structure;
one or more leaflets;
a perivalvular leakage preventing component connected to the inflow side,
wherein the flexible substrate of the perivalvular leakage preventing component is located inside the stent, and protrusions are embedded in the grid structure; or the flexible substrate of the perivalvular leakage preventing component is located outside the stent, and protrusions are located on the side of the flexible substrate facing away from the stent.
